# EUROPEAN PATENT APPLICATION

(11) **EP 4 631 947 A1**
(43) Date of publication of application: **15.10.2025**
(21) Application number: 25169209.1
(22) Date of filing: 08.04.2025
(51) Int. Cl.: C07D 471/18, A61P 3/00, A61K 31/551, A61P 7/00, A61P 25/00, A61P 29/00, A61P 31/00, A61P 35/00

(54) **SUBSTITUTED TRICYCLIC LIGANDS FOR FK506-BINDING PROTEINS FOR TREATMENT OF DISEASES**

(30) Priority: 08.04.2024 EP 24169007
(71) Applicant: Technische Universität Darmstadt, 64289 Darmstadt (DE)
(72) Inventor: Hausch, Felix, 64289 Darmstadt (DE); Krajczy, Patryk, 64289 Darmstadt (DE)
(74) Representative: Fuchs Patentanwälte Partnerschaft mbB

(57) **Abstract**

The present invention relates to compounds having a fused, substituted tricyclic scaffold and stereo-isomeric forms, solvates, hydrates and/or pharmaceutically acceptable salts of these compounds as well as pharmaceutical compositions containing at least one of these substituted tricyclic derivatives together with pharmaceutically acceptable carrier, excipient and/or diluents. Said substituted tricyclic derivatives have been identified as especially potent ligands of FK506 binding proteins (FKBPs), especially human FKBP12, FKBP12.6, FKBP51 and FKBP52 or bacterial homologs like LpMip, CtMip, CpMip, NgMip, KpMip, BpMip, TcMip, EcFKLB, EcFKPA, PaFKLB, PaFKPA, AbFKLB, AbFKPA, and are useful for treatment of diseases such as psychiatric, metabolic, infective, neurological and haematological disorders as well as pain and cancers and as anti-inflammatory drugs. Said substituted tricyclic derivatives may further be used as agents for inducing protein-protein interactions (PPIs) acting as molecular glues.

## Description

The present invention relates to compounds having a fused, substituted tricyclic scaffold and stereo-isomeric forms, solvates, hydrates and/or pharmaceutically acceptable salts of these compounds as well as pharmaceutical compositions containing at least one of these substituted tricyclic derivatives together with pharmaceutically acceptable carrier, excipient and/or diluents. Said substituted tricyclic derivatives have been identified as especially potent ligands of FK506 binding proteins (FKBPs), especially human FKBP12, FKBP12.6, FKBP51 and FKBP52 or bacterial homologs like LpMip, CtMip, CpMip, NgMip, KpMip, BpMip, TcMip, EcFKLB, EcFKPA, PaFKLB, PaFKPA, AbFKLB, AbFKPA, and are useful for treatment of diseases such as psychiatric, metabolic, infective, neurological and haematological disorders as well as pain and cancers and as anti-inflammatory and anti-infective drugs. Said substituted tricyclic derivatives may further be used as agents for inducing protein-protein interactions (PPIs) acting as molecular glues.

### Background of the invention

The FK506-binding protein (FKBP) family of immunophilins consists of proteins with a variety of protein-protein interaction domains and versatile cellular functions. The bacterial homologs are also called macrophage infectivity potentiators (Mip). This highly conserved protein family binds to immunosuppressive drugs, such as FK506 and rapamycin. This protein family displays peptidyl propyl isomerase (PPlase) activity as seen with cyclophilins and parvulins. FKBP12, a 12 kD protein is the most widely studied member of this family.

The immunosuppressant drugs FK506, rapamycin, and cyclosporin are well known as potent T-cell specific immunosuppressants, and are effective against autoimmunity, transplant or graft rejection, inflammation, allergic responses, other autoimmune or immune-mediated diseases.

FK506 and rapamycin apart from binding to FKBP12 also interact and inhibit calcineurin (CaN) and mTOR, respectively, thereby mediating their immunosuppressive action.

FKBP12 and FKBP12.6 are regulators of ryanodine receptors and of receptors from the TGF/ALK family and are involved for example in haematological and neurological disorder. The high molecular weight multidomain homologs of FKBP51 and FKBP 52, act as cochaperones for the heat shock protein 90 (Hsp90) and modulate the signal transduction of the steroid hormone receptors such as the glucocorticoid receptor by participating in the Heat shock protein 90 (Hsp90) - steroid hormone receptor complex.

In this complex, FKBP51 and FKBP52 modulate the binding competence and signalling of steroid hormone receptors and thereby regulate the cellular responsiveness to circulating hormone levels. This is supported by cellular studies and by knockout mice, where the crucial role of FKPB51 and FKBP52 on the Glucocorticoid Receptor (GR) Progesterone Receptor (PR) or Androgen Receptor (AR) activity have been clearly demonstrated. Moreover, polymorphisms in the FKBP51-encoding gene of psychiatric patients have been associated with numerous stress-related psychiatric disorders, with diabetes and obesity, and with chronic pain states. Both proteins have been shown to be overexpressed in various cancers.

Bacterial FKBPs, also called Mip, are involved in various steps of the infectivity process or the replication of the bacterial pathogens.

The immunosuppressive compounds disclosed in the prior art suppress the immune system, by definition, and also exhibit other toxic side effects. Accordingly, there is a need for non-immunosuppressant, small molecule compounds, and compositions and methods for use of such compounds, that are useful in treating psychiatric disorders and neurodegenerative diseases, disorders and conditions.

Further studies led to α-ketoamide analogues of FK506 devoid of immunosuppressive activity.

EP 3 875 151 A1, WO 2015/110271 A1, as well as SEBASTIAN POMPLUN ET AL: "Chemoge-nomic Profiling of Human and Microbial FK506 -Binding Proteins", JOURNAL OF MEDICINAL CHEMISTRY, vol. 61, no. 8, April 26, 2018 disclose bridged bicyclic compounds which also inhibit FKBP, but were found to be not suitable for all applications.

Therefore, it is the object of the present invention to provide compounds and/or pharmaceutically acceptable salts thereof, which inhibit FKBPs or Mips more effectively based on significantly increased affinity to FKBPs.

Another aspect of the invention is to provide compounds and/or pharmaceutically acceptable salts thereof which can be used as pharmaceutically active agents, especially for the treatment of psychiatric, metabolic, infective, neurological and haematological disorders as well as pain diseases and cancers, as well as compositions comprising at least one of those compounds and/or pharmaceutically acceptable salts thereof as pharmaceutically active ingredients.

A further aspect of the invention is to provide methods for preparing said compounds.

The object of the present invention is solved by the teaching of the independent claims. Further advantageous features, aspects and details of the invention are evident from the dependent claims, the description, and the examples of the present application.

### Description of the invention

The present invention relates to compounds having fused, substituted tricyclic scaffold and stereo-isomeric forms, solvates, hydrates and/or pharmaceutically acceptable salts of these compounds as well as pharmaceutical compositions containing at least one of these substituted tricyclic derivatives together with pharmaceutically acceptable carrier, excipient and/or diluents.

The molecular structure of the inventive molecule can be generally depicted as follows:

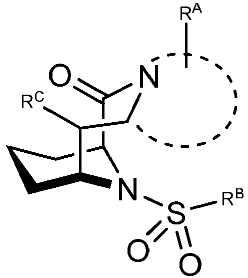

Said substituted tricyclic derivatives have been identified as specific and potent ligands of the FK506 binding proteins (FKBPs), especially to FKBP12, FKBP12.6, FKBP51 and FKBP52 as well as bacterial Mip such LpMip, CtMip, CpMip, NgMip, KpMip, BpMip, TcMip, EcFKLB, EcFKPA, PaFKLB, PaFKPA, AbFKLB, AbFKPA and are useful for the treatment of psychiatric disorders (such as depression or post-traumatic stress disorder), metabolic disorders (such as obesity or diabetes), infective disorders (such as Legionnaire's disease or Chagas diseases), neurological disorders (such as Alzheimer's diseases or Parkinson's diseases) and haematological disorders (such as hereditary haemorrhagic telangiectasia or pulmonary arterial hypertension) as well as pain diseases (such as chronic neuropathic pain or fibromyalgia) and cancers (such as prostate cancer, malignant melanoma, multiple myeloma, or glioblastoma).

The expression prodrug is defined as a pharmacological substance, a drug, which is administered in an inactive or significantly less active form. Once administered, the prodrug is metabolized in the body in vivo into the active compound.

The expression tautomer is defined as an organic compound that is interconvertible by a chemical reaction called tautomerization. Tautomerization can be catalysed preferably by bases or acids or other suitable compounds.

It is important to note that compounds of formula X: 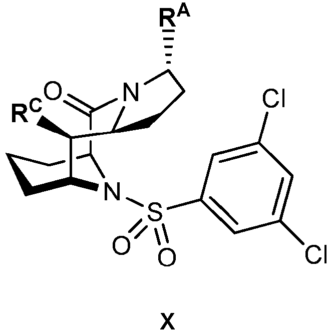 wherein R^{A} is a hydrogen do not show the improved effects as disclosed herein. It has been surprisingly found that R^{A} has to be selected from a specific list of substituents as disclosed hereinunder, in order to show the improved features of binding to FKBPs such as FKBP51 and FKBP12. Thus, in one embodiment a compound of formula X, wherein R^{A} is selected from a hydrogen is excluded from the claimed compounds. If R^{A} is a -COOH-group the binding is surprisingly already improved, but still less efficient than the other groups as disclosed hereinunder. Thus, in another embodiment a compound of formula X, wherein R^{A} is selected from a -COOH-group is excluded from the claimed compounds.

FKBP ligands as used herein are defined as compounds that:
(i) inhibit the peptidyl-prolyl isomerase activity of FKBPs or Mips (PPlase inhibitors, also referred to as rotamase inhibitors), or
(ii) displace FK506 or FK506 analogues from the PPlase active site of FKBPs or Mips, or
(iii) bind to the FK506-binding domain of FKBPs or Mips as determined biophysically by isothermal calorimetry, surface plasmon resonance, tryptophan quenching, NMR or X-ray crystallography.

In a first aspect, the molecules of the present invention may be represented as:
A compound of the general formula **1:** 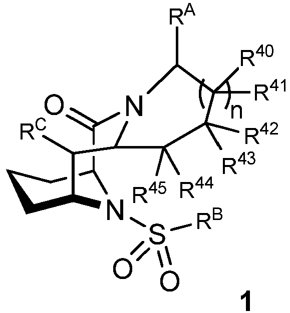
wherein n is 0 or 1,
and wherein **R^{A}** represents:
   -CH₂OR¹⁶,
   -CH₂NR³⁸R³⁹, 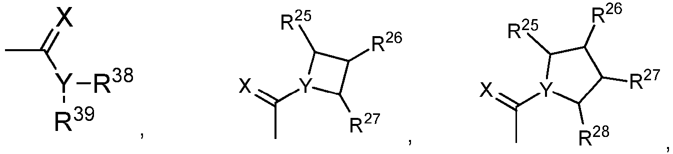 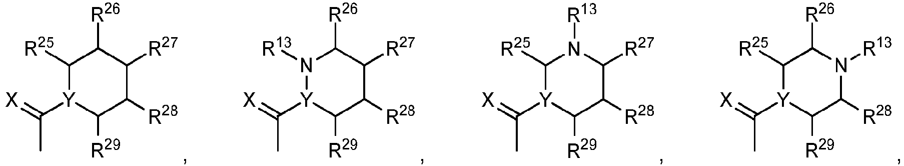 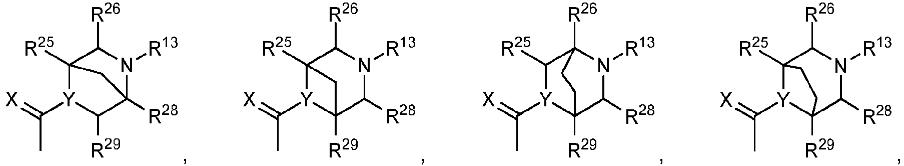 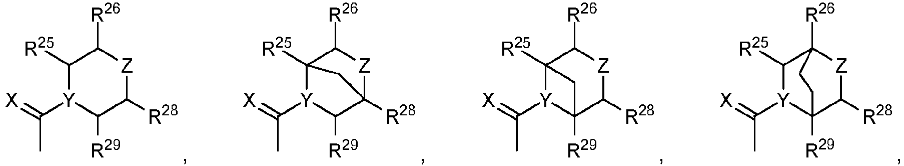 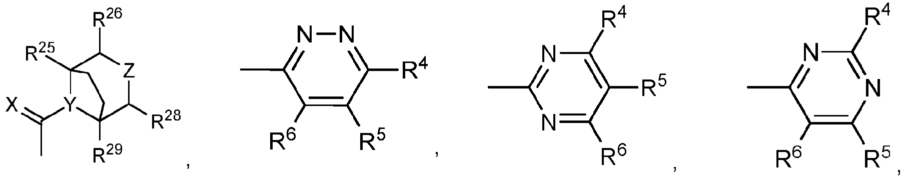 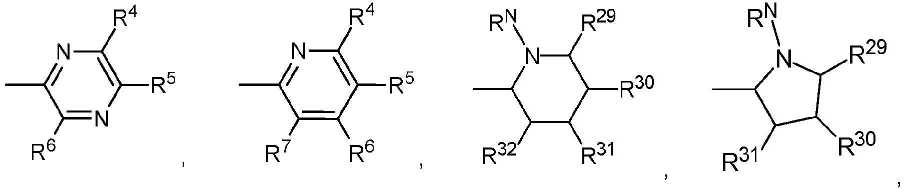 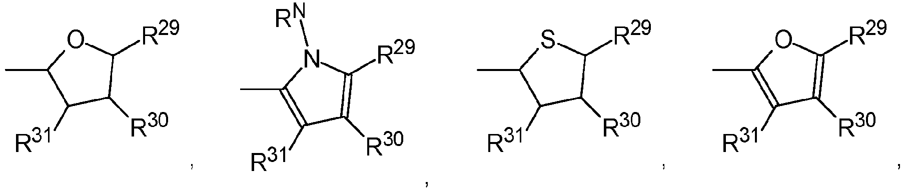 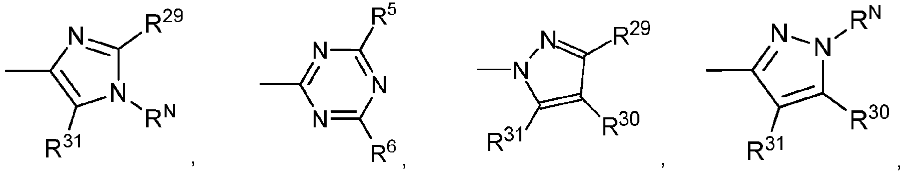 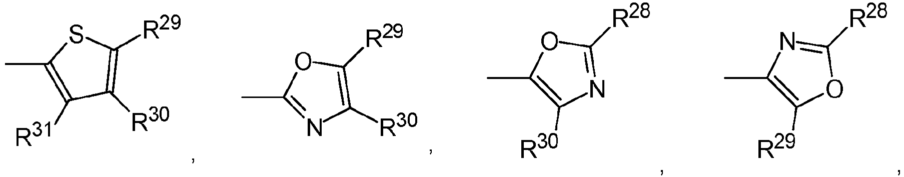 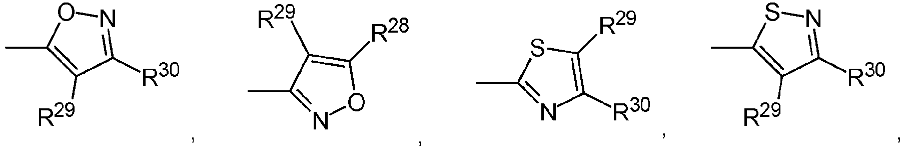 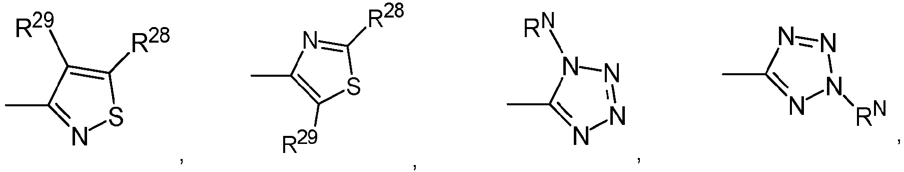 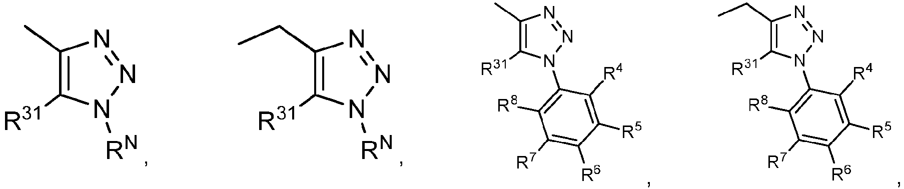 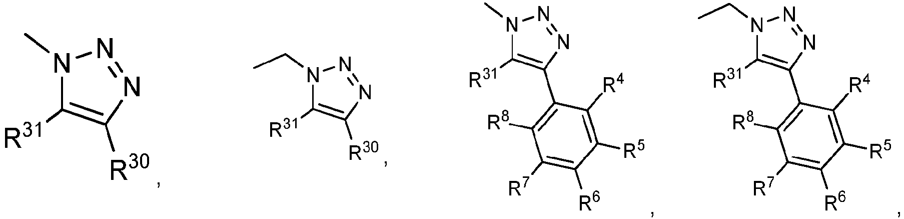 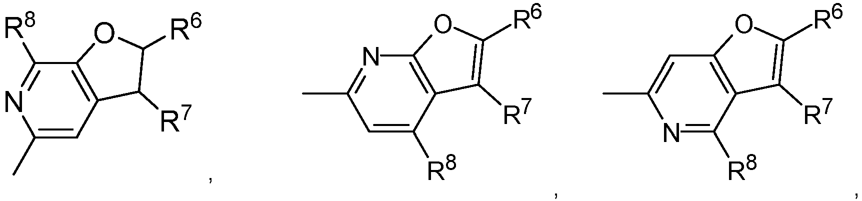 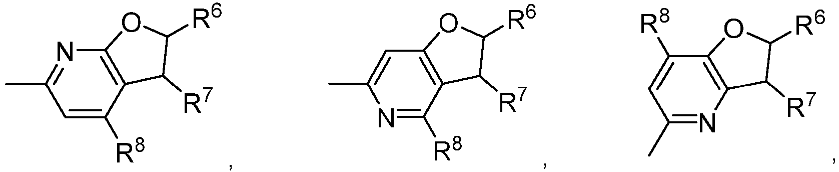 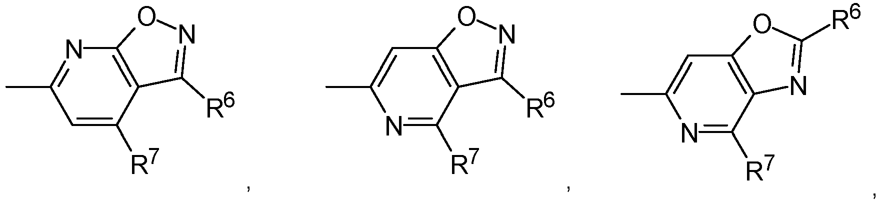 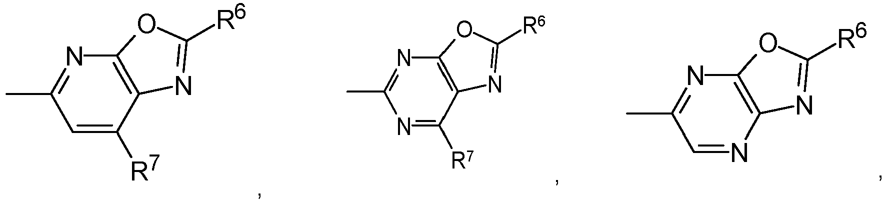 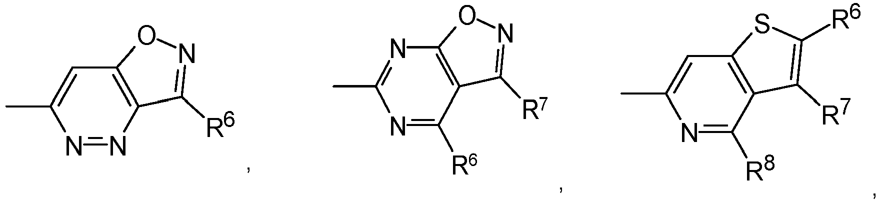 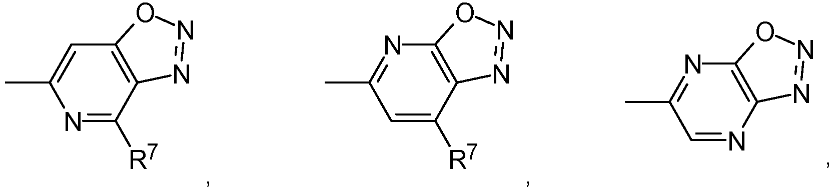 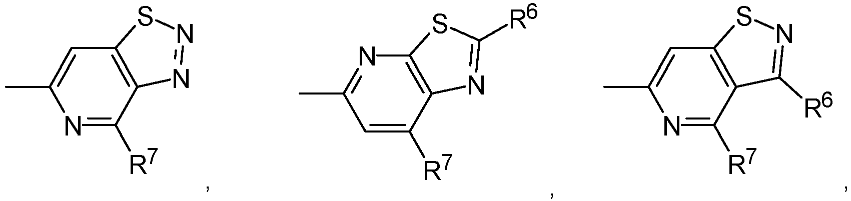 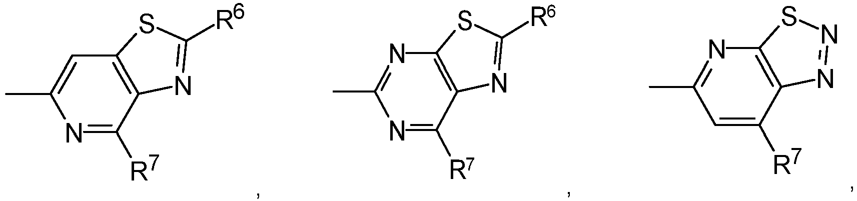 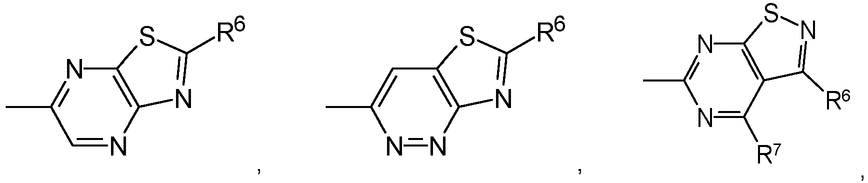 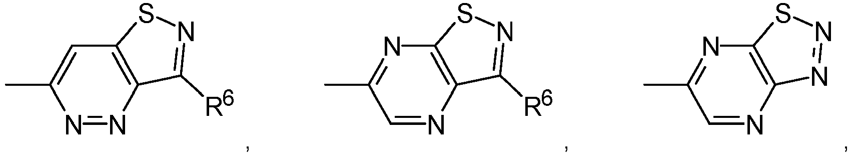 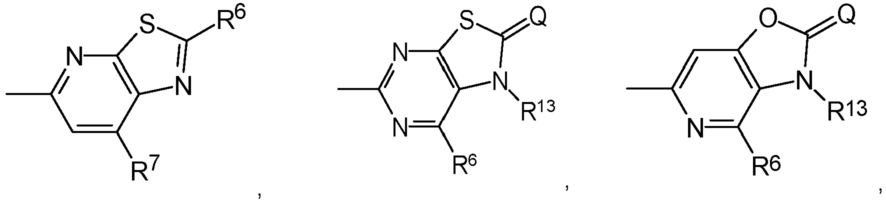 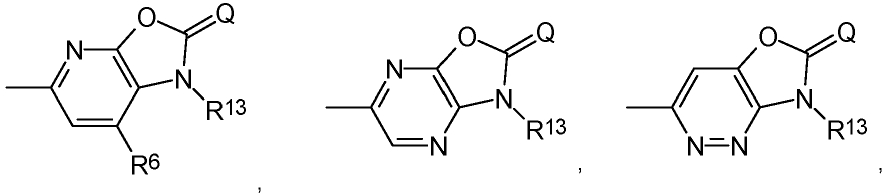 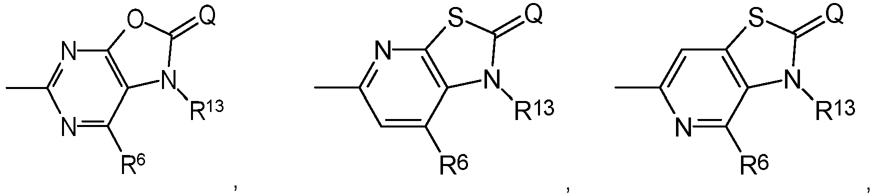 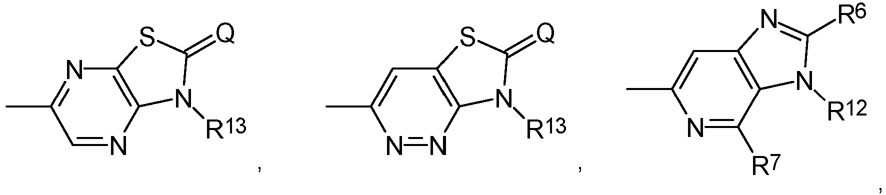 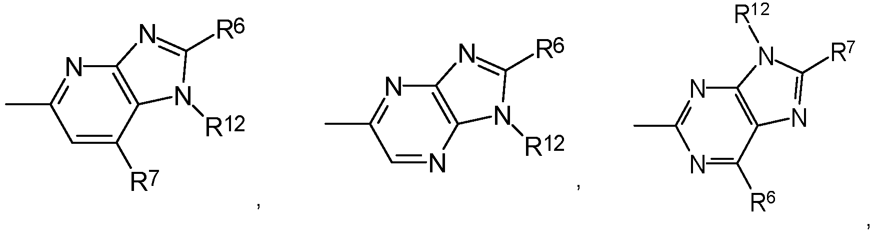 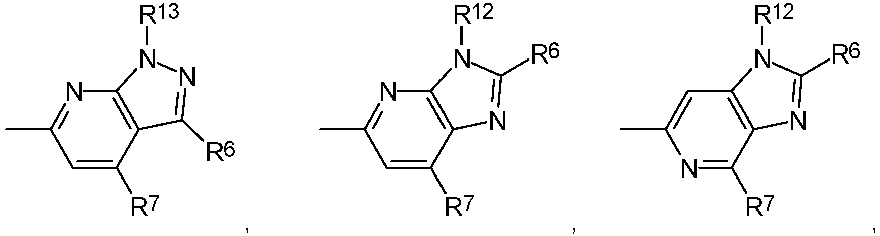 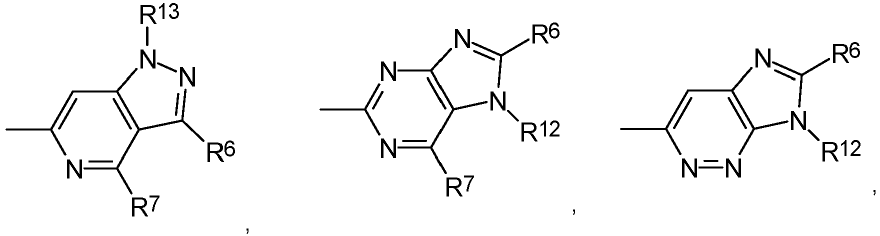 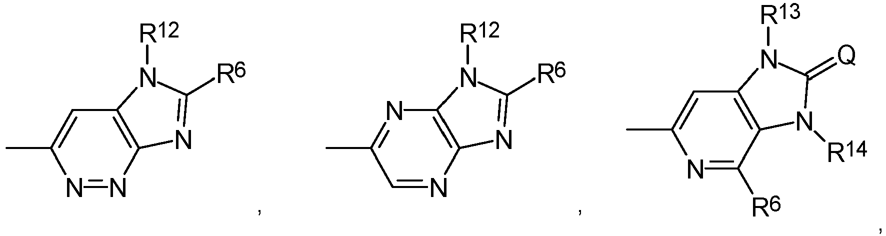 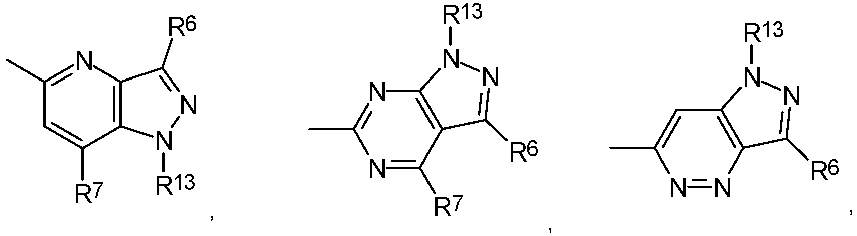 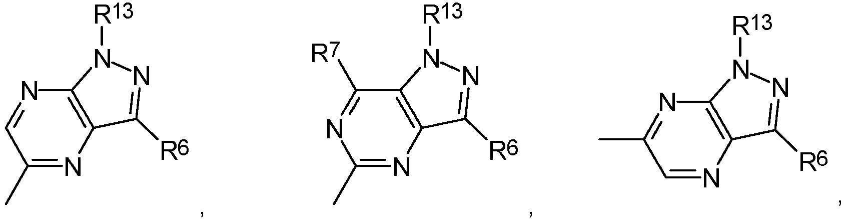 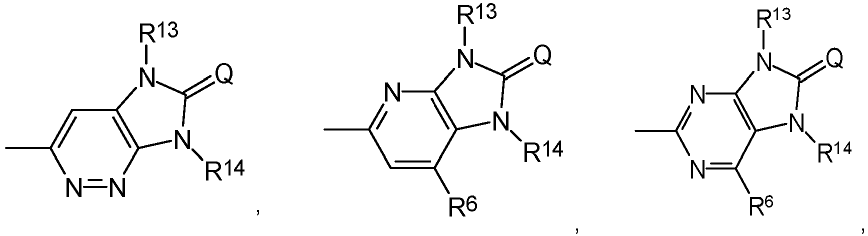 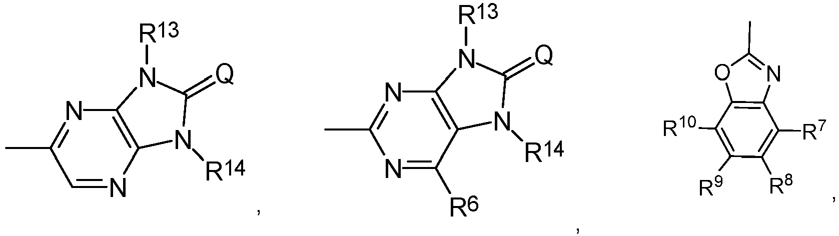 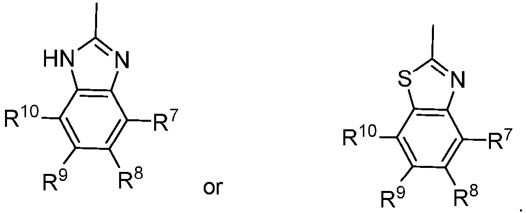
wherein **X** represents O, S, or H, H (e.g., C=X represents CH₂),
wherein **Y** represents N,-CH- or -CH₂-,
wherein **Z** represents O, N-R^{N}, C=O or SO₂,
wherein **Q** represents =O, =S, or =N-R¹²,
wherein **R^{N}** represents -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, -COC₃H₇, -CO-cyclo-C₃H₅, -COCH(CH₃)₂, -COC(CH₃)₃, -COPh, -COCN, -COOCH₃, -COOC₂H₅, -COOC₃H₇, -COO-cyclo-C₃H₅, -COOCH(CH₃)₂, -COOC(CH₃)₃, -COCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -CH₂NH₂, -CH₂NHCH₃, -CH₂NHC₂H₅, -CH₂NHC₃H₇, -CH₂NH-cyclo-C₃H₅, -CH₂NH[CH(CH₃)₂], -CH₂NH[C(CH₃)₃], -CH₂N(CH₃)₂, -CH₂N(C₂H₅)₂, -CH₂N(C₃H₇)₂, -CH₂N(cyclo-C₃H₅)₂, -CH₂N[CH(CH₃)₂]₂, -CH₂N[C(CH₃)₃]₂, -CH₂-NHCOCH₃, -CH₂-NHCHO, -CH₂-NHSO₂CH₃, -CONH₂, -CONHCH₃, -CONHC₂H₅,-CONHC₃H₇, -CH₂-NH(C₂H₅), -SO₂CH₃, -SO₂C₂H₅, -SO₂CH₂Ph, -SO₂C₃H₇, -SO₂-cyclo-C₃H₅, -SO₂CH(CH₃)₂, -SO₂C(CH₃)₃, -SO₂Ph, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH₂-C≡C-C₂H₅, -CH=CH-CH=C(CH₃)₂, -CH₂-CH(CH₃)-CH₂-C≡CH, -C₂H₄-C≡C-CH₃, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, - C≡C-CH(CH₃)-C≡CH, -CH(CH₃)-C≡C-C≡CH, -CH(C≡CH)-CH₂-C≡CH, -CH(C≡CH)-C≡C-CH₃,
**R^{B}** represents 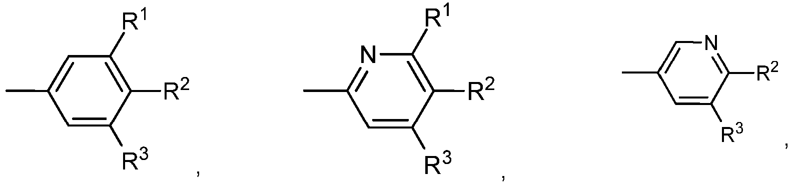 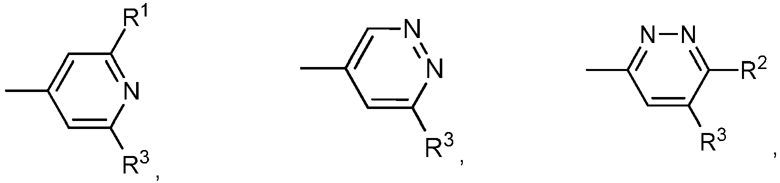 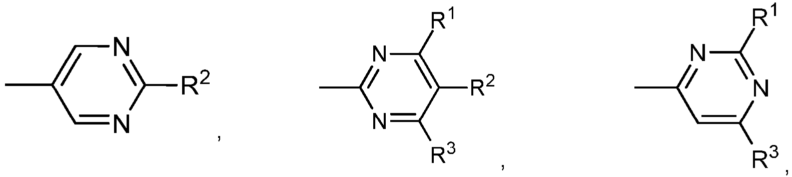 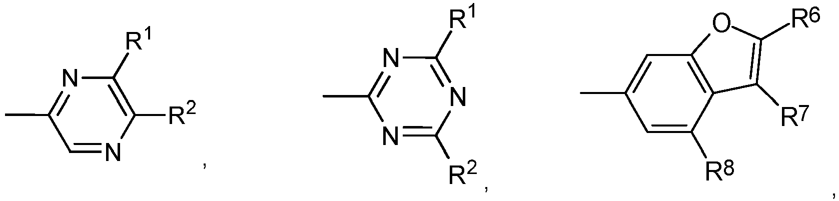 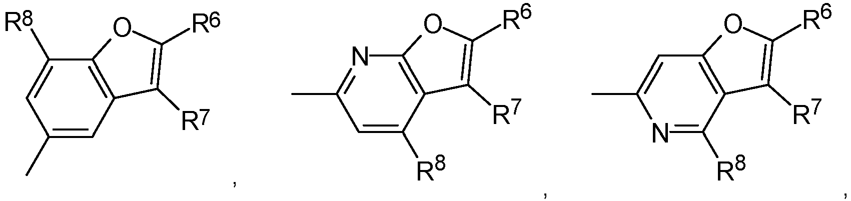 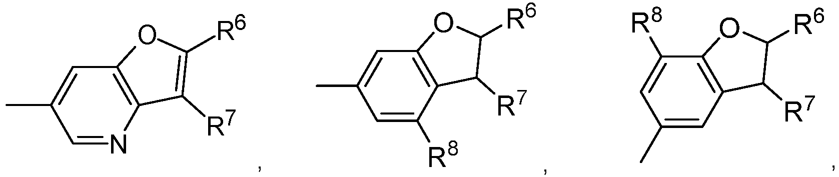 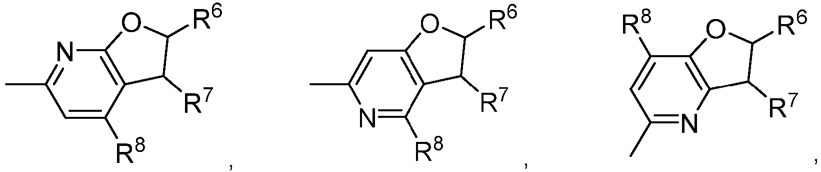 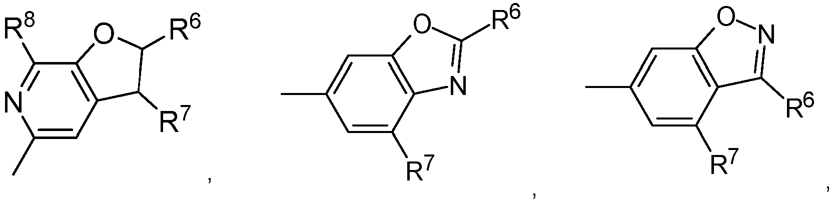 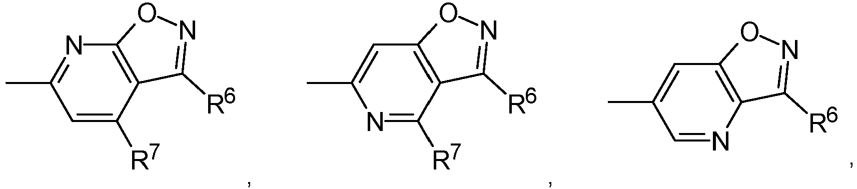 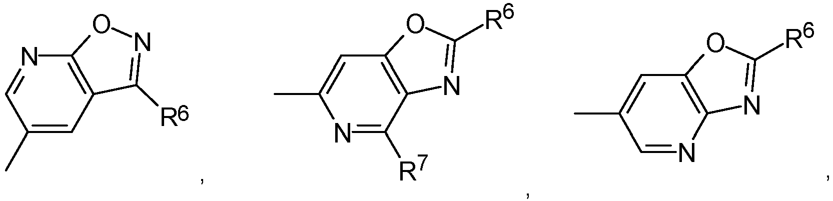 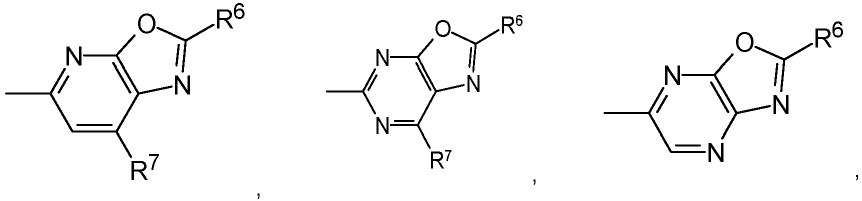 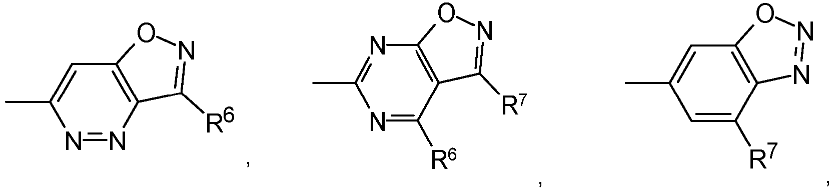 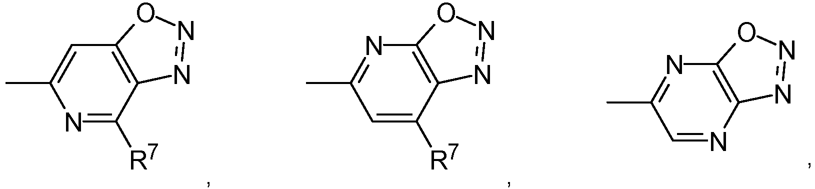 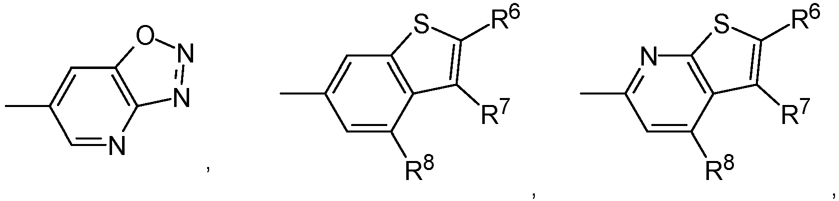 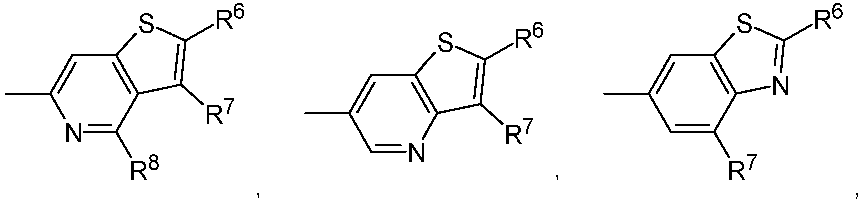 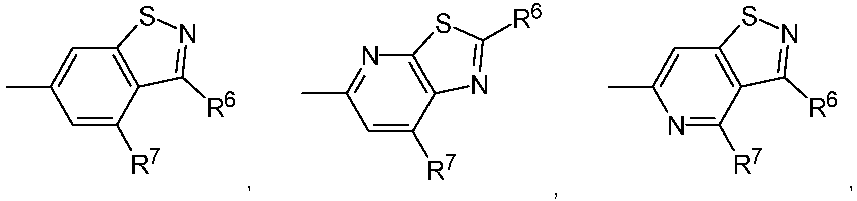 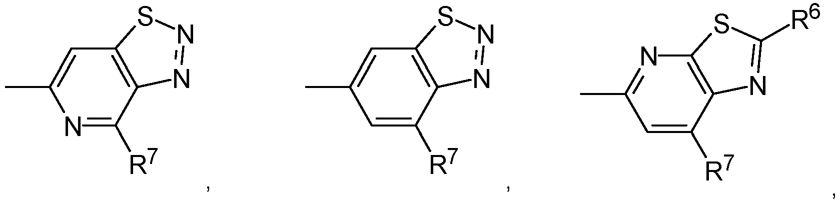 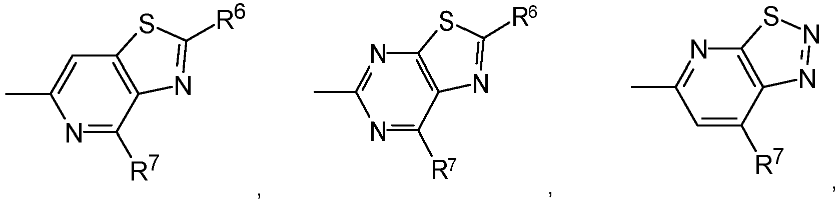 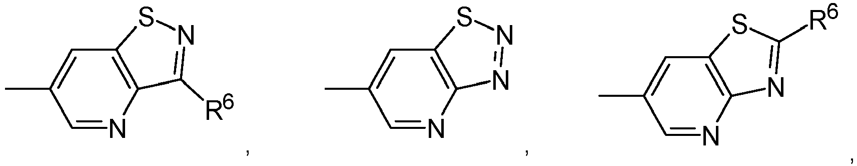 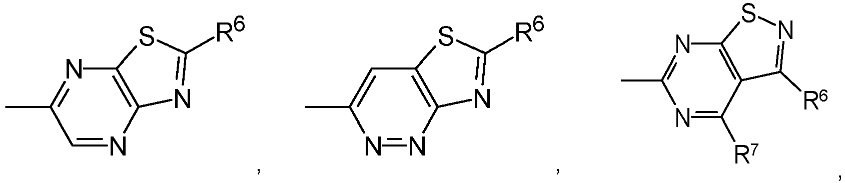 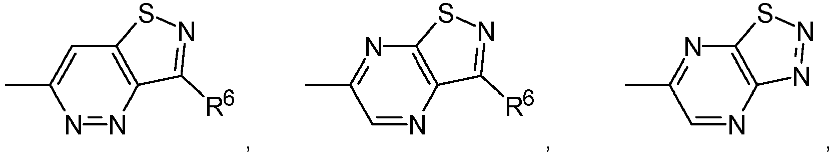 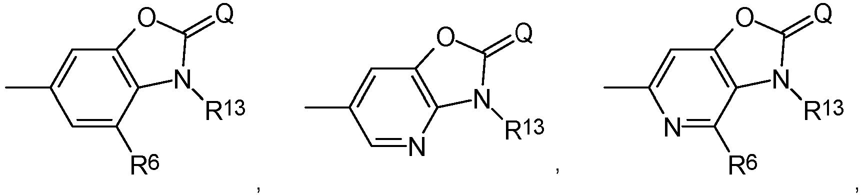 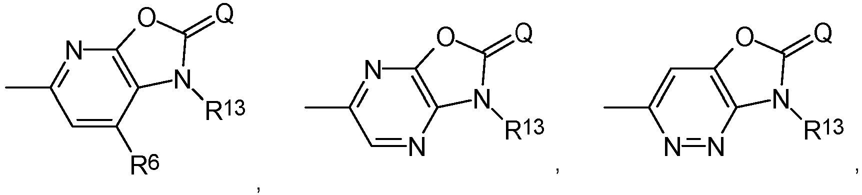 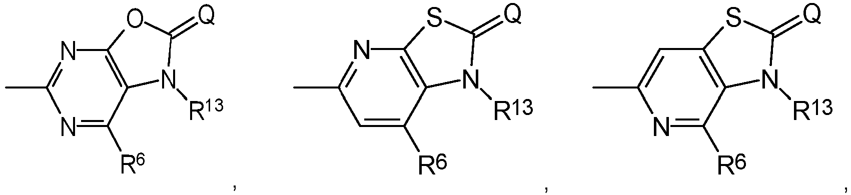 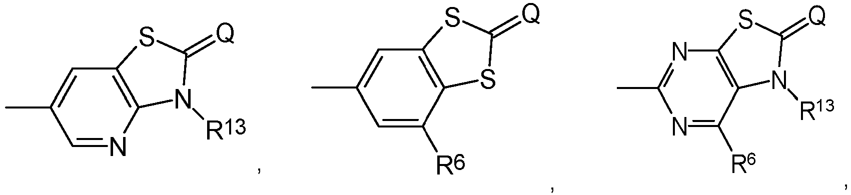 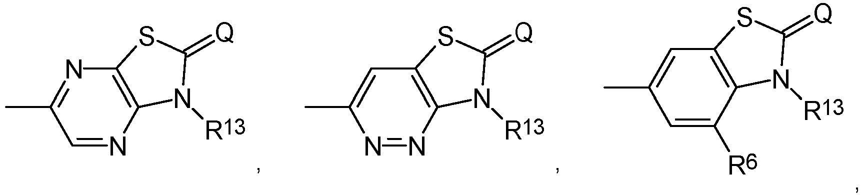 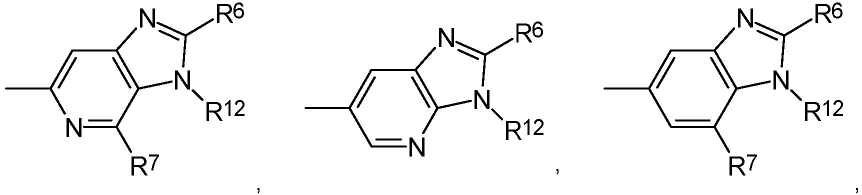 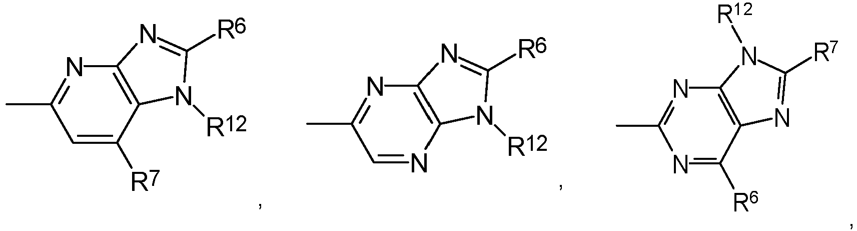 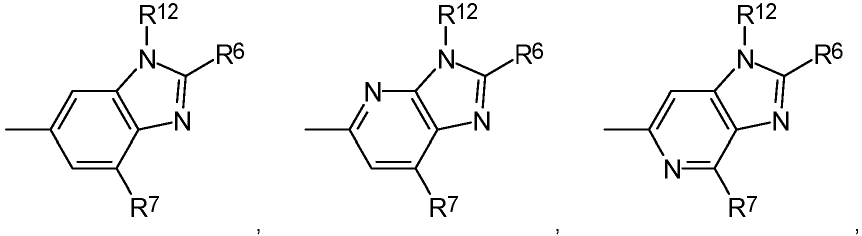 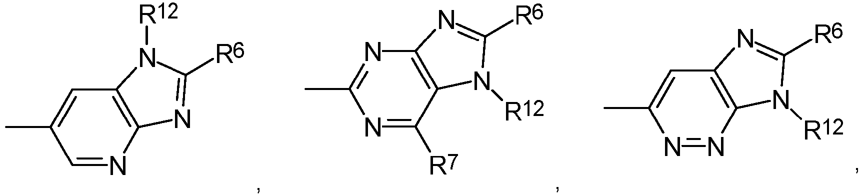 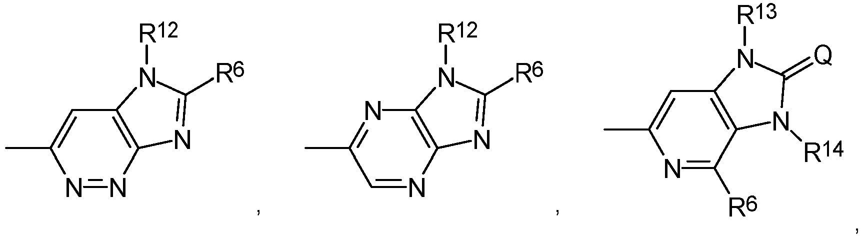 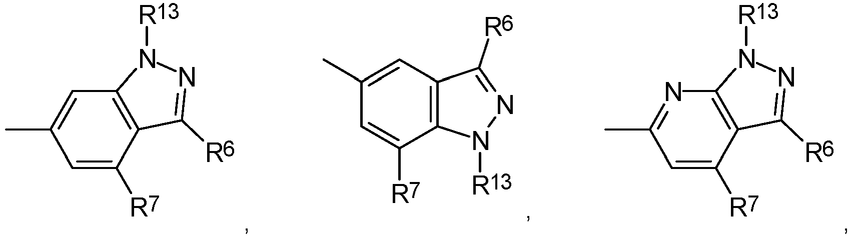 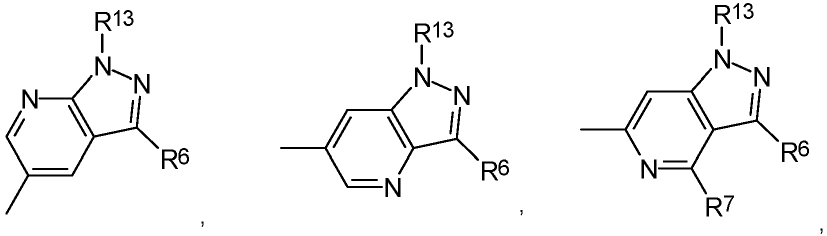 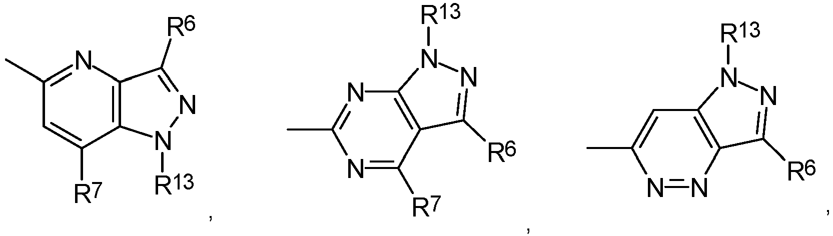 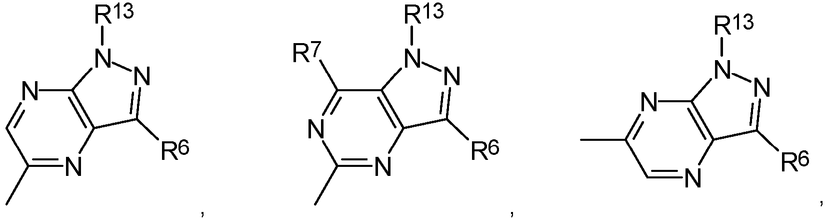 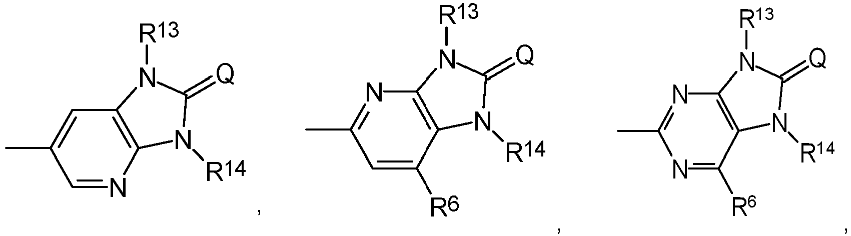 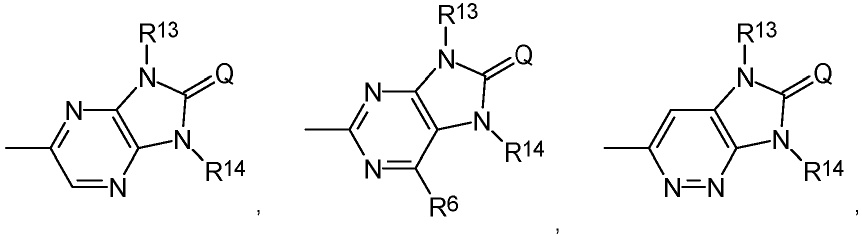 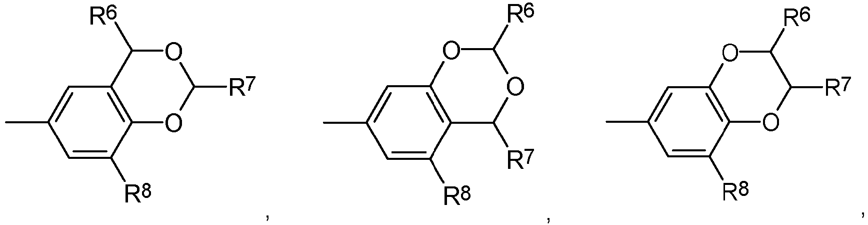 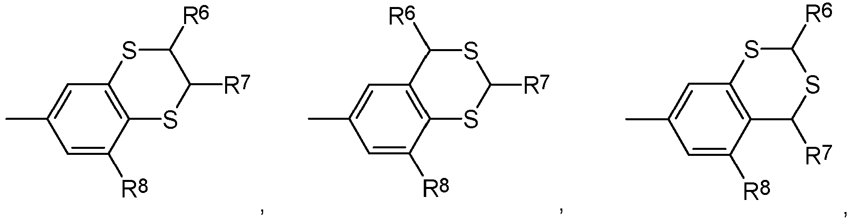 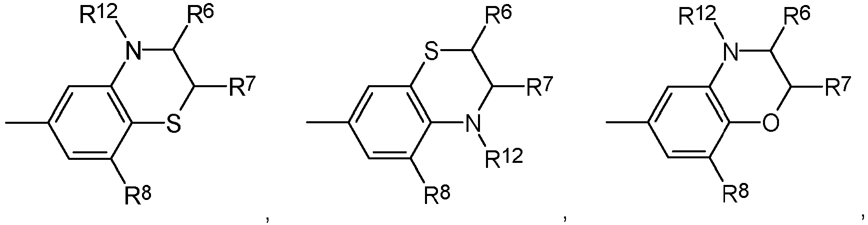 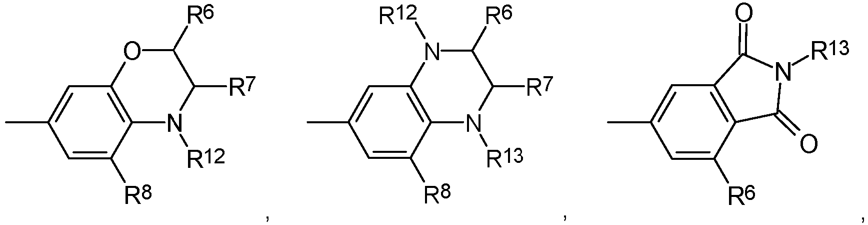 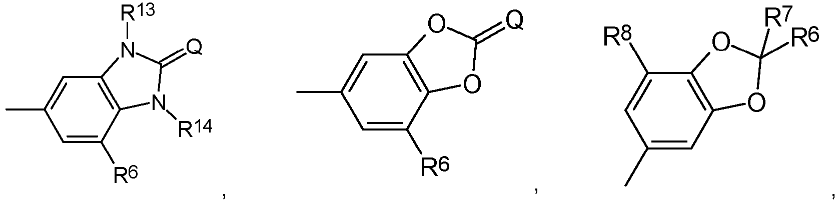 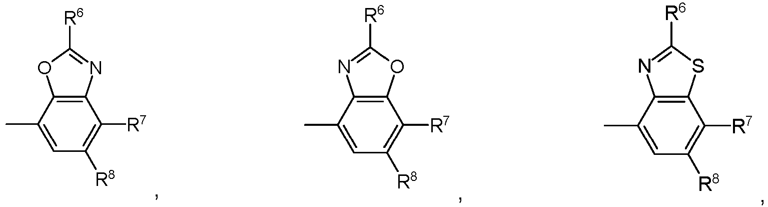 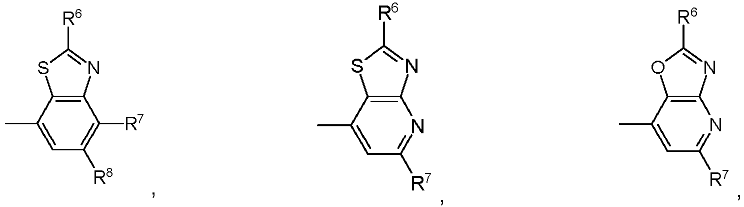 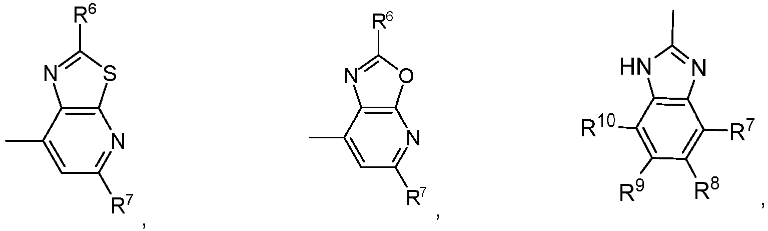 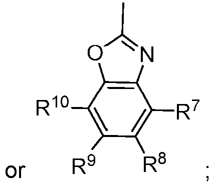
**Q** represents =O, =S, or =N-R¹²;
**R^{c}** represents -H, -OH, -CH₂-OH, -CHO, -CH₂CHO, -CH₂CH₂CHO, -C₂H₄-OH, - C₃H₆-OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH₂-OH, -CH₂O-C₂H₅, -CH₂-O-CH(CH₃)₂, -CH₂-O-CH(CH₂)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -Ch(OAc)-CH₂OH, -Ch(OAc)-Ch₂OAc, -CH(OH)-Ch₂OAc, -CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, -CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, -CH(N(CH₃)₂)CH₃, -CH(N(C₂H₅)₂)CH₃, -CH(NH₂)CH₂OH, -CH(NHCH₃)CH₂OH, -CH(NHC₂H₅)CH₂OH, -CH(N(CH₃)₂)CH₂OH, -CH(N(C₂H₅)₂)CH₂OH, -CH(NH₂)CH₂OCH₃, -CH(NHCH₃)CH₂OCH₃, -CH(NHC₂H₅)CH₂OCH₃, -CH(N(CH₃)₂)CH₂OCH₃, -CH(N(C₂H₅)₂)CH₂OCH₃, -CH(NH₂)CH₂OC₂H₅, -CH(NHCH₃)CH₂OC₂H₅, -CH(NHC₂H₅)CH₂OC₂H₅, -CH(N(CH₃)₂)CH₂OC₂H₅, -CH(N(C₂H₅)₂)CH₂OC₂H₅, -CH(NH₂)Ch₂OAc, -CH(NHCH₃)Ch₂OAc, -CH(NHC₂H₅)Ch₂OAc, -CH(N(CH₃)₂)Ch₂OAc, -CH(N(C₂H₅)₂)Ch₂OAc, -CH₂-CH(NHAc)CH₂OH, -CH₂-CH(NHAc)CH₂OCH₃, -CH₂-CH(NHAc)CH₂OC₂H₅, -CH₂-CH(NHCH₃)CH₃, -CH₂-CH(NHC₂H₅)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)Ch₂OAc, -CH₂-CH(NHCH₃)Ch₂OAc, -CH₂-CH(NHC₂H₅)Ch₂OAc, -CH₂-CH(N(CH₃)₂)Ch₂OAc, -CH₂-CH(N(C₂H₅)₂)Ch₂OAc, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -NHSO₂CH₃, -NHSO₂CF₃, -NHSO₂CH₂CF₃, -CH₂-NHSO₂CH₃, -CH₂-NHSO₂CF₃, -CH₂-NHSO₂CH₂CF₃, -C₂H₄-NHSO₂CH₃, -C₂H₄-NHSO₂CF₃, -C₂H₄-NHSO₂CH₂CF₃, -CONH₂, -CONHCH₃, -CONHC₂H₅,-CONHC₃H₇, -NH(C₂H₅), -N(C₂H₅)₂, -CH₂-NH(C₂H₅), - CH₂-N(C₂H₅)₂, -C₂H₄-NH(C₂H₅), -C₂H₄-N(C₂H₅)₂, -NO₂, -CH₂-NO₂, -C₂H₄-NO₂, -CH(OH)-NO₂, -CH(NO₂)-OH, -CO₂H, -CH₂-CO₂H, -C₂H₄-CO₂H, -CH=CH-CO₂H, -CO₂CH₃, -CO₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, -C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -O-Si(CH₃)₃, -O-Si(C₂H₅)₃, -CO-CHO, -CO-CO-CH₃, -C(OH)-CO-CH₃, -CO-C(OH)-CH₃, -CO-CH₂-CO-CH₃, -C(OH)-CH₂-CO-CH₃, -CO-CH₂-C(OH)-CH₃, -C(OH)-CH₂-C(OH)-CH₃, -F, -CI, -Br, -CH₂-F, -CHF₂, -CF₃, -CH₂Cl, -CH₂Br, -CH₂I, -C₂H₄-F, -CH₂-CHF₂, -CH₂-CF₃, -CF₂-CF₃, -O-CHF₂, -O-CF₃, -O-CH₂-CF₃, -O-C₂F₅, -CH₃, -CH₂CH₃, -C₃CH₇, -CH(CH₃)₂, -CH=CH₂, -C≡CH, -CH₂-CH=CH₂, or -CH₂-C≡CH, -CH₂-N₃, 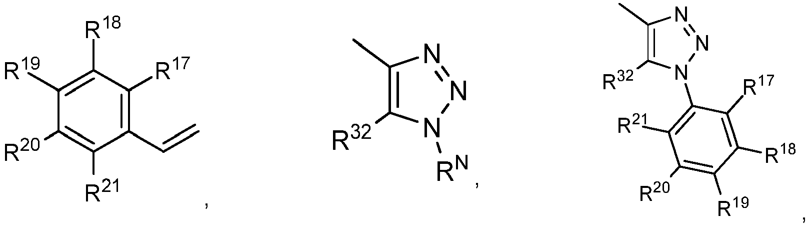 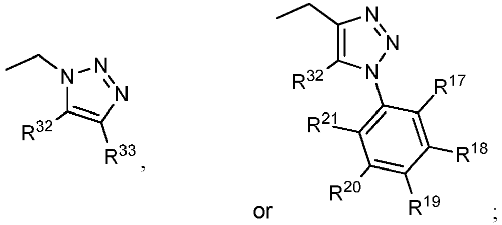
**R⁴⁰, R⁴¹, R⁴², R⁴³, R⁴⁴, R⁴⁵** represent independently of each other -H, -CH₃, -C₂H₅, -OH, - OCH₃, -F, -CI, -CN, -CF₃, or -NH₂, -NHMe, -NMe₂, or wherein **R⁴⁰** and **R⁴¹** or **R⁴²** and **R⁴³** or
**R⁴⁴** and **R⁴⁵** together form a double bond =CH₂ or a ketone =O.
**R¹ - R¹⁰** and **R¹⁷ - R²¹** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅,-CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -CH₂NH₂, -CH₂NHCH₃, -CH₂NHC₂H₅, -CH₂NHC₃H₇, -CH₂NH-cyclo-C₃H₅, -CH₂NH[CH(CH₃)₂], -CH₂NH[C(CH₃)₃], -CH₂N(CH₃)₂, -CH₂N(C₂H₅)₂, -CH₂N(C₃H₇)₂, -CH₂N(cyclo-C₃H₅)₂, -CH₂N[CH(CH₃)₂]₂, -CH₂N[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C=CH, -CH₂-CH(C≡CH)₂, 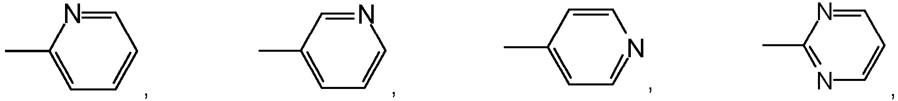 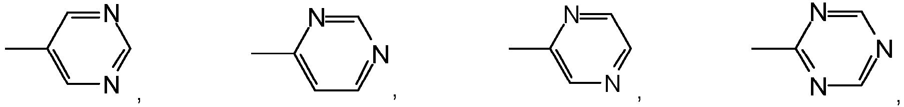 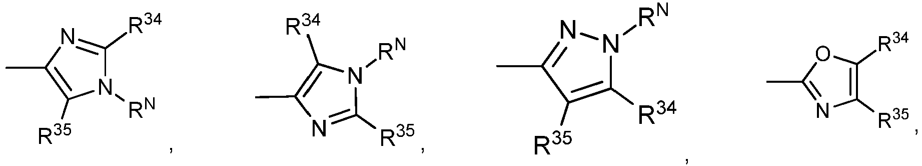 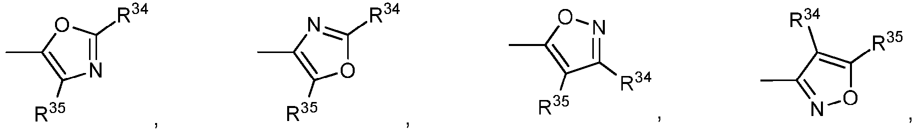 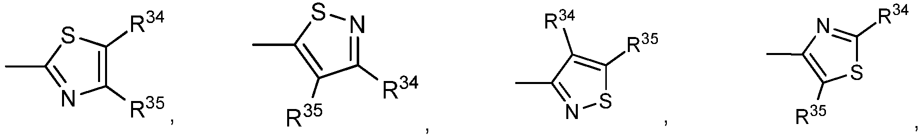 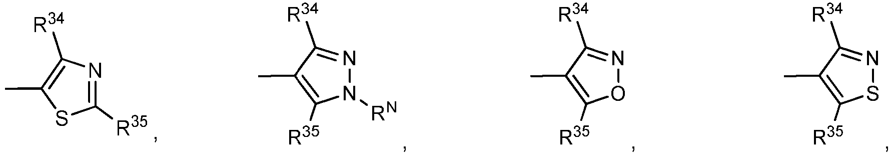 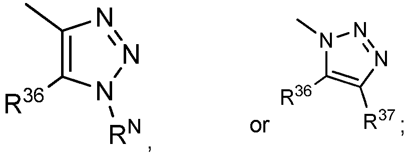
**R¹⁶, R³⁸, R³⁹** represent independently of each a lone pair, -H, -CH₃, -C₂H₅, -C₃H₇, -CH(CH₃)₂, -CH(CH₂)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH₂OH, -CH₂-SH, -CH₂CH(OH)CH₃, -OH, -OCH₃, -OCH₂CH₃, -OCH(CH₃)₂, -C₂H₄OH, -C₃H₆OH, -C₄H₈OH, -CH(CH₃)-C₂H₄OH, -C₅H₁₀OH, -CH₂-S-CH₃, -CH₂-CH₂-S-CH₃, -C₃H₆-S-CH₃, -CH₂OCH₃, -C₂H₄OCH₃, -C₃H₆OCH₃, -C₄H₈OCH₃, -CH(CH₃), -C₂H₄OCH₃, -C₅H₁₀OCH₃, -NH₂, -NHCH₃, -NHCH₂CH₃, -NHCH(CH₃)₂, -N(CH₃)₂, -N(CH₃)CH₂CH₃, -N(CH₂CH₃)₂, -N(CH₃)CH(CH₃)₂ -CH₂NH₂, - C₂H₄NH₂, -C₃H₆NH₂, -C₄H₈NH₂, -CH(CH₃)-C₂H₄NH₂, -C₅H₁₀NH₂, -CH₂-CH₂-CH₂-NH-C(NH)NH₂, -CH₂-CO₂H, -CH₂-CONH₂, -CH₂-CH₂-CO₂H, -CH₂-CH₂-CONH₂, -CH₂-CO₂CH₃, -CH₂-CONHCH₃, -CH₂-CON(CH₃)₂, -CH₂-CH₂-CO₂CH₃, -CH₂-CH₂-CONHCH₃, -CH₂-CH₂-CONH(CH₃)₂, -CH=CH-CO₂H, -CH=CH-CO₂CH₃, -CH=CH-CONHCH₃, -CH=CH-CONHC₂H₅, -CH=CH-CON(CH₃)₂, -CH=CH-CON(C₂H₅)₂, -CH₂-CH=CH-CO₂H, -CH₂-CH=CH-CO₂CH₃, -CH₂-CH=CH-CONHCH₃, -CH₂-CH=CH-CON(CH₃)₂, -CH₂-CH=CH-CONHC₂H₅, -CH₂-CH=CH-CON(C₂H₅)₂, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, - CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, - C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, - CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-Ph, 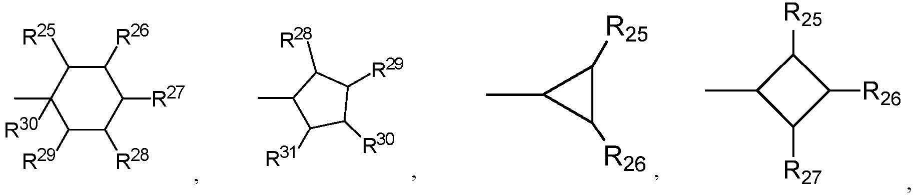 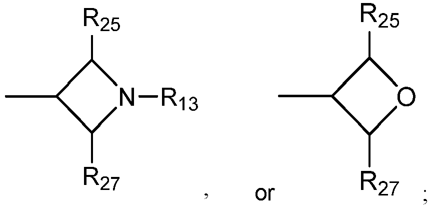
**R¹²- R¹⁴** represent independently of each other -H, -CH₂F, -CHF₂, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-C₆H₁₁, -cyclo-C₇H₁₃, -cyclo-C₈H₁₅, -SO₂CH₃, -Ph, -CH₂-Ph, -CH₂-CH₂-Ph, -CH=CH-Ph, -CPh₃, -CHO, -CO₂CH₃, -COCH₃ -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, or -CH₂-CH(C≡CH)₂;
**R²⁵-R³⁷** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -SO₂CH₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃**,** -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃,
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts.

The expression "prodrug" is defined as a pharmacological substance, a drug, which is administered in an inactive or significantly less active form. Once administered, the prodrug is metabolized in the body in vivo into the active compound.

The expression "tautomer" is defined as an organic compound that is interconvertible by a chemical reaction called tautomerization. Tautomerization can be catalyzed preferably by bases or acids or other suitable compounds.

In yet another embodiment of the present invention, wherein the compound according to the general formula 1 is selected from the group comprising or consisting of:
- Some embodiments, wherein substituents for R^{A} are selected from the group: 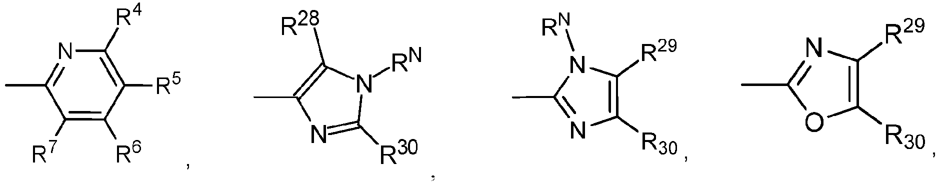 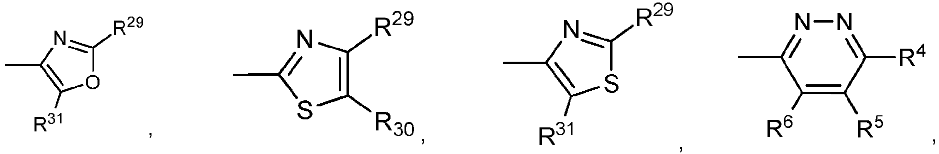 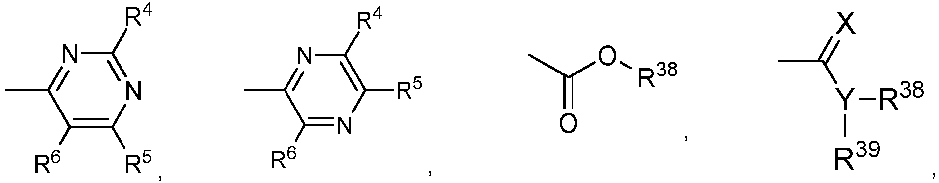 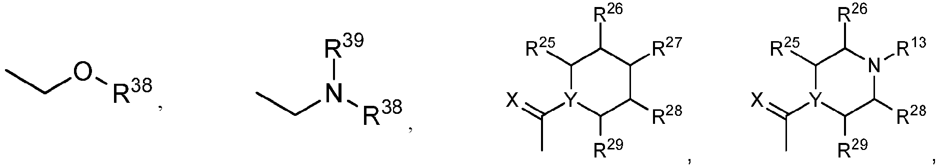 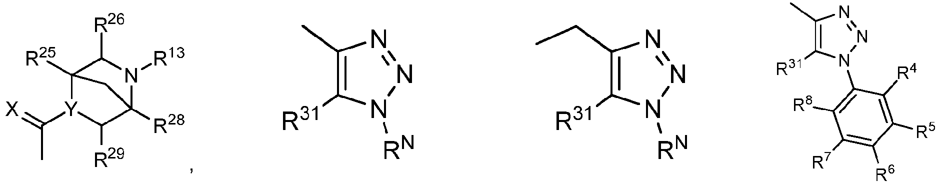 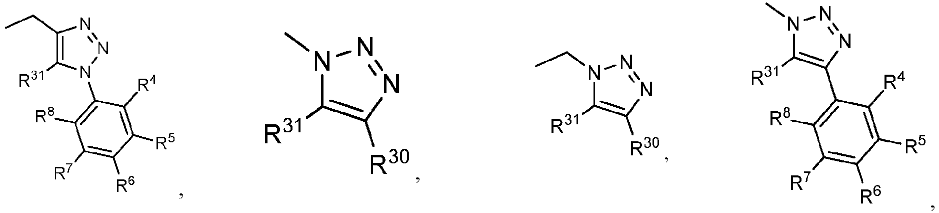 or 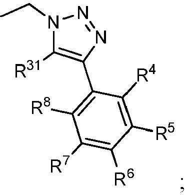 wherein R⁴ - R³⁹, R^{N} and X, Y have the meanings as defined in the general formula 1, in some embodiments R³⁸ and R³⁹ represent independently of each other -H, -CH₃, -C₂H₅, -C₃H₇, -CH(CH₃)₂, -CH(CH₂)₂, -C₄H₉, -CH₂-CH(CH₃)₂, -CH(CH₃)-C₂H₅, -C(CH₃)₃, -CH₂-Ph, -CF₃.
- In another embodiment R^{A} is selected from the group:
   -COOH, -COOCH₃, -COOC₂H₅, -COOC₃H₇, -CH₂NH₂, -CH₂NHCH₃, -CH₂NHC₂H₅, -CH₂NHC₃H₇, -CH₂N(CH₃)₂, -CH₂N(C₂H₅)₂, -CH₂N(C₃H₇)₂, -CONH₂, -CONHCH₃, -CONHC₂H₅, -CONHCH(CH₃)₂, -CONHC₃H₇, -CON(CH₃)₂, -CON(C₂H₅)₂, -CON(C₃H₇)₂;
- and in some embodiments R^{A} is selected from the group: -COOH, -COOCH₃, -COOC₂H₅, -CH₂NHCH₃, 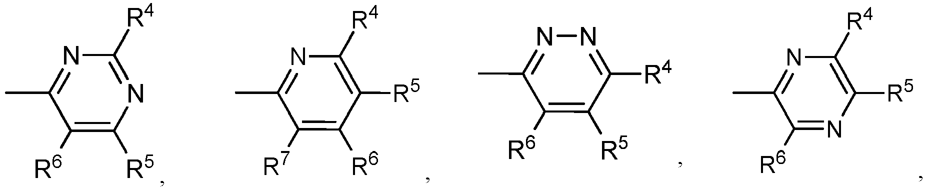 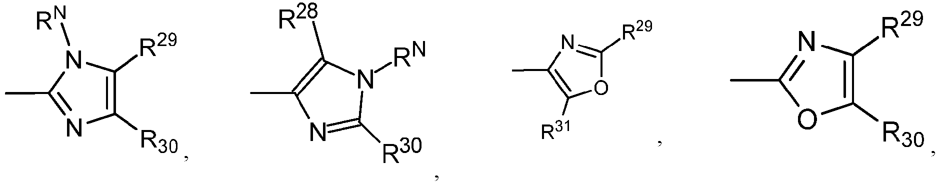 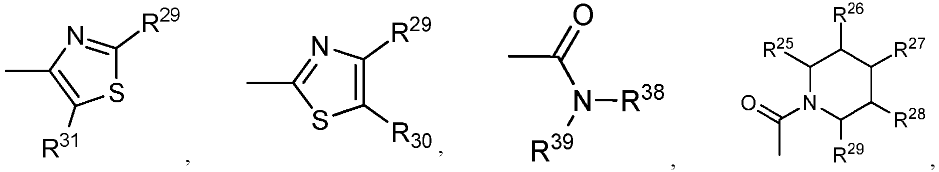 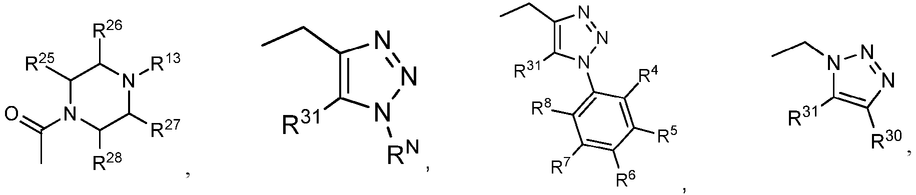 or 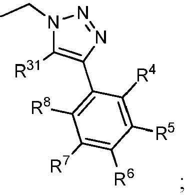
   wherein **R⁴** - **R⁸** have the meanings as defined in the general formula **1,** or in some embodiments **R⁴** - **R⁸** and **R²⁵** - **R³¹** represent independently of each other -H, -CH₃, -OMe, -F, and more preferably -H or -CH₃
   wherein **R³⁸** - **R³⁹** have the meanings as defined in the general formula **1,** or in some embodiments represent independently of each other -H, -CH₃, -C₂H₅, -C₃H₇, -CH(CH₃)₂, -CH(CH₂)₂, -C₄H₉, -CH₂-CH(CH₃)₂, -CH(CH₃)-C₂H₅, -C(CH₃)₃.
   wherein **R^{N}** has the meaning as defined in the general formula **1,** or in some embodiments represents -H, -COCH₃, -COC₂H₅, -COPh, -COCH₂Ph, -SO₂Ph, -SO₂CH₂Ph, -CH₃, -C₂H₅, -C₃H₇, -CH(CH₃)₂, -Ph, or -CH₂-Ph, and in further embodiments -H, -COPh, -SO₂Ph, -Ph, or -CH₂-Ph.

In a further embodiment compounds of the formula **1** are encompassed having a substituent **R^{A}** with a molecular weight of <200 g/mol, <100 g/mol, or <50 g/mol.

In further embodiments the substituents for **R^{B}** are selected from the group consisting of: 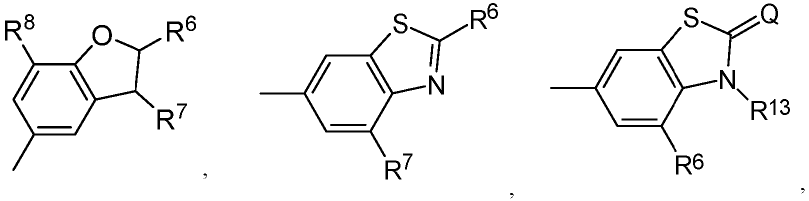 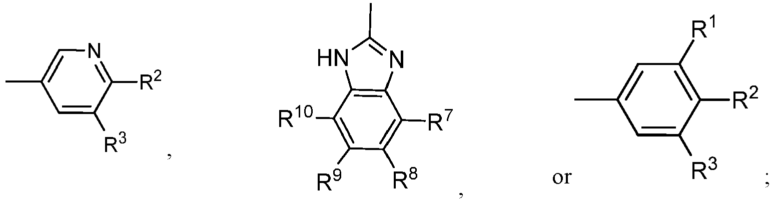 wherein **R¹** - **R³, R⁶ - R¹⁰, R¹³** and **Q** have the meanings as defined in the general formula 1;

In some embodiments **R¹ - R³**, **R⁶** - **R¹⁰**, **R¹³** and Q represent:
Q represents =O;
**R¹** - **R³** and **R⁷** - **R⁹** represent independently of each other -H, -F, -Cl, -Br, -I, -OH, -OCH₃, -OC₂H₅, -OC₃H₇, -CN, -CONH₂, -NHCOCH₃, -NHCOC₂H₅, -NHCOC₃H₇, -COOH, -COOCH₃, -COOC₂H₅, -COOC₃H₇, -Ph, 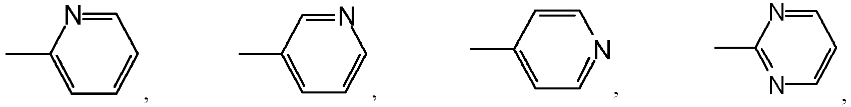 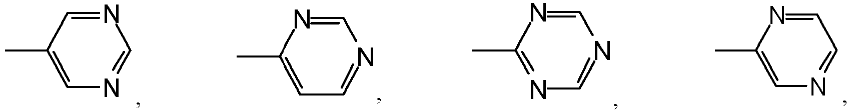 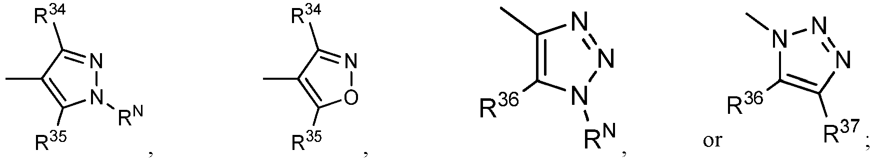
wherein **R¹⁰** represents -H;
wherein **R¹³** represents -H, -CH₃, or -C₂H₅, or-H;
wherein **R³⁴** - **R³⁵** represents -H or -CH₃,
wherein **R³⁶** - **R³⁷** and **R^{N}** have the meanings as defined in the general formula **1**
and in some embodiments one of **R¹ - R³** is different from hydrogen.

In other embodiments the compounds of the formula **1** are having one of the following substituents **R^{B}** : 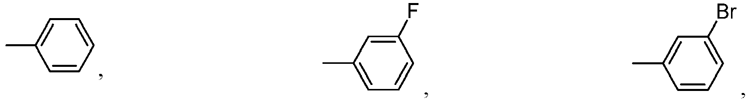 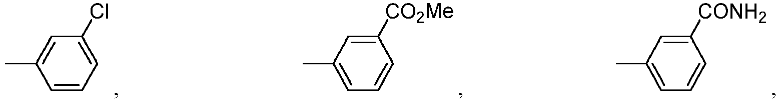 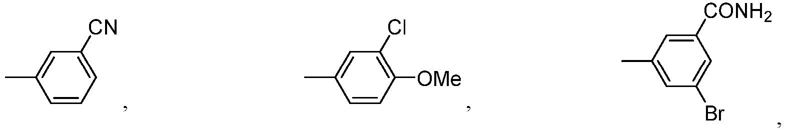 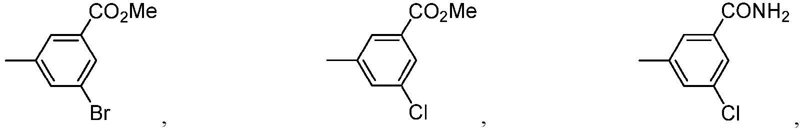 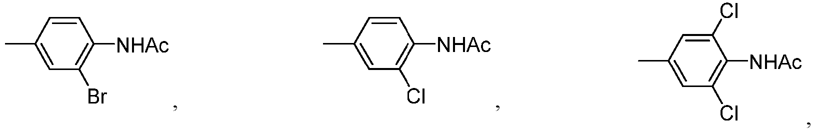 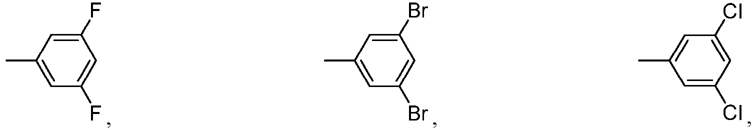 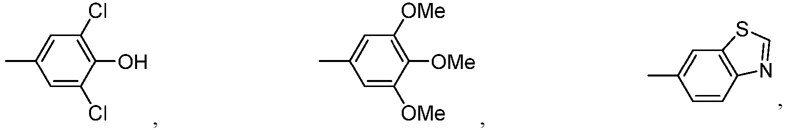 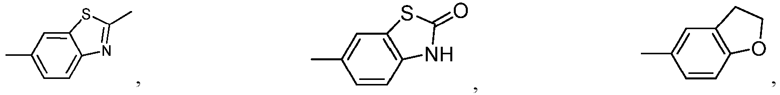 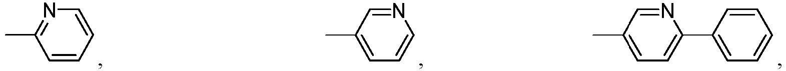 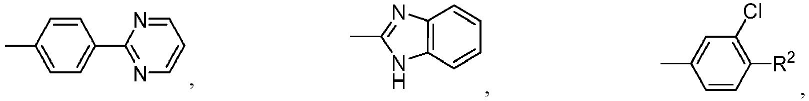 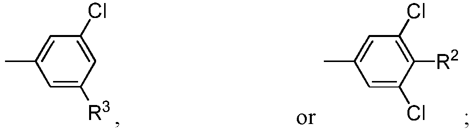
wherein **R²** represents 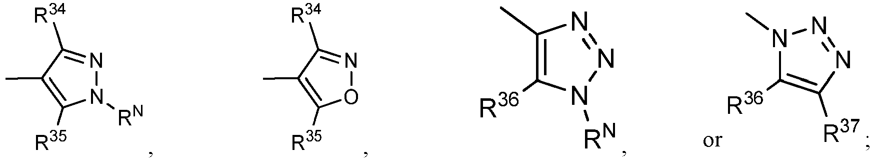
wherein **R³** represents 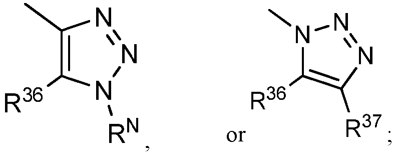
wherein **R³⁴** - **R³⁵** represents -H or -CH₃,
wherein **R³⁶** - **R³⁷** and **R^{N}** have the meanings as defined in the general formula **1**

In a further embodiment compounds of the formula **1** are encompassed having a substituent **R^{B}** with a molecular weight of <300 g/mol, <200 g/mol, or <130 g/mol.

In some embodiments compounds of formula **1** are encompassed having one of the following substituents **R^{C}:** -CH₂-OH, -CHO, -CH₂CHO, -CH₂CH₂CHO, -C₂H₄-OH, -C₃H₆-OH, -OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH(CH₂)₂, -CH₂-O-CH₂-OH, -CH₂O-C₂H₅, -CH₂-O-CH(CH₃)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -Ch(OAc)-CH₂OH, -Ch(OAc)-Ch₂OAc, -CH(OH)-Ch₂OAc, -CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, -CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, -CH(N(CH₃)₂)CH₃, -CH(N(C₂H₅)₂)CH₃, -CH(NH₂)CH₂OH, -CH(NHCH₃)CH₂OH, -CH(NHC₂H₅)CH₂OH, -CH(N(CH₃)₂)CH₂OH, -CH(N(C₂H₅)₂)CH₂OH, -CH(NH₂)CH₂OCH₃, -CH(NHCH₃)CH₂OCH₃, -CH(NHC₂H₅)CH₂OCH₃, -CH(N(CH₃)₂)CH₂OCH₃, -CH(N(C₂H₅)₂)CH₂OCH₃, -CH(NH₂)CH₂OC₂H₅, -CH(NHCH₃)CH₂OC₂H₅, -CH(NHC₂H₅)CH₂OC₂H₅, -CH(N(CH₃)₂)CH₂OC₂H₅, -CH(N(C₂H₅)₂)CH₂OC₂H₅, -CH(NH₂)Ch₂OAc, -CH(NHCH₃)Ch₂OAc, -CH(NHC₂H₅)Ch₂OAc, -CH(N(CH₃)₂)Ch₂OAc, -CH(N(C₂H₅)₂)Ch₂OAc, -CH₂-CH(NHAc)CH₂OH, -CH₂-CH(NHAc)CH₂OCH₃, -CH₂-CH(NHAc)CH₂OC₂H₅, -CH₂-CH(NHCH₃)CH₃, -CH₂-CH(NHC₂H₅)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)Ch₂OAc, -CH₂-CH(NHCH₃)Ch₂OAc, -CH₂-CH(NHC₂H₅)Ch₂OAc, -CH₂-CH(N(CH₃)₂)Ch₂OAc, -CH₂-CH(N(C₂H₅)₂)Ch₂OAc, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -CONH₂, -CONHCH₃, -CONHC₂H₅, -CONHC₃H₇, -NH(C₂H₅), -N(C₂H₅)₂, -CH₂-NH(C₂H₅), -CH₂-N(C₂H₅)₂, -C₂H₄-NH(C₂H₅), -C₂H₄-N(C₂H₅)₂, -CO₂H, -CH₂-CO₂H, -C₂H₄-CO₂H, -CH=CH-CO₂H, -CO₂CH₃, -CO₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, -C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -CH₂-F, -CH₂Cl, -CH₂Br, -CH₂I, -CHF₂, -CF₃, -C₂H₄-F, -CH₂-CF₃, -CF₂-CF₃, -O-CHF₂, -O-CF₃, -CH₃, -CH₂CH₃, -C₃CH₇, -CH(CH₃)₂, -CH=CH₂, -C≡CH, -CH₂-CH=CH₂, -CH₂-C≡CH, -CH₂-N₃, 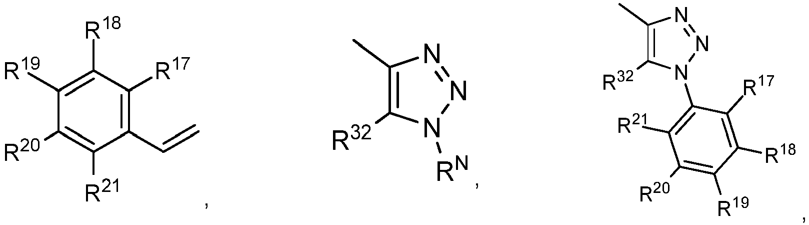 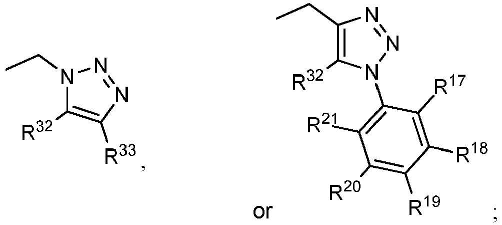 wherein R¹⁷ - R²¹, R³²- R³³ and R^{N} have the meanings as defined in the general formula 1;

In other embodiments R^{C} represents: -OH, -CH₂-OH, -C₂H₄-OH, -C₃H₆-OH, -CHO, -CH₂CHO, -CH₂CH₂CHO, -O-CH₃, -O-C₂H₅, -O-C₃H₇, -O-CH(CH₃)₂, -CONH₂, -CONHCH₃, -CONHC₂H₅, -CONHC₃H₇, -CO-CH₃, -CO-C₂H₅, -CO-C₃H₇, -CO-CH(CH₃)₂, -CO₂H, -CH₂-CO₂H, -C₂H₄-CO₂H, -O-CH₂-OH, -O-CH₂-O-CH₃, -O-C₂H₄-O-CH₃, -CH₂-O-CH₃, -CH₂-O-CH(CH₂)₂, -CH₂-O-CH₂-OH, -CH₂O-C₂H₅, -CH₂-O-CH(CH₃)₂, -CH₂-O-C₃H₇, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -NH(C₂H₅), -N(C₂H₅)₂, -CH₂-NH(C₂H₅), -CH₂-N(C₂H₅)₂, -C₂H₄-NH(C₂H₅), -C₂H₄-N(C₂H₅)₂, -CH₂-F, -CH₂Cl, -CH₂Br, -CH₂I, -CHF₂, -CF₃, -C₂H₄-F, -CH₂-CF₃, -CF₂-CF₃, -CH₃, -CH₂CH₃, -C₃CH₇, -CH(CH₃)₂, -CH=CH₂, -C≡CH,-CH₂-CH=CH₂, -CH₂-C≡CH, -CH₂-N₃, In even further embodiments **R^{C}** represents: -OH, -NH₂, -CH₂F, -CHF₂, -CH₂Br, -CH₂I, -CH₂CH₃, -CH=CH₂, -CH₂OH, -CHO, -CO₂H, -CONH₂, -COCH₃, -OCH₃, -OCH₂CH₃, -OCH(CH₃)₂, -OCH₂OCH₃, -OC₂H₄OCH₃, -CH₂OCH₃, -CH₂OCH₂CH₃, -CH₂-O-CH(CH₂)₂, -CH₂CH₂OH, -CH₂CHO, -CH₂CH₂CHO, -CH₂NH₂, -CH₂NHCH₃, -CH₂N(CH₃)₂, -CH(OH)CH₃, -C(OH)(CH₃)₂, -CH₂CH(OH)CH₃, -CH(OH)CH_{2O}H, -C≡CH,-CH₂-N₃

In even further embodiments **R^{C}** substituents comprise a hydroxyl group or an alkoxy group such as -CH₂OH, -CH₂CH₂OH, -CH(OH)CH₃, -C(OH)(CH₃)₂, -CH₂CH(OH)CH₃, - CH(OH)CH₂OH -CH₂OCH₃, -CH₂-O-CH(CH₂)₂

In an alternative embodiment compounds of the formula 1 are encompassed having a substituent **R^{c}** with a molecular weight of <200 g/mol, <100 g/mol, or <50 g/mol.

In some embodiments substituents for **R⁴⁰**, **R⁴¹, R⁴²**, **R⁴³, R⁴⁴**, **R⁴⁵** are -H, -CH₃, -C₂H₅, -OH, -OCH₃, -F, -Cl, -NH₂.

In further embodiments substituents for **R⁴⁰**, **R⁴¹, R⁴²**, **R⁴³, R⁴⁴**, **R⁴⁵** are selected from the following: -H, -CH₃, -OH, -OCH₃, -F.

In yet a further embodiment compounds of the formula **1** are encompassed having substituents **R^{A}, R^{B}, R^{C}**, **R⁴⁰**, **R⁴¹, R⁴²**, **R⁴³, R⁴⁴**, **R⁴⁵** with a combined molecular weight of <400 g/mol, <300 g/mol, or <250 g/mol.

In another embodiment the general formula **1** is formula **2** and **3:** 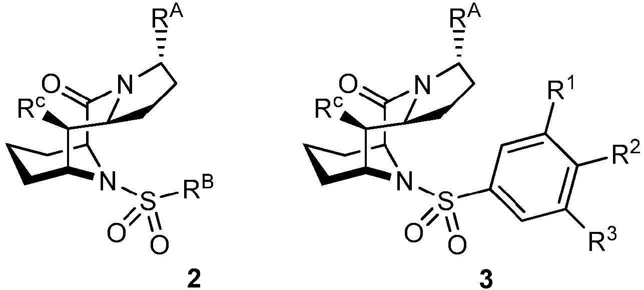 wherein and the substituents **R^{A}, R^{B}, R^{C}** and **R¹** - **R³** have the meanings as defined for formula 1 herein.

In one embodiment the compounds disclosed herein do have a K_{d} [nM] to FK506-binding proteins selected from the group consisting of human FKBP12, FKBP12.6, FKBP51 and FKBP52 and/or bacterial homologs LpMip, CtMip, CpMip, NgMip, KpMip, BpMip, TcMip, EcFKLB, EcFKPA, PaFKLB, PaFKPA, AbFKLB, and AbFKPA, as well as combinations thereof, of 100 or less, of 90 or less, of 80 or less, of 70 or less, of 60 or less, of 50 or less, of 40 or less, of 30 or less, of 20 or less, or of 10 or less.

In one embodiment the compounds disclosed herein do have a IC50 [nM] measured by nanoBRET to FK506-binding proteins selected from the group consisting of human FKBP12, FKBP12.6, FKBP51 and FKBP52, as well as combinations thereof, of 500 or less, of 400 or less, of 300 or less, of 250 or less, of 200 or less, of 100 or less, or 50 or less.

### Synthetic Methods

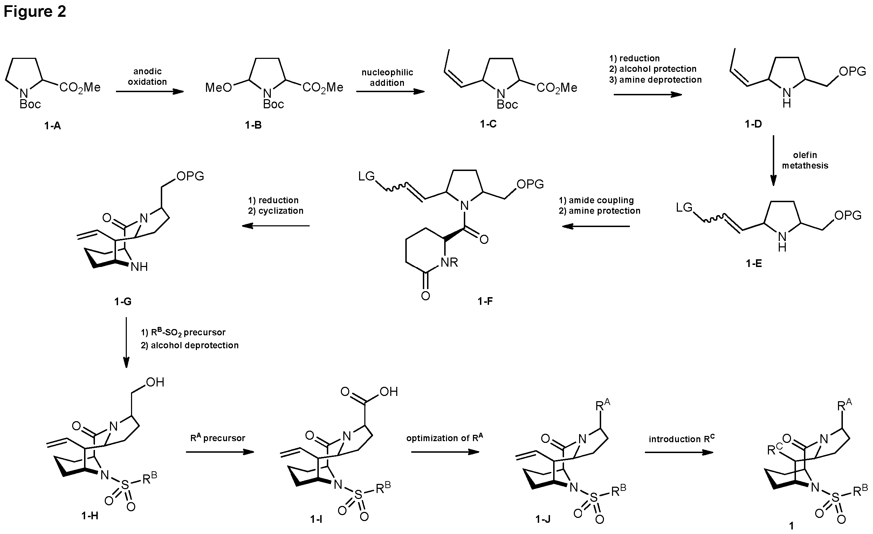

Compounds of the general formula 2 can be prepared according to the following synthetic route depicted in Figure 2. Accordingly, the building block 1-C can be prepared by metoxylation at C5 position followed by nucleophilic substitution. The 2,5-disubstituted pyrrolidine 1-C undergoes a sequence of reactions comprised of ester reduction and functional group protection/deprotection manipulations which allows to obtain a substrate for olefin metathesis. An amine compound 1-E which has a suitable leaving group (LG) such as trimethylsilyl (TMS) and a carbon-carbon double bond in allyl position to the LG reacts with carboxylic group of 6-carboxy-2-piperidone. Subsequently, this compound undergoes a selective reduction and cyclization reactions upon which the leaving group LG is detached from the starting molecule and the amine group is deprotected. This leads to the formation of the tricyclic compound 1-G. Obtained intermediate can subsequently be reacted with a suitable precursor for the moiety -SO₂-R^{B}. The R^{A} precursor 1-I synthesized by oxidation of deprotected alcohol 1-H gives a starting point for further optimization of R^{A} substituent. The compound of the general formula 1 can be obtained by suitable transformation reactions the vinyl group of the tricyclic sulfonamides.

### Pharmaceutical Composition

The present invention also comprises pharmaceutically acceptable salts of the compounds according to the general formula **1,** all stereoisomeric forms of the compounds according to the general formula **1** as well as solvates, especially hydrates or prodrugs thereof.

In case, the inventive compounds bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH₄OH, tetraalkyl-ammonium hydroxide, lysine or arginine and the like. Salts may be prepared in a conventional manner using methods well known in the art, for example by treatment of a solution of the compound of the general formula 1 with a solution of an acid, selected out of the group mentioned above.

Some of the compounds of the present invention may be crystallised or re-crystallised from solvents such as aqueous and organic solvents. In such cases solvates may be formed. This invention includes within its scope stoichiometric solvates including hydrates as well as compounds containing variable amounts of water that may be produced by processes such as lyophilisation.

Certain compounds of the general formula **1** may exist in the form of optical isomers if substituents with at least one asymmetric center are present, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula **1** contains an alkene moiety, the alkene can be presented as a cis- or trans-isomer or a mixture thereof. When an isomeric form of a compound of the invention is provided substantially free of other isomers, it will preferably contain less than 5% w/w, more preferably less than 2% w/w and especially less than 1% w/w of the other isomers.

Therefore, one aspect of the present invention is that the compounds according to the general formula **1** are suitable for use as ligand of FK506-binding proteins (FKBP).

In one embodiment these compounds are very potent binders to FK506-binding protein 12 (FKBP12) and FK506-binding protein 12.6 (FKBP12.6) with no immunosuppressive side effects and are therefore a valuable agents for blocking the function of FKBP12 and FKBP12.6.FKBP12 and FKBP12.6 have been implicated in cardiac diseases due to their role as regulators of ryanodine receptors and in haematological diseases due to their role as regulators of receptors of the TGRβ/ALK family. Consequently, FKBP12 and FKBP12.6 ligands are useful for the treatment of diseases characterized by an aberrant activity of these receptors.

Another aspect of the present invention relates to the use of the inventive FKBP51/52 ligand derivatives as drugs, i.e. as pharmaceutically active agents applicable in medicine.

In one embodiment said compound is suitable for use as ligand of the FK506-binding protein 51 (FKBP51) and/or the FK506-binding protein 52 (FKBP52).

FKBP51 has been implicated in numerous in human diseases. Consequently, FKBP51 is a target which is addressed in order to prevent and/or treat the diseases disclosed in the aforementioned literature.

Thus, FKBP51 and/or FKBP 52 ligand compounds of the present invention can be used as pharmaceutically active agent in medicine.

Preferred, the FKBP51/52 ligand compounds of the present invention can be used for treatment, or for the preparation of a pharmaceutical formulation for prophylaxis and/or treatment of these FKBP51/52-associated diseases such as depression, obesity or chronic pain.

In one embodiment said compound is suitable for use as ligand of bacterial Mip proteins such as LpMip, CtMip, CpMip, BpMip, TcMip, EcMip, PaMip, AbMip, KpMip, NgMip. These Mips have been implicated in the infectivity or intracellular replication of the bacterial pathogens. Consequently, Mip ligands are useful antiinfective agents, e.g. for the treatment of Legionnaire's disease, Chagas' disease or infections by *Chlamydiae* or *Burkholderiae* species.

The inventive compound of any one of formula **1,** subformula **2** and/or subformula **3** is used in the manufacture of a medicament or of a pharmaceutical composition for the treatment and/or prevention of FKBP- or Mip-associated diseases.

Another aspect of the present invention relates to a method of treating FKBP- or Mip-associated diseases comprising administration a therapeutically effective amount of at least one inventive compound or a pharmaceutical composition comprising at least one inventive compound.

These FKBP- or Mip-associated diseases include psychiatric and neurodegenerative diseases, disorders and conditions, for metabolic diseases such as localized adiposity or obesity, for sleep disorders, neuroprotection or neuroregeneration, for the treatment of neurological disorders, for the treatment of diseases relating to neurodegeneration, for the treatment of cancers such as malignant melanoma, multiple myeloma, or acute lymphoblastic leukaemia and especially steroid-hormone dependent cancers such as prostate cancer, for the treatment of glucocorticoid hyposensitivity syndromes and for peripheral glucocorticoid resistance, for asthma, especially steroid-resistant asthma, and for the treatment of infectious diseases, for stimulating neurite growth or neuroregeneration, for neuroprotection, for the use as wound healing agents for treating wounds resulting from injury or surgery; for the use in limiting or preventing haemorrhage or neovascularization for treating macular degeneration, and psychiatric disorders (such as depression or post-traumatic stress disorder), metabolic disorders (such as obesity or diabetes), infective disorders (such as Legionnaire's disease or Chagas' diseases), neurological disorders (such as Alzheimer's diseases or Parkinson's diseases) and haematological disorders (such as hereditary haemorrhagic telangiectasia or pulmonary arterial hypertension) as well as pain diseases (such as chronic neuropathic pain) and cancers (such as prostate cancer, melanoma, multiple myeloma, or glioblastoma).

The FKBP51 and/or FKBP52 ligand compounds of the present invention are preferably suitable for treatment, or for the preparation of a pharmaceutical formulation for prophylaxis and treatment of psychiatric diseases. It is especially preferred if these psychiatric diseases are an affective disorder (ICD-10 classification: F30-F39) or an anxiety disorder.

Affective disorder is a mental disorder characterized by dramatic changes or extremes of mood. The affective disorder according to the invention is selected from the group comprising or consisting of depression, bipolar disorder, mania, substance induced mood disorder and seasonal affective disorder (SAD). Among the psychiatric diseases and disorders, the most preferred is depression, the most commonly diagnosed psychiatric disorder.

The anxiety disorder according to the invention is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

Among the hundreds of different neurodegenerative disorders, the attention has been given only to a handful, including Alzheimer's Disease, Parkinson's Disease, and amyotrophic lateral sclerosis.

Among the glucocorticoid hyposensitivity syndromes, the attention has been given to the group of related diseases enclosing resistant asthma, eosinophilic esophagitis, AIDS, rheumatoid arthritis, hypertension and diabetes, metabolic syndrome or obesity.

Among the cancers, the attention has been given to malignant melanoma, acute lymphoblastic leukaemia, gliomas, idiopathic myelofibrosis, pancreatic and breast cancers, steroid-hormone dependent cancers or prostate cancer.

Among the hundreds of infectious diseases, the attention has been given to malaria and the Legionnaires' disease and Chlamydia infections.

Among the metabolic disorders, attention has been given to obesity and type 2 diabetes.

Among the neurological disorders, attention has been given to neuropathic pain and fibromyalgia.

Among the haematological disorders, attention has been given to hereditary haemorrhagic telangiectasia or pulmonary arterial hypertension.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and colouring agents may also be incorporated into the tablet or capsule. Powders and tablets may contain about 5 to about 95 weight-% of the benzothiophene-1,1-dioxide derived compound and/or the respective pharmaceutically active salt as active ingredient.

Suitable binders include starch, gelatine, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may comprise an additional pharmaceutically active compound or drug. The pharmaceutically active compound or drug may belong to the group of glucocorticoids. Thus, an embodiment of the current invention comprises the administration of a compound of the current invention in addition to a co-administration of glucocorticoids.

Moreover, the pharmaceutical compositions of the present invention may be formulated in sustained release form to provide the rate-controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatines or starch for holding or containing compositions comprising the active ingredient(s). Capsules with hard shells are typically made of blended of relatively high gel strength gelatines from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscarmellose, alginates such as alginic acid and sodium alginate, clays such as bentonites, and effervescent mixtures. The amount of disintegrant in the composition may range from about 2 to about 20 weight-% of the composition, more preferably from about 5 to about 10 weight %.

Binders are substances, which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatine and tragacanth, derivatives of seaweed such as alginic acid, sodium alginate and ammonium calcium alginate, cellulose materials such as methylcellulose, sodium carboxymethylcellulose and hydroxypropylmethyl-cellulose, polyvinylpyrrolidone, and inorganic compounds such as magnesium aluminium silicate. The amount of binder in the composition may range from about 2 to about 20 weight-% of the composition, preferably from about 3 to about 10 weight %, and more preferably from about 3 to about 6 weight %.

Lubricants refer to a class of substances, which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water-soluble lubricants such as sodium chloride, sodium benzoate, sodium acetate, sodium oleate, polyethylene glycols and D, L-leucine. Lubricants are usually added at the very last step before compression since they must be present at the surface of the granules. The amount of lubricant in the composition may range from about 0.2 to about 5 weight-% of the composition, preferably from about 0.5 to about 2 weight %, and more preferably from about 0.3 to about 1.5 weight-% of the composition.

Glidants are materials that prevent caking of the components of the pharmaceutical composition and improve the flow characteristics of granulate so that flow is smooth and uniform. Suitable glidants include silicon dioxide and talc. The amount of glidant in the composition may range from about 0.1 to about 5 weight-% of the final composition, preferably from about 0.5 to about 2 weight %.

Colouring agents are excipients that provide coloration to the composition or the dosage form. Such excipients can include food grade dyes adsorbed onto a suitable adsorbent such as clay or aluminium oxide. The amount of the colouring agent may vary from about 0.1 to about 5 weight-% of the composition, preferably from about 0.1 to about 1 weight %.

Said pharmaceutical compositions may further comprise at least one FKBP ligand of the general formula 1.

The pharmaceutical compositions may further comprise at least one further active agent. It is preferred if this active agent is selected from the group consisting of anti-depressant and other psychotropic drugs. It is further preferred if the anti-depressant is selected from amitriptyline, aminoxide clomipramine, doxepin, duloxetine, imipramine trimipramine, mirtazapine, reboxetine, citalopram, fluoxetine, moclobemide and sertraline.

### Definitions

FKPB (FK506-binding protein) ligands are a class of organic compounds that bind to FKBPs or inhibit the activity of FKPB proteins. FKPB proteins are involved in various cellular processes, including immune response regulation. Enantiomers of FKPB ligands may have different effects on the binding affinity and activity of these proteins due to their mirrored structures, potentially resulting in differences in biological activity, pharmacokinetics, and toxicity. Therefore, it is important to consider the stereochemistry of FKPB ligands when studying their properties and effects.

Stereoisomeric forms of an organic compound, including FKPB ligands, refer to different spatial arrangements of atoms within molecules that result in distinct structural forms but have the same molecular formula and connectivity of atoms. Stereoisomers arise due to differences in the spatial orientation of atoms or groups around one or more stereo-centres within a molecule.

In the case of FKPB ligands, which are organic compounds designed to inhibit FK506-binding proteins, stereoisomeric forms may include:
An "enantiomer", in the context of the present disclosure, refers to one of a pair of molecules that are mirror images of each other but cannot be superimposed onto one another.

Enantiomers have identical physical and chemical properties in an achiral environment but can exhibit different interactions in chiral environments, such as biological systems. This is because biological systems often contain chiral molecules or receptors, and enantiomers can interact differently with these systems due to their mirrored structures.

"Diastereoisomers" as disclosed herein are a type of stereoisomers that are not mirror images of each other and exist when a molecule has two or more chiral centres but differs in configuration at some, but not all, of these centres. In other words, diastereoisomers have different spatial arrangements of atoms around at least one chiral centre but share the same configuration at others.

"Anomers" as disclosed herein are a specific type of stereoisomers that occur in cyclic forms of carbohydrates, particularly when they undergo intramolecular cyclization to form hemiacetals or hemiketals. Anomers differ from one another in the configuration around the anomeric carbon, which is the carbon atom that becomes a new chiral centre during the cyclization process. Thus, if an FKPB-ligand were to undergo cyclization reactions involving a hemiacetal or hemiketal formation, it might produce anomeric forms if it contained a chiral centre at the site of cyclization.

"Prodrugs" are defined herein as inactive or less active forms of drugs that are designed to undergo metabolic or chemical transformation in the body to become active pharmacological agents. They are typically synthesized by chemically modifying the parent drug molecule to render it inactive or less active. Once administered, prodrugs undergo specific enzymatic or chemical reactions, such as hydrolysis, oxidation, or reduction, to release the active drug moiety.

In the context of FKPB ligands, prodrugs could be designed to improve pharmacokinetic properties, enhance bioavailability, or reduce toxicity. For example, if a particular FKPB ligands has poor oral bioavailability due to rapid metabolism or poor absorption, a prodrug form could be developed that is more readily absorbed or metabolized into the active form in vivo. Similarly, prodrugs could be designed to target specific tissues or cells more effectively or to prolong the duration of action of the active drug.

Overall, prodrugs offer a strategy to optimize the therapeutic properties of FKPB ligands and other organic compounds by modifying their chemical structure to improve pharmacokinetics, bioavailability, and target specificity.

In the context of this disclosure, "deoxy-forms" typically refer to molecules that lack one or more oxygen atoms compared to their corresponding fully oxygenated counterparts. The prefix "deoxy-" indicates the removal or absence of oxygen. It includes modifications to the structure of the ligand involving the removal of oxygen-containing functional groups in order to alter the pharmacological properties of the compound, such as its potency, selectivity, or metabolic stability.

In the context of this disclosure, a "racemate" (or racemic mixture) refers to a mixture of equal amounts of two enantiomers of a chiral compound. Enantiomers are mirror-image isomers of each other that cannot be superimposed onto each other. When a chiral compound exists as a racemate, it means that both enantiomers are present in equal proportions, resulting in no overall optical activity (no net rotation of plane-polarized light). In the context of FKBP ligands or any other organic compound, if the compound contains one or more chiral centres and can exist as enantiomers, a racemate would consist of an equal mixture of both enantiomers. Racemates are commonly encountered in organic chemistry, and they may exhibit properties that are intermediate between those of the individual enantiomers. However, it's essential to note that racemates may also have different pharmacological properties compared to their individual enantiomers due to interactions with chiral biological receptors or enzymes.

In this disclosure, a "tautomer" refers to one of two or more isomeric forms of a compound that readily interconvert through a chemical reaction known as tautomerization. Tautomerization involves the migration of a proton and the rearrangement of bonds within the molecule, resulting in a structural change. The two primary types of tautomers are keto-enol tautomers and aldehyde-ketone tautomers, although other types also exist. Keto-enol tautomerization involves the reversible interconversion between a keto form (containing a carbonyl group) and an enol form (containing a hydroxyl group attached to a carbon-carbon double bond). Aldehyde-ketone tautomerization involves the reversible interconversion between an aldehyde and a ketone, typically involving an intramolecular proton transfer. Tautomerism can affect the chemical and biological properties of FKPB ligands, potentially influencing factors such as solubility, reactivity, and binding affinity to target proteins.

In this context, a "solvate" refers to a type of solid compound formed when solvent molecules are incorporated into the crystal lattice of a solid organic molecule. Solvates are formed through the interaction between solvent molecules and the solute molecules during the crystallization process. Solvates can be categorized based on the type of interaction between the solvent molecules and the solute molecules. Common types of solvates include:
Solvates with solvent molecules physically trapped within the crystal lattice without any significant chemical interaction with the solute molecules. These solvates are often referred to as nonstoichiometric solvates.

Solvates where solvent molecules form specific interactions (such as hydrogen bonds or dipole-dipole interactions) with functional groups on the solute molecules. In these cases, the solvate may contain a defined ratio of solvent molecules to solute molecules, resulting in a stoichiometric solvate.

"Pharmaceutically acceptable salts" refer herein to salts of organic compounds that are suitable for pharmaceutical use. In the context of FKBP-ligands or any other pharmaceutical compound, these salts are formed by reacting the active pharmaceutical ingredient (API) with an appropriate acid or base to produce a salt that is pharmaceutically acceptable.

The choice of counterion (the ion that pairs with the charged API to form the salt) is critical in producing a pharmaceutically acceptable salt. Common counterions used to form salts include inorganic acids (e.g., hydrochloric acid, sulfuric acid) for basic drugs and inorganic bases (e.g., sodium hydroxide, potassium hydroxide) for acidic drugs. However, organic acids and bases can also be used to form salts.

The formation of pharmaceutically acceptable salts can offer several advantages, including:
- Improved solubility: Salt formation can enhance the solubility of the API, which can be particularly beneficial for poorly water-soluble compounds, thereby improving bioavailability.
- Stability: Salt forms of APIs can exhibit improved stability, both in solid-state and in solution, compared to the free base or acid forms.
- Dosing flexibility: Different salts of the same API may have different physicochemical properties, allowing for flexibility in formulation and dosing.
- Taste masking: Some salts may have a more favourable taste profile than the parent compound, making them more palatable for oral administration.

"Molecular glue" as used herein refers to the small molecules of the present disclosure that possess the ability to promote interactions between proteins or other biomolecules that do not typically interact under normal cellular conditions. These molecules act as bridges or facilitators, bringing together proteins that may not naturally bind to each other, thereby influencing various cellular processes. Molecular glues have potential applications in modulating protein-protein interactions (PPI) and developing novel therapeutic agents. They can be utilized to target specific proteins involved in diseases such as cancer, neurodegenerative disorders, and infectious diseases.

In one embodiment any designated substituent, such as R¹, R², R³, ... (or any other number) can only be one specific and selected substituent within in one compound. Thus, R¹ cannot represent in the same compound two different substituents such as -CH₃ and -COOH.

### Description of the Drawings

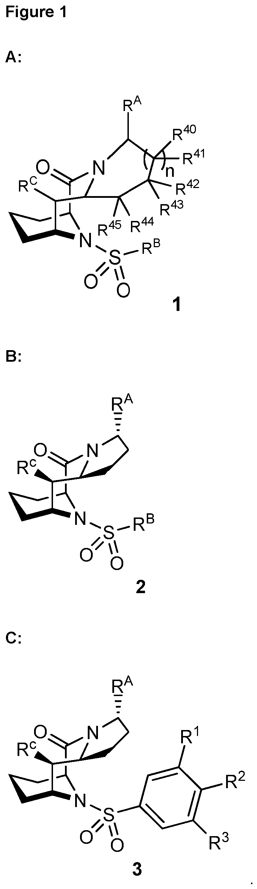

**Figure 1** - Depiction of the general formula of compounds according to the present invention. A: Compound of the general formula 1; B: Compound of the general formula 2; C: Compound of the general formula 3.
**Figure 2** - Synthetic route to obtain the compounds of the present disclosure. The building block 1-C can be prepared by metoxylation at C5 position followed by nucleophilic substitution. The 2,5-disubstituted pyrrolidine 1-C undergoes a sequence of reactions comprised of ester reduction and functional group protection/deprotection manipulations which allows to obtain a substrate for olefin metathesis. An amine compound 1-E which has a suitable leaving group (LG) such as trimethylsilyl (TMS) and a carbon-carbon double bond in allyl position to the LG reacts with carboxylic group of 6-carboxy-2-piperidone. Subsequently, this compound undergoes a selective reduction and cyclization reactions upon which the leaving group LG is detached from the starting molecule and the amine group is deprotected. This leads to the formation of the tricyclic compound 1-G. Obtained intermediate can subsequently be reacted with a suitable precursor for the moiety -SO₂-R^{B}. The R^{A} precursor 1-I synthesized by oxidation of deprotected alcohol 1-H gives a starting point for further optimization of R^{A} substituent. Compounds of the **general formulas 2 and 3** can be also prepared according to the synthetic route depicted in Figure 2. The compound of the **general formula 1** can be obtained by suitable transformation reactions of the vinyl group of the tricyclic sulfonamides.

### Examples

### Experiment 1: Synthesis of tricyclic ligand 3

The synthesis began by methylation of Boc-protected *L*-proline **7** to give methyl ester **8 (Scheme 1).** The resulting carbamate was subjected electrochemical oxidation in 0.05M methanolic solution of Et₄NOTs as an supporting electrolyte. After 5.0 F/mol has passed the methoxylated product **9** was obtained as a 1:1 mixture of diastereoisomers. The transformation to the *trans-*2,5-disubstituted pyrrolidine derivative **10** was accomplished through BF₃ · Et₂O-mediated reaction with the vinyl cuprate prepared *in situ* from *trans*-1-bromopropene. The primary alcohol **11** was obtained by reduction with LiBH₄ and later was converted into benzyl derivative **12.** Deprotection of the Boc group with bromotrimethylsilane (TMSBr) provided *trans*-5-propenylproline benzyl ether **13,** which was subsequently used in cross metathesis with commercially available allyltrimethylsilane in the presence of chlorodicyclohexyl borane as Lewis acid additive to give internal olefin **14.** The stereochemically pure amide **15** was obtained by HATU coupling with (*S*)-6-oxo-2-piperidinecarboxylic acid and following Boc protection furnished compound **16.** Reduction by DIBAL-H followed by HF-mediated *N*-acyliminium cyclization gave the tricyclic building block **17** as the only observed diastereomer. Reaction with 3,5-dichlorosulfonyl chloride furnished sulfonamide **18,** which after treatment with boron trichloride methyl sulfide complex afforded alcohol **19.** Jones oxidation of primary alcohol provided the functionalized FKBP ligand 3 ready for testing and further derivatization. 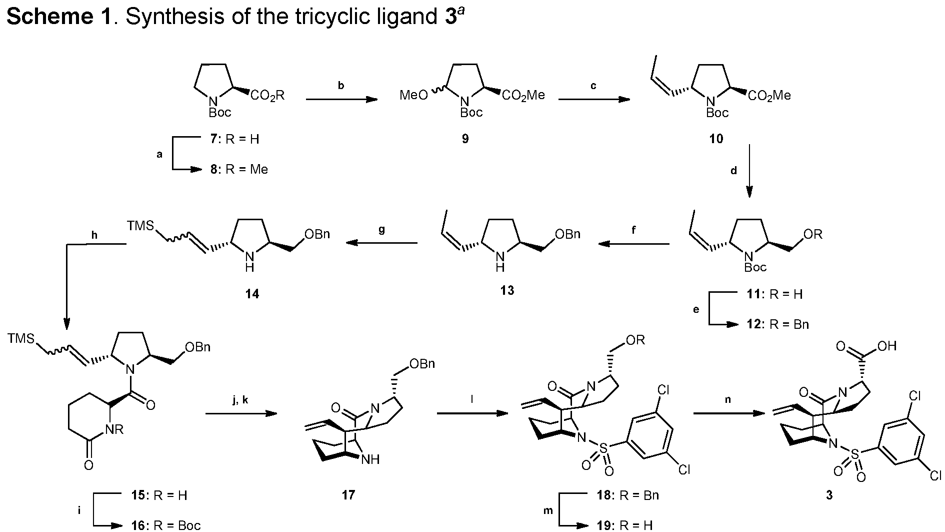 *^{a}* Reagents and conditions: (a) K₂CO₃, Mel, DMF, rt, 17h, 90%; (b) graphite anode, Et₄NOTs/MeOH, 250 mA, 5.0 F/mol, 5°C, 88%; (c) (2)-prop-1-en-1-yllithium, CuBr·Me₂S, BF₃·Et₂O, -78°C to rt, 30 min, 76%; (d) LiBH₄, THF, 0°C to rt, 17h, 87%; (e) NaH, benzyl bromide, THF, 0°C to rt, 17h, 81%; (f) TMSBr, DCM**,** rt, 17h, quant.; (g) allyltrimethylsilane, Cy₂BCl, Grubbs II, DCM, reflux, 3h, 71%; (h) (S)-6-oxo-2-piperidinecarboxylic acid **5,** HATU, DIPEA, DMF**,** rt, 4h, 56%; (i) Boc₂O, DIPEA, DMAP, DCM, rt, 36h, 88%; (j) DIBAL, THF, -78°C, 15 min; (k) HF, DCM**,** -78°C to 0°C, 3h, 65% (over 2 steps); (I) 3,5-dichlorobenzenesulfonyl chloride, DIPEA, MeCN, rt, 17h, 68%; (m) BCl₃·SMe₂, DCM**,** rt, 1h, 84%; (n) Jones reagent, acetone, 0°C to rt, 2h, 87%.

### Experiment 2: Optimization of R^{A} substituent

The optimization of tricyclic scaffold depicted in **Scheme 2** started with methylation of the parent compound **3** to get a tricyclic-based methyl ester **20.** The CDI-mediated amidation with aqueous ammonia gave the primary amide **21.** The amides **22-28** were obtained by coupling respective amines in the presence of HATU and DIPEA. However, to obtain the final compound **28,** the intermediate was subjected to Boc deprotection. Starting from the *N*-hydroxyphthalimide (NHP) ester of 3, compound **29** was successfully coupled with 2-iodopyridine in the presence of a NiCl₂bpy as the pre-catalyst, chlorosilane additive and zinc reductant. This provided a pyridine containing ligand **30** and compound **30-*sp*** with unfunctionalized ring system obtained as a side product. 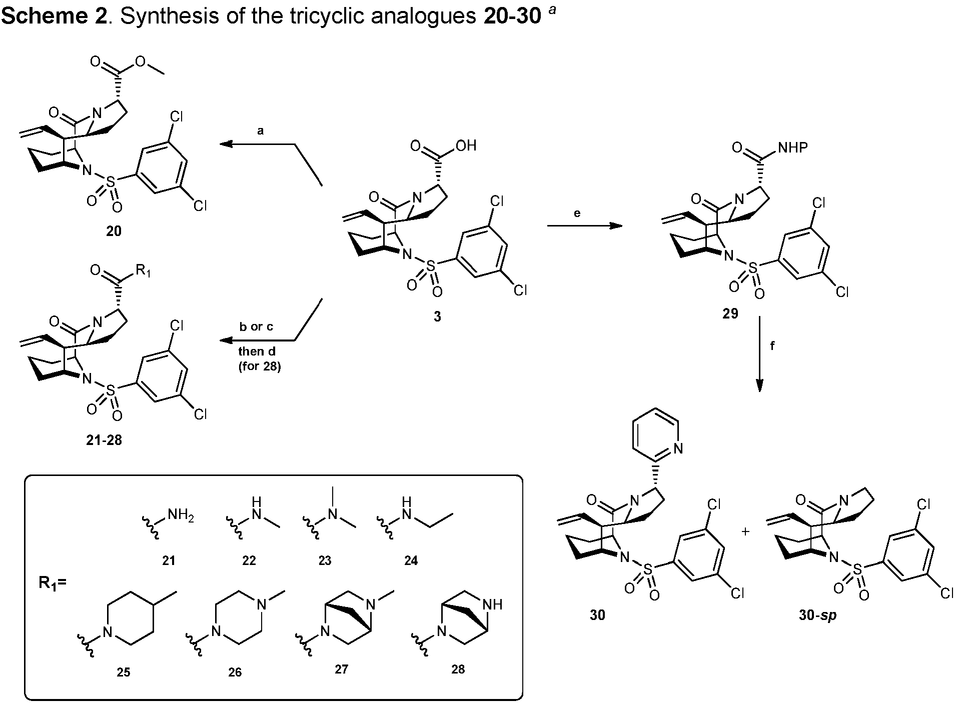 *^{a}* Reagents and conditions: (a) K₂CO₃, Mel, DMF, rt, 2h, 94%; (b) CDI, NH₃ (30% in H₂O), THF, rt, 30 min, quant. for 21; (c) methylamine hydrochloride (e) N-hydroxyphthalimide, EDC·HCl, DMAP, DCM, 0°C to rt, 17h, 97%; (f) 2-iodopyridine, NiCl₂(bpy), TMSCI, Zn, DMA, rt, 3h, 21% for 30

### Experiment 3: Functionalization of the vinyl group

In order to functionalize the vinyl group **(Scheme 3)** compounds **24** and **25** were subjected to the oxidative cleavage with osmium tetroxide. Reduction of respective aldehydes with sodium borohydride furnished alcohols **31** and **32.** 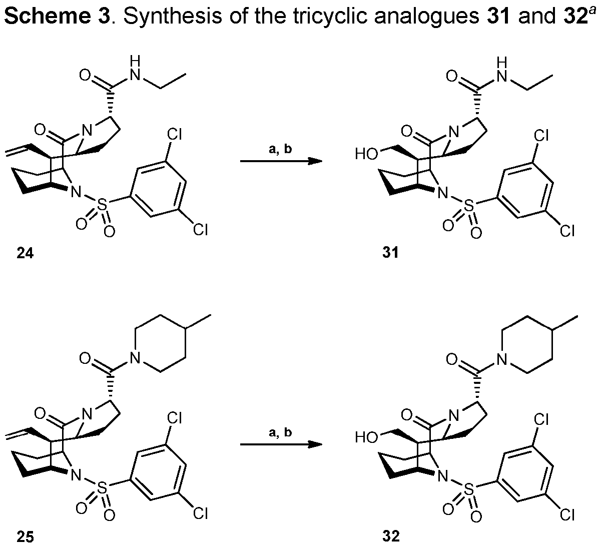 *^{a}* Reagents and conditions: (a) OsO₄, NalO₄, 2,6-lutidine, dioxane/H₂O (3:1), 0°C to rt, 48h; (b) NaBH₄, EtOH, 0°C to rt, 17h, 78% for 31, 76% for 32 (over 2 steps).

### Experiment 4: FP Assay

Affinity determination by competitive fluorescence polarization assay. The synthesized tricyclic sulfonamides were tested for their binding affinities to human FKBPs (FKBP12 and FKBP51) by the fluorescence polarization assay (Table 1).

**Table 1: Overview of binding affinities of the tricyclic analogues 3-32 obtained by a competitive fluorescence polarization assay.**

| 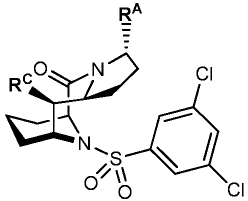 | | | | |
|---|---|---|---|---|
| **Entry** | **R₁** | **R3** | **FKBP51 Kᵢ [nM]** | **FKBP12 Kᵢ [nM]** |
| **C.** | - H | vinyl | 3893 | 146 ± 15 |
| **3** | 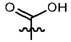 | vinyl | 698 ± 49 | 12 ± 1.7 |
| **20** | 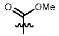 | vinyl | 76 ± 10 | 1.0 ± 0.1 |
| **21** | 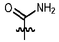 | vinyl | 18 ± 3.0 | 0.48 ± 0.08 |
| **22** | 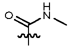 | vinyl | 7.7 ± 0.9 | 0.32 ± 0.04 |
| **23** | 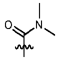 | vinyl | 121 ± 8.0 | 2.3 ± 0.2 |
| **24** | 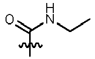 | vinyl | 1.1 ± 0.4 | 0.043 + 0.01 |
| **25** | 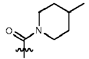 | vinyl | 0.93 ± 0.2 | 0.067 ± 0.01 |
| **26** | 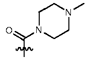 | vinyl | 11 ± 0.7 | 0.16 ± 0.018 |
| **27** | 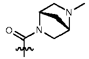 | vinyl | 17 ± 1.7 | 0.55 ± 0.07 |
| **28** | 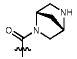 | vinyl | 52 ± 6.0 | 0.72 ± 0.1 |
| **30** | 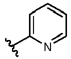 | vinyl | 464 ± 79 | 12 ± 1.6 |
| **31** | 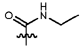 | - CH₂OH | 0.76 ± 0.2 | 0.013 ± 0.004 |
| **32** | 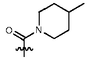 | - CH₂OH | 0.72 ± 0.2 | 0.019 ± 0.007 |

| | | | | |
|---|---|---|---|---|
| Wherein C. is a comparative compound, which may be an intermediate product. Wherein R₁ corresponds to RA, and R₃ corresponds to RC. | | | | |

### Experiment 5: nanoBRET Assay

For compounds **31** and **32** target engagement of FKBP12 and FKBP51 in the HEK293T cells was tested (Table 2). Compounds **31** and **32** displaced the tracer for FKBP51 with low nanomolar potency, whereas compound **32** could compete with the tracer for FKBP12 with even subnanomolar potency.

**Table 2. Overview of the nanoBRET data for intracellular FKBP occupancy in HEK293T cells.**

| **Entry** | **Structure** | **nanoBRET FKBP51 IC₅₀ [nM]** | **nanoBRET FKBP12 IC₅₀ [nM]** |
|---|---|---|---|
| **31** | 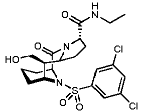 | 8.5 ± 0.6 | 3.9 ± 0.3 |
| **32** | 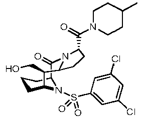 | 3.3 ± 0.3 | 0.49 ± 0.04 |

### Experiment 6: Exemplary synthesis and characterization of different compounds

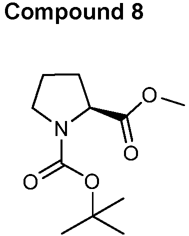

To a solution of *N*Boc-L-proline **7** (20.0 g, 92.92 mmol, 1.0 eq.) in DMF (90.0 mL) K₂CO₃ (12.2 g, 88.27 mmol, 0.95 eq.) was added. The resulting heterogenous mixture was stirred at room temperature for 30 min and then methyl iodide (5.2 mL, 83.62 mmol, 0.90 eq.) was added. The reaction mixture was stirred for 17h at room temperature. After completion, to the reaction mixture brine (100 mL) was added and the aqueous layer was extracted with Et₂O (3 x 200 mL). The combined organic layers were dried over MgSO₄, filtered and concentrated under reduced pressure. The crude ester was purified by flash column chromatography (Biotage Isolera One, 0-40% ethyl acetate in cyclohexane) to give 19.2 g (90%) of **8** as colorless thick oil.
**HPLC** (5 - 100 % solvent B, 3 min) Rt = 1.801 min, purity (220 nm): 95%
**¹H NMR** (500 MHz, Chloroform-d, mixture of rotamers) δ 4.24 (ddd, *J* = 51.2, 8.6, 3.7 Hz, 1H), 3.70 (d, *J =* 2.6 Hz, 3H), 3.56 - 3.30 (m, 2H), 2.29 - 2.07 (m, 1H), 2.01 - 1.74 (m, 3H), 1.41 (d, J = 25.8 Hz, 9H).
**¹³C NMR** (126 MHz, CDCl₃, mixture of rotamers) δ 173.86, 173.60, 154.52, 153.88, 79.93, 79.88, 59.21, 58.82, 52.18, 52.02, 46.66, 46.41, 30.98, 30.02, 28.53, 28.40, 24.44, 23.79.
**HRMS** (ESI) *m*/*z* calculated for sum formula C₁₁H₁₉NO₄ [M+H]: 252.12063 found 252.12069. 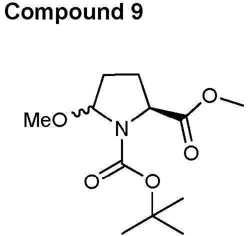
A methyl ester **8** (5.9 g, 25.86 mmol, 1.0 eq.) was dissolved in a 0.05 M solution of tetraethylammonium p-toluenesulfonate in MeOH (120.0 mL). The reaction mixture was cooled to 5 °C and two identical graphite rods (6.3 x 150 mm) were placed in the solution at a distance of 5 mm. A constant current of 250 mA was passed through the stirred solution. The electrolysis was terminated when 95% of the starting material was converted into product (after passage of 5.0 F/mol). The solvent was removed under reduced pressure and the residue was partitioned between water and Et₂O. The aqueous layer was extracted with Et₂O (3 × 100 mL). The combined organic layers were dried over MgSO₄, filtered and concentrated under reduced pressure. The crude product was purified by flash column chromatography (Biotage Isolera One, 0-40% ethyl acetate in cyclohexane) to give 5.9 g (88%) of 9 as a pale yellow oil.
**HPLC** (5 - 100 % solvent B, 3 min) Rt = 1.773 min and 1.798 min, purity (220 nm): 90%
**¹H NMR** (500 MHz, Chloroform-d, mixture of rotamers/diastereomers) δ 5.36 - 5.08 (m, 1H), 4.42 - 4.19 (m, 1H), 3.77 - 3.67 (m, 3H), 3.43 - 3.32 (m, 3H), 2.50 - 2.24 (m, 1H), 2.13 (dddd, J = 16.8, 14.1, 10.8, 7.2 Hz, 1H), 2.03 - 1.73 (m, 2H), 1.48 - 1.39 (m, 9H).
**¹³C NMR** (126 MHz, CDCl₃, mixture of rotamers/diastereomers) δ 173.30, 173.00, 154.24, 154.05, 153.91, 89.29, 89.23, 88.53, 88.35, 80.80, 80.59, 80.55, 59.61, 59.24, 58.74, 56.18, 55.91, 55.40, 54.97, 52.13, 52.09, 52.00, 51.95, 48.22, 32.90, 32.21, 31.10, 30.12, 28.30, 28.14, 27.05.
**HRMS** (ESI) *m*/*z* calculated for sum formula C₁₂H₂₁NO₅ [M+H]: 260.14925 found 260.14921. 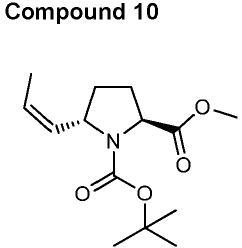
In an oven-dried Schlenk flask lithium chunks (940.0 mg, 136.06 mmol, 6.0 eq.) was washed three times with hexane under argon atmosphere. To the washed lithium chunks anhydrous Et₂O (90.0 mL) was added and the mixture was cooled to -20 °C followed by the addition of cis-1-bromo-1-propene (5.5 g, 45.35 mmol, 2.0 eq.).

After stirring the mixture for 2h at -20 °C, the milky-off grey solution was cannulated dropwise into a separate, over-dried flask containing a heterogeneous mixture of copper(I) bromide-dimethyl sulfide complex (9.3 g, 45.35 mmol, 2.0 eq.) in anhydrous Et₂O (115.0 mL) at -50°C over a period of 80 min (before the lithium species was added, heterogeneous mixture was chilled at-50°C for at least 10 min). The Schlenk flask and cannula were rinsed with anhydrous Et₂O (2 x 10 mL).

The resulting red solution was stirred at -50 °C for 45 min, cooled to -78 °C and chilled for at least 10 min before treating with boron trifluoride etherate (4.8 mL, 38.57 mmol, 2.0 eq.). After 10 min compound 9 (5.9 g, 22.68 mmol, 1.0 eq.) in 5 mL of Et₂O was added over a period of 20 min and the -78°C bath was removed and the reaction was warmed up to room temperature.

After 30 min the reaction mixture was quenched with a mixture of ammonium hydroxide and sat. aq. ammonium chloride solution (1:1, 100 mL) and diluted with EtOAc (150 mL). The aqueous layer was extracted with EA (3 x 150 mL). The organic layers were combined, dried over MgSO₄, filtered and concentrated under reduced pressure. The crude product was purified by flash column chromatography (Biotage Isolera One, 0-40% ethyl acetate in cyclohexane) to give 4.7 g (76%) of **10** as a pale yellow oil.
**HPLC** (5 - 100 % solvent B, 3 min) Rt = 2.107 min, purity (220 nm): 95%
**¹H NMR** (500 MHz, Chloroform-d, mixture of rotamers) δ 5.55 - 5.27 (m, 2H), 4.77 (dt, J = 42.7, 8.2 Hz, 1H), 4.35 (ddd, J = 42.9, 8.6, 2.1 Hz, 1H), 3.71 (s, 3H), 2.23 (dddt, J = 27.2, 22.4, 12.4, 6.0 Hz, 2H), 1.92 (td, J = 8.0, 6.9, 3.4 Hz, 1H), 1.77 - 1.64 (m, 3H), 1.60 (ddd, J = 11.9, 6.1, 2.7 Hz, 1H), 1.40 (d, J = 14.4 Hz, 9H).
**¹³C NMR** (126 MHz, CDCl₃, mixture of rotamers) δ 173.90, 173.53, 154.58, 153.58, 132.19, 131.86, 125.08, 123.73, 79.99, 79.94, 59.77, 59.34, 54.81, 54.55, 52.22, 52.05, 31.69, 30.94, 29.10, 28.53, 28.49, 28.45, 28.14, 13.20, 13.16.
**HRMS** (ESI) *m*/*z* calculated for sum formula C₁₄H₂₃NO₄ [M+H]: 270.16998 found 270.17011. 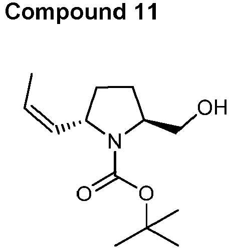
To an ice-cold solution of **10** (18.7 g, 69.47 mmol, 1.0 eq.) in anhydrous THF (620.0 mL) was added 2M solution of lithium borohydride in THF (69.5 mL, 138.93 mmol, 2.0 eq.). The cold bath was removed after the addition, and the reaction was stirred for 17h at room temperature.

The reaction was quenched at 0°C by addition of water (75 mL). The aqueous layer was extracted with EtOAc (4 x 300 mL). The organic layers were combined, dried over MgSO₄, filtered and concentrated under reduced pressure. The crude product was purified by flash column chromatography (gradient of cyclohexane/ethyl acetate 5:1 to 1:1) to give 14.6 g (87%) of **11** as a colorless oil.
**HPLC** (5 - 100 % solvent B, 3 min) Rt = 1.916 min, purity (220 nm): 97%
**¹H NMR** (500 MHz, Chloroform-d) δ 5.41 (dq, J = 17.9, 7.2 Hz, 1H), 5.33 (t, J = 10.3 Hz, 1H), 4.58 (t, J = 8.0 Hz, 1H), 4.08 (dq, J = 8.5, 4.6, 4.0 Hz, 1H), 3.67 (dd, J = 11.1, 7.6 Hz, 1H), 3.61 (dd, J = 11.2, 3.3 Hz, 1H), 2.16 - 1.96 (m, 2H), 1.65 (d, J = 6.9 Hz, 3H), 1.58 (dq, J = 12.4, 4.3 Hz, 2H), 1.43 (d, J = 10.1 Hz, 9H).
**¹³C NMR** (126 MHz, CDCl₃) δ 156.99, 132.44, 123.41, 80.33, 67.89, 60.17, 55.86, 31.56, 28.57, 27.19, 13.13.
**HRMS** (ESI) *m*/*z* calculated for sum formula C₁₃H₂₃NO₃ [M+H]: 242.17507 found 242.17519. 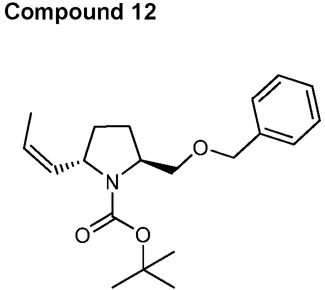
An ice-cold solution of alcohol **11** (13.66 g, 56.60 mmol, 1.0 eq.) in anhydrous THF (63 mL) was treated with sodium hydride (60%, susp. in mineral oil, 2.64 g, 65.09 mmol, 1.15 eq.). After stirring for 30 min at 0°C, benzyl bromide (7.73 mL, 11.13 mmol, 1.15 eq.) was added dropwise. The ice bath was removed and the reaction was stirred at room temperature for 17h. After completion, the reaction was quenched with water (100 mL) and the aqueous layer was extracted with EtOAc (3 x 200 mL). The organic layers were combined, dried over MgSO₄, filtered and concentrated under reduced pressure. The crude product was purified by flash column chromatography (cyclohexane/ethyl acetate 5:1) to give 15.2 g (81%) of **12** as a colorless oil.
**HPLC** (5 - 100 % solvent B, 3 min) Rt = 2.503 min, purity (220 nm): 97%
**¹H NMR** (500 MHz, Chloroform-d) δ 7.31 (d, J = 32.8 Hz, 5H), 5.64 - 5.27 (m, 2H), 4.71 - 4.38 (m, 3H), 4.06 (dt, J = 72.0, 8.2 Hz, 1H), 3.78 - 3.54 (m, 1H), 3.40 (dt, J = 79.7, 8.6 Hz, 1H), 2.19 (dtd, J = 23.0, 12.0, 6.0 Hz, 1H), 2.01 (tq, J = 21.0, 12.3, 9.6 Hz, 2H), 1.75 - 1.63 (m, 3H), 1.58 (dq, J = 14.6, 7.8, 7.0 Hz, 1H), 1.42 (d, J = 4.0 Hz, 9H).
**¹³C NMR** (126 MHz, CDCl₃) δ 154.38, 138.66, 132.82, 128.34, 127.52, 127.47, 122.87, 79.19, 73.17, 70.23, 56.66, 54.67, 31.20, 28.51, 26.18, 13.04.
**HRMS** (ESI) *m*/*z* calculated for sum formula C₂₀H₂₉NO₃ [M+H]: 332.22202 found 332.22210. 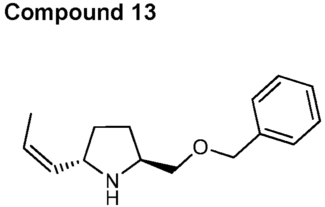
To a solution of **12** (17.3 g, 52.29 mmol, 1.0 eq.) in DCM (490.0 mL) TMSBr (34.5 mL, 261.43 mmol, 5.0 eq.) was added. After stirring for 17h at room temperature the reaction mixture was concentrated under reduced pressure affording brown oil. The crude product was purified by flash column chromatography (cyclohexane/ethyl acetate 5:1) to yield **13** quantitively as an orange oil.
**HPLC** (5 - 100 % solvent B, 3 min) Rt = 1.342 min, purity (220 nm): >99%
**¹H NMR** (500 MHz, Chloroform-d) δ 7.40 (d, J = 7.5 Hz, 2H), 7.31 (t, J = 7.5 Hz, 2H), 7.27 (d, J = 7.2 Hz, 1H), 5.76 (t, J = 6.5 Hz, 2H), 4.74 (d, J = 11.4 Hz, 1H), 4.49 (t, J = 10.1 Hz, 2H), 4.07 (tt, J = 8.0, 3.9 Hz, 1H), 3.93 (dd, J = 10.3, 3.7 Hz, 1H), 3.62 (dd, J = 10.3, 4.0 Hz, 1H), 2.21 - 2.06 (m, 2H), 1.97 (ddd, J = 15.1, 11.9, 7.8 Hz, 1H), 1.83 (qd, J = 11.2, 8.0 Hz, 1H), 1.72 (d, J = 5.0 Hz, 3H).
**¹³C NMR** (126 MHz, CDCl₃) δ 137.77, 132.00, 128.45, 128.32, 127.88, 124.67, 73.41, 68.81, 58.10, 57.54, 31.74, 26.95, 13.85.
**HRMS** (ESI) *m*/*z* calculated for sum formula C₁₅H₂₁NO [M+H]: 232.16959 found 232.16966. 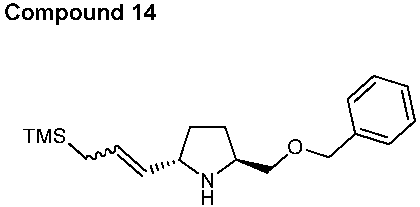
In an oven-dried three-neck flask a vinylproline derivative **13** (12.1 g, 52.31 mmol, 1.0 eq.) was dissolved in anhydrous DCM (200.0 mL) under argon atmosphere. Allyltrimethylsilane (66.5 mL, 418.45 mmol, 8.0 eq.) and Cy₂BCl (1M solution in hexane, 10.5 mL, 10.46 mmol, 20.0 mol%) were added, and the reaction mixture was pre-heated at 50°C for 5 min. The Grubbs 2^{nd} gen. catalyst (3.1 g, 3.66 mmol, 7.0 mol%). was added in one portion in a solid form. The reaction was stirred for 3h in reflux and afterwards quenched with SnatchCat (2.0 eq. in reference to the catalyst were used). After 30 min of stirring in reflux the reaction mixture was cooled down to room temperature and filtrated through a pad of Celite. The solvent was removed under reduced pressure. The crude product was purified by flash column chromatography (cyclohexane/ethyl acetate 1:1 + 3% TEA) to give 11.2 g (71%) of **14** as a pale yellow oil.
**HPLC** (5 - 100 % solvent B, 3 min) Rt = 1.804 min, purity (220 nm): 90%
**¹H NMR** (500 MHz, Chloroform-d) δ 7.34 (d, J = 4.3 Hz, 5H), 5.57 - 5.46 (m, 1H), 5.26 (dddd, J = 14.9, 8.8, 6.1, 1.6 Hz, 1H), 4.54 (d, J = 2.2 Hz, 2H), 3.59 (q, J = 7.1 Hz, 1H), 3.55 - 3.48 (m, 1H), 3.37 (qt, J = 9.2, 4.6 Hz, 2H), 1.99 - 1.95 (m, 2H), 1.51 - 1.43 (m, 2H), 1.42 (d, J = 8.0 Hz, 2H), -0.01 (d, J = 9.3 Hz, 9H).
**¹³C NMR** (126 MHz, CDCl₃) δ 138.58, 131.51, 128.50, 127.83, 127.70, 127.27, 74.36, 73.27, 60.13, 56.95, 32.85, 28.30, 22.70, -1.83.
**HRMS** (ESI) *m*/*z* calculated for sum formula C₁₈H₂₉NOSi [M+H]: 304.20912 found 304.20948. 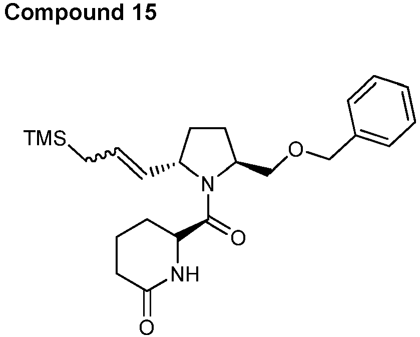
To a solution of amine **14** (11.2 g, 37.03 mmol, 1.0 eq.) in DMF (45.0 mL) (S)-6-oxopiperidine-2-carboxylic acid (5.8 g, 40.74 mmol, 1.1 eq.) was added. Resulting mixture was treated with DIPEA (19.3 mL, 111.10 mmol, 3.0 eq.) and after 5 min HATU (18.3 g, 48.14 mmol, 1.3 eq.) was added. The reaction mixture was stirred for 4h at room temperature. After the complete conversion of the starting material, the reaction was quenched by addition of sat. aq. NaHCO₃ solution (50 mL). The aqueous layer was extracted with mixture of DCM/Et₂O (1:1, 4 x 200 mL). The organic layers were combined and washed with brine (2 x 100 mL), then dried over MgSO₄, filtered and concentrated under reduced pressure. The crude product was purified by flash column chromatography (gradient of cyclohexane/ethyl acetate 2:1 + 3% TEA to pure ethyl acetate + 5% MeOH + 3% TEA) to give 8.9 g (56%) of **15** as a dark orange oil.
**HPLC** (5 - 100 % solvent B, 3 min) Rt = 2.504 min, purity (220 nm): >99% **¹H NMR** (500 MHz, Chloroform-d) δ 7.35 - 7.29 (m, 2H), 7.29 - 7.24 (m, 3H), 5.41 (dt, J = 15.8, 8.0 Hz, 1H), 5.36 - 5.22 (m, 1H), 4.53 - 4.41 (m, 2H), 4.34 (td, J = 6.2, 3.0 Hz, 1H), 4.31 - 4.25 (m, 2H), 3.61 - 3.49 (m, 2H), 2.45 - 2.26 (m, 3H), 2.03 - 1.89 (m, 4H), 1.76 - 1.55 (m, 3H), 1.49 - 1.34 (m, 2H), 0.07 - -0.08 (m, 9H).
**¹³C NMR** (126 MHz, CDCl₃) δ 172.30, 170.79, 138.51, 128.79, 128.47, 128.30, 127.66, 127.52, 73.36, 69.70, 59.97, 57.78, 54.46, 32.28, 30.74, 26.29, 24.97, 22.74, 19.28, -1.81.
**HRMS** (ESI) *m*/*z* calculated for sum formula C₂₄H₃₆N₂O₃Si [M+H]: 429.25680 found 429.25675. 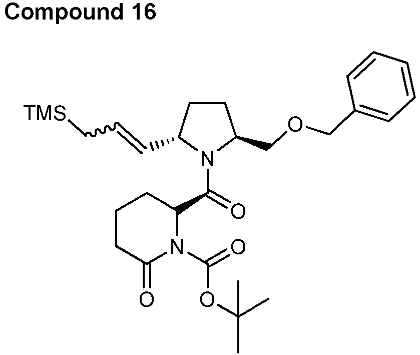
To a solution of amine **15** (8.9 g, 20.81 mmol, 1.0 eq.) in DCM (50.0 mL) Boc₂O (36.3 g, 166.48 mmol, 8.0 eq.) and DIPEA (18.1 mL, 104.05 mmol, 5.0 eq.) were added. After addition of DMAP (7.6 mg, 62.43 mmol, 3.0 eq.) the resulting mixture was stirred for 36h at room temperature. After completion, the reaction mixture was diluted with DCM and washed with brine (2 x 100 mL). The organic layers were combined, dried over MgSO₄, filtered and concentrated under reduced pressure. The crude product was purified by flash column chromatography (cyclohexane/ethyl acetate 1:1) to give 9.7 g (88%) of **16** as an yellow oil, which solidified over time.
**HPLC** (50 - 100 % solvent B, 3 min) Rt = 2.599 min, purity (220 nm): 97%
**¹H NMR** (500 MHz, Chloroform-d) δ 7.36 - 7.25 (m, 5H), 5.61 - 5.46 (m, 1H), 5.42 (dd, J = 15.4, 6.8 Hz, 1H), 4.93 (t, J = 4.5 Hz, 0.5H), 4.68 (dd, J = 5.6, 3.6 Hz, 0.3H), 4.57 - 4.49 (m, 1H), 4.47 - 4.41 (m, 1H), 4.41 - 4.34 (m, 1H), 4.28 (t, J = 7.3 Hz, 1H), 3.67 (ddd, J = 18.5, 9.5, 6.0 Hz, 1H), 3.57 (td, J = 8.4, 7.5, 2.8 Hz, 1H), 2.54 (ddd, J = 17.2, 6.2, 3.8 Hz, 1H), 2.47 - 2.27 (m, 2H), 1.93 (qd, J = 9.0, 8.2, 5.6 Hz, 3H), 1.70 - 1.61 (m, 4H), 1.53 - 1.44 (m, 11H), 0.10 - -0.08 (m, 9H).
**¹³C NMR** (126 MHz, CDCl₃) δ 171.56, 170.41, 153.78, 138.71, 128.58, 128.46, 128.07, 127.62, 127.51, 82.80, 73.32, 69.88, 60.38, 58.00, 57.55, 34.44, 33.35, 28.19, 25.58, 25.44, 22.57, 18.51, -1.50, -1.68, -1.80.
**HRMS** (ESI) *m*/*z* calculated for sum formula C₂₉H₄₄N₂O₅Si [M+H]: 529.30923 found 529.30955. 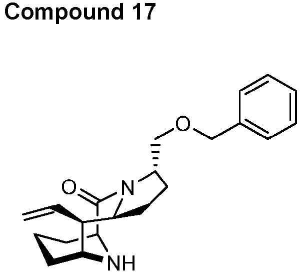
To a solution of Boc-protected amine **16** (8.7 g, 16.42 mmol, 1.0 eq.) in THF (160.0 mL) DIBAL-H (1M in hexane, 21.0 mL, 21.00 mmol, 1.3 eq.) was added dropwise (over 15 min) at -78°C under argon. The reaction mixture was stirred for 15 min at the same temperature and monitored by LCMS. If conversion was not completed an extra portion (0.1 eq.) of DIBAL-H was added dropwise. After the complete conversion of the starting material (in total 1.7 eq. of DIBAL-H was used), the reaction was quenched by the addition of Glauber's salt (15.0 g) at -78°C and the resulting mixture was warmed to room temperature. The resulting heterogeneous suspension was filtered through a pad of Celite and washed with THF. The solvent was removed under reduced pressure (using rotavap's water bath at 30°C). The residue (yellow oil) was used in the next step without further purification.

The residue for the first step was dissolved in DCM (800.0 mL) in HDPE bottle and cooled down to -78°C. A solution of HF (42.0 mL, 1.62 mol, 100.0 eq., 70% in pyridine) was added and the mixture was stirred for 5 min at the same temperature. The reaction mixture was transferred to an ice-bath and stirred for 3h at 0°C. The reaction was quenched at 0°C by the addition of with sat. aq. CaCO₃ solution (450 mL) and 10 M NaOH (270 mL - until pH ~14). The resulting heterogeneous mixture was transferred to centrifuge bottles and the solid side products were separated. The aqueous layer was extracted with DCM (5 x 350 mL). The organic layers were combined, dried over MgSO₄, filtered and concentrated under reduced pressure. The crude product was purified by flash column chromatography (gradient of cyclohexane/ethyl acetate 1:1 + 3% TEA to 100% ethyl acetate + 5% MeOH + 3% TEA) to give 3.6 g (65% over 2 steps) of 17 as an yellow oil.
**HPLC** (5 - 100 % solvent B, 3 min) Rt = 1.836 min, purity (220 nm): 88%
**¹H NMR** (500 MHz, Chloroform-d) δ 7.30 (tt, J = 13.8, 7.1 Hz, 5H), 5.53 (dt, J = 16.7, 9.8 Hz, 1H), 5.01 - 4.89 (m, 2H), 4.56 - 4.44 (m, 2H), 4.41 (dq, J = 9.9, 4.7, 2.5 Hz, 1H), 4.04 (q, J = 8.0 Hz, 1H), 3.70 - 3.62 (m, 2H), 3.61 (d, J = 3.7 Hz, 1H), 2.77 (d, J = 7.4 Hz, 1H), 2.38 (q, J = 9.2 Hz, 1H), 2.30 - 2.19 (m, 2H), 2.05 - 1.97 (m, 1H), 1.97 - 1.88 (m, 1H), 1.88 - 1.80 (m, 1H), 1.60 (dddd, J = 32.0, 19.4, 11.7, 7.5 Hz, 5H).
**¹³C NMR** (126 MHz, CDCl₃) δ 175.07, 138.78, 138.71, 128.44, 127.62, 116.14, 73.26, 69.82, 60.02, 59.16, 57.13, 55.14, 53.00, 31.60, 27.86, 26.81, 25.45, 16.87.
**HRMS** (ESI) *m*/*z* calculated for sum formula C₂₁H₂₈N₂O₂ [M+H]: 341.22235 found 341.22289. 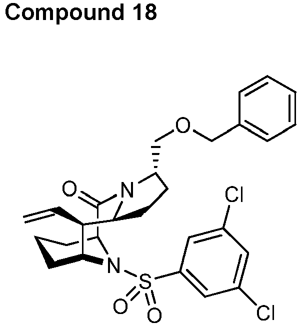
A solution of tricyclic amine **17** (1.0 g, 2.94 mmol, 1.0 eq.) and 3,5-dichlorobenzenesulfonyl chloride (1.4 g, 5.87 mmol, 2.0 eq.) in acetonitrile (29.5 mL, HPLC grade) was treated with DIPEA (1.1 mL, 5.87 mmol, 2.0 eq.). The reaction mixture was stirred for 17h under argon atmosphere at room temperature. After completion, the mixture was diluted with ethyl acetate (75 mL) and washed with brine (2 x 30 mL). The organic layers was dried over MgSO₄, filtered and concentrated under reduced pressure. The crude product was purified by flash column chromatography (Biotage Isolera One, 0-100% ethyl acetate in cyclohexane) to give 1.1 g (68%) of **18** as an yellow oil.
**HPLC** (50 - 100 % solvent B, 3 min) Rt = 2.558 min, purity (220 nm): 97%
**¹H NMR** (500 MHz, Chloroform-d) δ 7.69 (d, J = 1.8 Hz, 2H), 7.54 (t, J = 1.9 Hz, 1H), 7.34 (d, J = 4.4 Hz, 4H), 7.28 (dd, J = 8.4, 4.2 Hz, 1H), 5.69 (dt, J = 16.8, 9.8 Hz, 1H), 5.11 (dd, J = 10.0, 1.5 Hz, 1H), 5.02 (dd, J = 16.9, 1.4 Hz, 1H), 4.67 (dt, J = 6.3, 1.9 Hz, 1H), 4.54 (d, J = 2.2 Hz, 2H), 4.43 (qd, J = 6.9, 3.2 Hz, 1H), 4.16 - 4.05 (m, 1H), 3.94 - 3.87 (m, 1H), 3.75 (dd, J = 9.4, 3.2 Hz, 1H), 3.49 (dd, J = 9.3, 7.0 Hz, 1H), 2.35 (td, J = 9.7, 7.4 Hz, 1H), 2.32 - 2.25 (m, 1H), 2.09 - 2.00 (m, 1H), 1.91 (dq, J = 13.9, 7.1 Hz, 1H), 1.87 - 1.80 (m, 1H), 1.70 (dq, J = 12.7, 7.3 Hz, 1H), 1.61 - 1.46 (m, 3H), 1.23 (dddd, J = 19.2, 17.3, 8.8, 5.1 Hz, 2H).
¹³C NMR (126 MHz, CDCl₃) δ 170.30, 144.29, 138.67, 137.34, 136.45, 132.77, 128.49, 127.74, 127.63, 125.09, 117.71, 73.44, 69.74, 60.56, 59.26, 56.64, 54.89, 54.43, 30.84, 26.30, 25.40, 25.14, 15.66.
**HRMS** (ESI) *m*/*z* calculated for sum formula C₂₇H₃₀Cl₂N₂O₄S [M+H]: 549.13761 found 549.13844. 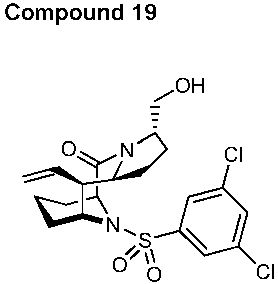
To a solution of tricylic sulfonamide **18** (2.5 g, 4.57 mmol, 1.0 eq.) boron trichloride methyl sulfide complex solution (2M in DCM, 6.9 mL, 13.71 mmol, 3.0 eq.) was added. The reaction mixture was stirred for 1h at room temperature under argon atmosphere. After the completion, the reaction mixture was quenched and washed with sat. aq. NaHCO₃ solution (1 x 50 mL). The organic layer was dried over MgSO₄, filtered and concentrated under reduced pressure. The crude product was purified by flash column chromatography (Biotage Isolera One, 0-100% ethyl acetate in cyclohexane) to give 1.8 g (84%) of **19** as a beige crystalline solid.
**HPLC** (5 - 100 % solvent B, 3 min) Rt = 2.777 min, purity (220 nm): 94%
**¹H NMR** (500 MHz, Chloroform-d) δ 7.69 (d, J = 1.8 Hz, 2H), 7.57 (t, J = 1.9 Hz, 1H), 5.70 (dt, J = 16.8, 9.8 Hz, 1H), 5.12 (dd, J = 10.0, 1.4 Hz, 1H), 5.05 (dd, J = 16.9, 1.4 Hz, 1H), 4.59 (dt, J = 6.4, 1.9 Hz, 1H), 4.27 (dddd, J = 9.5, 7.1, 4.9, 1.9 Hz, 1H), 4.09 (td, J = 9.6, 5.9 Hz, 1H), 3.98 (ddd, J = 7.1, 4.5, 2.1 Hz, 1H), 3.83 (dd, J = 12.2, 2.1 Hz, 1H), 3.56 (dd, J = 12.1, 5.0 Hz, 1H), 2.39 (td, J = 9.6, 7.7 Hz, 1H), 2.27 (ddt, J = 14.0, 4.0, 1.9 Hz, 1H), 2.02 - 1.91 (m, 2H), 1.70 - 1.49 (m, 5H), 1.35 - 1.17 (m, 3H).
**¹³C NMR** (126 MHz, CDCl₃) δ 172.15, 144.02, 136.61, 136.52, 132.94, 125.03, 118.12, 65.37, 65.15, 60.54, 56.50, 54.59, 54.52, 31.89, 25.93, 25.88, 25.18, 15.58.
**HRMS** (ESI) *m*/*z* calculated for sum formula C₂₀H₂₄Cl₂N₂O₄S [M+H]: 459.09066 found 459.09107. 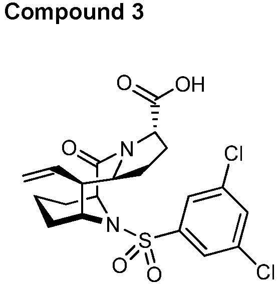
To an ice-cold solution of tricylic alcohol **19** (1.8 g, 3.83 mmol, 1.0 eq.) in acetone (96.0 mL) Jones reagent (3.8 mL, 7.66 mmol, 2.0 eq.) was added dropwise. Next, the ice bath was removed and the reaction was stirred for 2h at room temperature. After the complete conversion of the starting material, the reaction was quenched by slow addition of sat. aq. NaHCO₃ solution (25 mL). The aqueous phase was extracted with a mixture of CHCl₃/*i*-PrOH (3:1, 5 x 60 mL). The organic layers were combined, dried over MgSO₄, filtered and concentrated under reduced pressure. The crude product was purified by flash column chromatography (Biotage Isolera One, 0-70% ethyl acetate in cyclohexane + 2% FA) to give 1.6 g (87%) of **3** as a white solid.
**HPLC** (30 - 100 % solvent B, 3 min) Rt = 2.189 min, purity (220 nm): 97%
**¹H NMR** (500 MHz, Chloroform-d) δ 7.73 (d, J = 1.9 Hz, 2H), 7.58 (t, J = 1.9 Hz, 1H), 5.68 (dt, J = 16.9, 9.8 Hz, 1H), 5.13 (dd, J = 10.0, 1.4 Hz, 1H), 5.06 (dd, J = 16.8, 1.4 Hz, 1H), 4.76 (dt, J = 6.2, 1.9 Hz, 1H), 4.53 (dd, J = 8.7, 7.1 Hz, 1H), 4.06 (td, J = 9.1, 6.3 Hz, 1H), 3.99 (dt, J = 7.2, 3.4 Hz, 1H), 2.34 (td, J = 9.7, 7.2 Hz, 1H), 2.30 - 2.23 (m, 1H), 2.16 (dtd, J = 13.2, 6.7, 3.7 Hz, 1H), 2.06 (dtd, J = 12.8, 6.4, 3.8 Hz, 1H), 1.94 - 1.83 (m, 1H), 1.77 - 1.65 (m, 1H), 1.56 (dt, J = 11.4, 3.5 Hz, 3H), 1.41 - 1.29 (m, 2H).
**¹³C NMR** (126 MHz, CDCl₃) δ 174.91, 171.25, 143.81, 136.68, 136.49, 132.99, 125.26, 118.20, 62.73, 59.70, 56.20, 54.70, 54.55, 32.25, 26.61, 26.50, 25.63, 15.70.
**HRMS** (ESI) *m*/*z* calculated for sum formula C₂₀H₂₂Cl₂N₂O₅S [M+H]: 473.06992 found 473.07004. 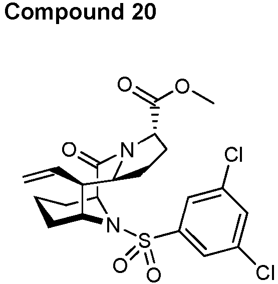
To a solution of tricyclic acid 3 (15.0 mg, 31.69 µmol, 1.0 eq.) in DMF (320.0 µL) was added K₂CO₃ (9.0 mg, 63.38 µmol, 2.0 eq.). The resulting heterogenous mixture was stirred at room temperature for 30 min and then methyl iodide (6.0 µL, 95.06 µmol, 3.0 eq.) was added. The reaction mixture was stirred for 2h at room temperature. After completion, to the reaction mixture was diluted with EtOAc and washed with brine (1 x 20 mL). The aqueous phase was extracted with EtOAc (3 x 20 mL). The combined organic layers were dried over MgSO₄, filtered and concentrated under reduced pressure. The crude product was purified by preparative HPLC on a C-18 reverse-phase column eluting with a gradient of water (0.1% TFA) and acetonitrile (0.1% TFA) giving after lyophilization 14.5 mg (94%) of **20** as a white solid.
**HPLC** (5 - 100 % solvent B, 3 min) Rt = 2.814 min, purity (220 nm): >99%
**¹H NMR** (500 MHz, Chloroform-d) δ 7.73 (d, J = 1.9 Hz, 2H), 7.56 (t, J = 1.8 Hz, 1H), 5.70 (dt, J = 16.8, 9.8 Hz, 1H), 5.13 (d, J = 10.0 Hz, 1H), 5.06 (d, J = 16.8 Hz, 1H), 4.76 (d, J = 6.0 Hz, 1H), 4.51 (t, J = 8.4 Hz, 1H), 4.15 (td, J = 9.5, 6.0 Hz, 1H), 3.96 (t, J = 6.1 Hz, 1H), 3.74 (s, 3H), 2.33 (q, J = 9.0 Hz, 1H), 2.27 - 2.14 (m, 2H), 2.05 (dt, J = 13.2, 6.2 Hz, 1H), 1.79 (qd, J = 11.5, 6.3 Hz, 1H), 1.72 - 1.60 (m, 1H), 1.55 (d, J = 9.5 Hz, 3H), 1.29 (qd, J = 11.2, 10.6, 5.3 Hz, 2H).
**¹³C NMR** (126 MHz, CDCl₃) δ 171.58, 170.93, 144.14, 136.63, 136.50, 132.87, 125.15, 118.18, 62.88, 59.71, 56.14, 54.91, 54.55, 52.46, 32.60, 26.93, 26.38, 25.41, 15.62.
**HRMS** (ESI) *m*/*z* calculated for sum formula C₂₁H₂₄Cl₂N₂O₅S [M+H]: 487.08557 found 487.08612. 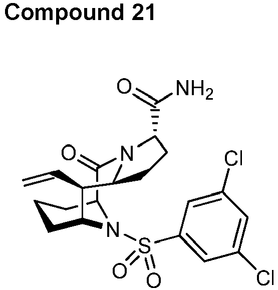
To a solution of tricyclic acid **3** (17.0 mg, 35.91 µmol, 1.0 eq.) in anhydrous THF (3.6 mL) was added CDI (58.2 mg, 359.1 µmol, 10.0 eq.). The resulting mixture was stirred at room temperature for 2h and then aqueous ammonia solution (30%, 62.0 µL, 1077.0 µmol, 30.0 eq.) was added. After stirring for additional 30 min reaction was completed. The reaction solution was diluted with EtOAc and washed with brine (1 x 20 mL). The aqueous phase was extracted with EtOAc (3 x 20 mL). The combined organic layers were dried over MgSO₄, filtered and concentrated under reduced pressure. The crude product was purified by flash column chromatography (Biotage Isolera One, 50-100% ethyl acetate in cyclohexane) to give 17.1 mg (98%) of **21** as a white solid
**HPLC** (5 - 100 % solvent B, 3 min) Rt = 2.498 min, purity (220 nm): 95%
**¹H NMR** (500 MHz, Chloroform-d) δ 7.68 (d, J = 1.8 Hz, 2H), 7.59 (d, J = 1.9 Hz, 1H), 6.51 (s, 1H), 5.74 (dt, J = 16.8, 9.8 Hz, 1H), 5.36 (s, 1H), 5.17 (dd, J = 10.1, 1.3 Hz, 1H), 5.14 - 5.06 (m, 1H), 4.64 (t, J = 8.0 Hz, 1H), 4.49 - 4.39 (m, 1H), 4.27 (td, J = 9.1, 6.2 Hz, 1H), 4.11 (dd, J = 7.3, 4.7 Hz, 1H), 2.47 (td, J = 9.7, 7.8 Hz, 1H), 2.31 - 2.22 (m, 2H), 2.15 - 2.08 (m, 1H), 1.93 (dddd, J = 12.5, 10.6, 8.5, 6.4 Hz, 1H), 1.78 - 1.72 (m, 1H), 1.72 - 1.66 (m, 1H), 1.60 - 1.55 (m, 1H), 1.41 (tt, J = 12.2, 4.8 Hz, 1H), 1.26 (tt, J = 7.1, 2.9 Hz, 2H).
**¹³C NMR** (126 MHz, CDCl₃) δ 173.96, 171.43, 143.43, 136.47, 136.20, 133.05, 124.84, 118.46, 63.98, 60.59, 56.02, 54.71, 54.06, 32.37, 26.95, 25.73, 25.44, 15.35.
**HRMS** (ESI) *m*/*z* calculated for sum formula C₂₀H₂₈Cl₂N₈O₄S [M+H]: 472.08591 found 472.08663. 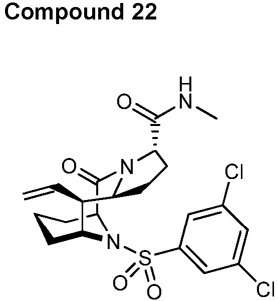
To a solution of methylamine hydrochloride (6.5 mg, 95.06 µmol, 3.0 eq.) in DMF (300.0 µL) tricyclic acid **3** (15.0 mg, 31.69 µmol, 1.0 eq.) was added. Resulting mixture was treated with DIPEA (22.0 µL, 126.75 µmol, 4.0 eq.) and after 5 min HATU (36.1 mg, 95.06 µmol, 3.0 eq.) was added. The reaction mixture was stirred for 6h at room temperature. After the complete conversion of the starting material, the reaction was quenched by addition of sat. aq. NaHCO₃ solution (2.0 mL) and diluted with DCM. The organic phase was washed with brine (2 x 5 mL), then dried over MgSO₄, filtered and concentrated under reduced pressure. The crude product was purified by preparative HPLC on a C-18 reverse-phase column eluting with a gradient of water (0.1% TFA) and acetonitrile (0.1% TFA) giving after lyophilization 13.4 mg (87%) of **22** as a white solid.
**HPLC** (5 - 100 % solvent B, 3 min) Rt = 2.581 min, purity (220 nm): >99%
**¹H NMR** (500 MHz, Chloroform-d) δ 7.68 (d, J = 1.8 Hz, 2H), 7.59 (t, J = 1.9 Hz, 1H), 6.69 (q, J = 4.8 Hz, 1H), 5.73 (dt, J = 16.8, 9.8 Hz, 1H), 5.17 (dd, J = 10.0, 1.4 Hz, 1H), 5.10 (dd, J = 16.9, 1.3 Hz, 1H), 4.74 (t, J = 8.0 Hz, 1H), 4.45 (dt, J = 6.6, 1.9 Hz, 1H), 4.25 (td, J = 9.2, 6.1 Hz, 1H), 4.11 (tt, J = 6.5, 5.0, 2.1 Hz, 1H), 2.81 (d, J = 4.7 Hz, 3H), 2.48 (td, J = 9.6, 7.8 Hz, 1H), 2.33 - 2.21 (m, 2H), 2.08 (dtd, J = 12.7, 6.2, 3.7 Hz, 1H), 1.86 (dddd, J = 12.6, 10.6, 8.4, 6.4 Hz, 1H), 1.79 - 1.65 (m, 2H), 1.57 (dtd, J = 10.0, 7.2, 6.7, 3.5 Hz, 2H), 1.40 (tt, J = 13.5, 12.2, 4.9 Hz, 1H), 1.26 (ddt, J = 19.0, 12.8, 6.0 Hz, 1H).
**¹³C NMR** (126 MHz, CDCl₃) δ 173.10, 171.94, 143.55, 136.66, 136.17, 133.25, 124.90, 118.76, 64.06, 60.94, 56.13, 54.86, 54.05, 32.51, 27.21, 26.59, 25.73, 25.50, 15.42.
**HRMS** (ESI) *m*/*z* calculated for sum formula C₂₁H₂₅Cl₂N₈O₄S [M+H]: 486.10156 found 486.10171. 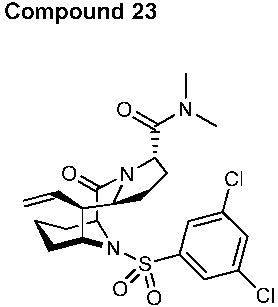
To a solution of dimethylamine (2M in THF, 47.5 µL, 95.06 µmol, 3.0 eq.) in DMF (300.0 µL) tricyclic acid **3** (15.0 mg, 31.69 µmol, 1.0 eq.) was added. Resulting mixture was treated with DIPEA (22.0 µL, 126.75 µmol, 4.0 eq.) and after 5 min HATU (36.1 mg, 95.06 µmol, 3.0 eq.) was added. The reaction mixture was stirred for 17h at room temperature. After the complete conversion of the starting material, the reaction was quenched by addition of sat. aq. NaHCO₃ solution (2.0 mL) and diluted with DCM. The organic phase was washed with brine (2 x 5 mL), then dried over MgSO₄, filtered and concentrated under reduced pressure. The crude product was purified by preparative HPLC on a C-18 reverse-phase column eluting with a gradient of water (0.1% TFA) and acetonitrile (0.1% TFA) giving after lyophilization 15.1 mg (95%) of **23** as a white solid.
**HPLC** (5 - 100 % solvent B, 3 min) Rt = 2.624 min, purity (220 nm): >99%
**¹H NMR** (500 MHz, Chloroform-d) δ 7.74 (d, J = 1.8 Hz, 2H), 7.55 (d, J = 1.9 Hz, 1H), 5.71 (dt, J = 16.8, 9.8 Hz, 1H), 5.12 (d, J = 10.0 Hz, 1H), 5.05 (d, J = 16.8 Hz, 1H), 4.88 (dd, J = 9.4, 7.0 Hz, 1H), 4.79 (d, J = 5.9 Hz, 1H), 4.26 (td, J = 9.5, 6.1 Hz, 1H), 3.96 (t, J = 6.1 Hz, 1H), 3.14 (s, 3H), 3.00 (s, 3H), 2.33 (td, J = 9.6, 7.4 Hz, 1H), 2.19 (d, J = 13.9 Hz, 1H), 2.11 (dq, J = 13.9, 7.4 Hz, 2H), 1.83 (dtd, J = 18.6, 11.5, 10.4, 6.9 Hz, 1H), 1.73 - 1.62 (m, 1H), 1.56 - 1.50 (m, 3H), 1.25 (dq, J = 11.1, 5.9, 5.4 Hz, 2H).
**¹³C NMR** (126 MHz, CDCl₃) δ 171.44, 171.05, 144.15, 136.80, 136.44, 132.83, 125.27, 118.04, 60.73, 59.76, 56.09, 55.13, 54.50, 37.49, 36.69, 32.64, 26.29, 26.25, 25.24, 15.69.
**HRMS** (ESI) *m*/*z* calculated for sum formula C₂₂H₂₇Cl₂N₃O₄S [M+H]: 500.11721 found 500.11716. 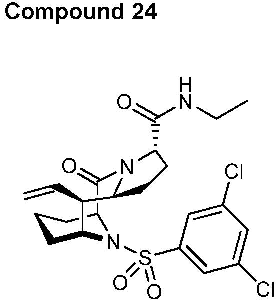
To a solution of ethylamine (2M in THF, 47.5 µL, 95.06 µmol,3.0 eq.) in DMF (300.0 µL) tricyclic acid **3** (15.0 mg, 31.69 µmol, 1.0 eq.) was added. Resulting mixture was treated with DIPEA (22.0 µL, 126.75 µmol, 4.0 eq.) and after 5 min HATU (36.1 mg, 95.06 µmol, 3.0 eq.) was added. The reaction mixture was stirred for 6h at room temperature. After the complete conversion of the starting material, the reaction was quenched by addition of sat. aq. NaHCO₃ solution (2.0 mL) and diluted with DCM. The organic phase was washed with brine (2 x 5 mL), then dried over MgSO₄, filtered and concentrated under reduced pressure. The crude product was purified by preparative HPLC on a C-18 reverse-phase column eluting with a gradient of water (0.1% TFA) and acetonitrile (0.1% TFA) giving after lyophilization 12.5 mg (79%) of **24** as a white solid.
**HPLC** (5 - 100 % solvent B, 3 min) Rt = 2.670 min, purity (220 nm): >99%
**¹H NMR** (500 MHz, Chloroform-d) δ 7.69 (d, J = 1.9 Hz, 2H), 7.62 (t, J = 1.8 Hz, 1H), 6.67 (t, J = 5.6 Hz, 1H), 5.75 (dt, J = 16.9, 9.8 Hz, 1H), 5.20 (dd, J = 10.1, 1.3 Hz, 1H), 5.13 (dd, J = 16.9, 1.3 Hz, 1H), 4.74 (t, J = 8.2 Hz, 1H), 4.49 - 4.42 (m, 1H), 4.29 (td, J = 9.4, 6.0 Hz, 1H), 4.18 - 4.10 (m, 1H), 3.31 (dh, J = 13.1, 6.0 Hz, 2H), 2.51 (td, J = 9.6, 7.9 Hz, 1H), 2.41 - 2.30 (m, 1H), 2.31 - 2.23 (m, 1H), 2.11 (dtd, J = 12.6, 6.1, 3.1 Hz, 1H), 1.91 - 1.79 (m, 1H), 1.79 - 1.68 (m, 2H), 1.67 - 1.54 (m, 2H), 1.43 (tt, J = 12.7, 4.8 Hz, 1H), 1.34 - 1.22 (m, 1H), 1.17 (t, J = 7.3 Hz, 3H).
**¹³C NMR** (126 MHz, CDCl₃) δ 172.53, 171.97, 143.51, 136.65, 136.09, 133.26, 124.90, 118.82, 64.16, 61.09, 56.08, 54.81, 54.09, 34.81, 32.66, 27.46, 25.68, 25.50, 15.41, 14.41.
**HRMS** (ESI) *m*/*z* calculated for sum formula C₂₂H₂₇Cl₂N₃O₄S [M+H]: 500.11721 found 500.11708. 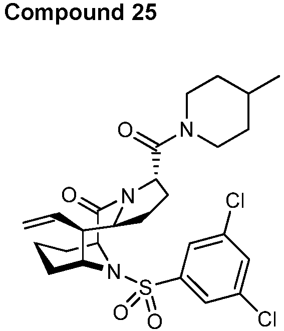
To a solution of 4-methylpiperidine (9.5 mg, 95.06 µmol, 3.0 eq.) in DMF (300.0 µL) tricyclic acid **3** (15.0 mg, 31.69 µmol, 1.0 eq.) was added. Resulting mixture was treated with DIPEA (22.0 µL, 126.75 µmol, 4.0 eq.) and after 5 min HATU (36.1 mg, 95.06 µmol, 3.0 eq.) was added. The reaction mixture was stirred for 6h at room temperature. After the complete conversion of the starting material, the reaction was quenched by addition of sat. aq. NaHCO₃ solution (2.0 mL) and diluted with DCM. The organic phase was washed with brine (2 x 5 mL), then dried over MgSO₄, filtered and concentrated under reduced pressure. The crude product was purified by preparative HPLC on a C-18 reverse-phase column eluting with a gradient of water (0.1% TFA) and acetonitrile (0.1% TFA) giving after lyophilization 15.0 mg (85%) of **25** as a white solid.
**HPLC** (5 - 100 % solvent B, 3 min) Rt = 2.907 min, purity (220 nm): >99%
**¹H NMR** (500 MHz, Chloroform-d) δ 7.74 (d, J = 1.8 Hz, 2H), 7.54 (t, J = 1.9 Hz, 1H), 5.71 (dt, J = 16.9, 9.9 Hz, 1H), 5.12 (dd, J = 10.0, 1.4 Hz, 1H), 5.04 (dd, J = 16.9, 1.4 Hz, 1H), 4.95 (d, J = 8.3 Hz, 1H), 4.80 (s, 1H), 4.54 (t, J = 14.3 Hz, 1H), 4.25 (td, J = 9.4, 6.0 Hz, 1H), 3.95 (q, J = 9.3, 7.6 Hz, 1H), 3.90 (s, 1H), 3.10 (d, J = 55.1 Hz, 1H), 2.63 (dt, J = 35.3, 12.3 Hz, 1H), 2.33 (q, J = 9.1 Hz, 1H), 2.20 (d, J = 13.8 Hz, 1H), 2.09 (ddq, J = 14.5, 7.1, 3.7, 3.1 Hz, 2H), 1.78 (s, 1H), 1.72 - 1.59 (m, 4H), 1.56 - 1.50 (m, 3H), 1.24 (dddd, J = 19.8, 13.5, 10.1, 5.6 Hz, 3H), 1.07 (dd, J = 16.4, 9.7 Hz, 1H), 0.95 (d, J = 6.4 Hz, 3H).
**¹³C NMR** (126 MHz, CDCl₃) δ 171.06, 169.32, 144.15, 136.86, 136.43, 132.81, 125.27, 117.99, 60.94, 59.76, 56.13, 55.07, 54.53, 43.69, 43.17, 33.81, 32.54, 31.41, 30.97, 26.46, 26.22, 25.26, 21.65, 15.68.
**HRMS** (ESI) *m*/*z* calculated for sum formula C₂₆H₃₃Cl₂N₃O₄S [M+H]: 554.16416 found 554.16407. 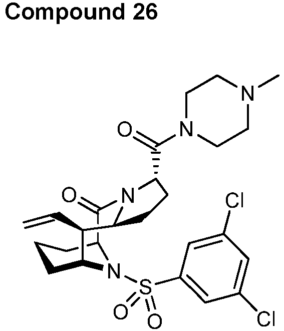
To a solution of 1-methylpiperazine (9.5 mg, 95.06 µmol, 3.0 eq.) in DMF (300.0 µL) tricyclic acid **3** (15.0 mg, 31.69 µmol,1.0 eq.) was added. Resulting mixture was treated with DIPEA (22.0 µL, 126.75 µmol, 4.0 eq.) and after 5 min HATU (36.1 mg, 95.06 µmol, 3.0 eq.) was added. The reaction mixture was stirred for 6h at room temperature. After the complete conversion of the starting material, the reaction was quenched by addition of sat. aq. NaHCO₃ solution (2.0 mL) and diluted with DCM. The organic phase was washed with brine (2 x 5 mL), then dried over MgSO₄, filtered and concentrated under reduced pressure. The crude product was purified by preparative HPLC on a C-18 reverse-phase column eluting with a gradient of water (0.1% TFA) and acetonitrile (0.1% TFA) giving after lyophilization 15.8 mg (90%) of **26** as a white solid.
**HPLC** (5 - 100 % solvent B, 3 min) Rt = 2.151 min, purity (220 nm): >99%
**¹H NMR** (500 MHz, Chloroform-d) δ 7.72 (s, 2H), 7.56 (s, 1H), 5.69 (dt, J = 18.5, 9.8 Hz, 1H), 5.10 (dd, J = 26.1, 13.2 Hz, 2H), 4.90 - 4.82 (m, 1H), 4.77 - 4.65 (m, 2H), 4.28 (d, J = 14.7 Hz, 1H), 4.18 (q, J = 8.5 Hz, 1H), 3.90 (d, J = 25.7 Hz, 1H), 3.70 (q, J = 23.5, 19.4 Hz, 1H), 3.55 (dd, J = 29.8, 11.6 Hz, 2H), 3.38 (s, 1H), 3.17 (t, J = 13.3 Hz, 1H), 3.06 (s, 1H), 2.92 (s, 3H), 2.52 - 2.27 (m, 1H), 2.11 (d, J = 17.1 Hz, 3H), 1.80 (d, J = 14.2 Hz, 1H), 1.68 (s, 1H), 1.54 (d, J = 11.5 Hz, 3H), 1.32 - 1.19 (m, 2H).
**¹³C NMR** (126 MHz, CDCl₃) δ 174.81, 170.79, 144.06, 136.54, 133.07, 132.95, 125.14, 118.34, 60.06, 56.06, 54.91, 54.52, 53.90, 53.43, 44.04, 43.65, 32.87, 27.09, 26.28, 25.23, 15.52.
**HRMS** (ESI) *m*/*z* calculated for sum formula C₂₅H₈₂Cl₂N₄O₄S [M+H]: 555.15941 found 555.15935. 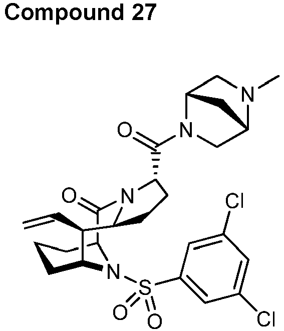
To a solution of (1*R*,4*R*)-5-methyl-2,5-diazabicyclo-[2.2.1]heptane dihydrochloride (17.6 mg, 95.06 µmol, 3.0 eq.) in DMF (300.0 µL) tricyclic acid **3** (15.0 mg, 31.69 µmol, 1.0 eq.) was added. Resulting mixture was treated with DIPEA (22.0 µL, 126.75 µmol, 4.0 eq.) and after 5 min HATU (36.1 mg, 95.06 µmol, 3.0 eq.) was added. The reaction mixture was stirred for 4h at room temperature. After the complete conversion of the starting material, the reaction was quenched by addition of sat. aq. NaHCO₃ solution (2.0 mL) and diluted with DCM. The organic phase was washed with brine (2 x 5 mL), then dried over MgSO₄, filtered and concentrated under reduced pressure. The crude product was purified by preparative HPLC on a C-18 reverse-phase column eluting with a gradient of water (0.1% TFA) and acetonitrile (0.1% TFA) giving after lyophilization 16.7 mg (93%) of **27** as a white solid.
**HPLC** (5 - 100 % solvent B, 3 min) Rt = 2.126 min, purity (220 nm): 99%
**¹H NMR** (500 MHz, Chloroform-d) δ 7.71 (d, J = 1.9 Hz, 2H), 7.56 (t, J = 1.9 Hz, 1H), 5.71 (dt, J = 16.9, 9.8 Hz, 1H), 5.17 - 5.10 (m, 1H), 5.06 (d, J = 16.8 Hz, 1H), 4.74 (s, 1H), 4.64 (d, J = 6.2 Hz, 1H), 4.49 (d, J = 12.4 Hz, 2H), 4.38 (dd, J = 10.2, 6.5 Hz, 1H), 4.21 (td, J = 10.0, 5.8 Hz, 1H), 3.96 (t, J = 6.1 Hz, 1H), 3.75 (d, J = 11.1 Hz, 1H), 3.61 (d, J = 11.4 Hz, 1H), 3.09 (d, J = 23.4 Hz, 3H), 2.94 (d, J = 28.8 Hz, 1H), 2.32 (dt, J = 17.0, 10.5 Hz, 2H), 2.13 (dd, J = 17.6, 12.5 Hz, 3H), 2.03 (dt, J = 12.8, 6.4 Hz, 1H), 1.98 - 1.87 (m, 1H), 1.62 (ddd, J = 10.6, 6.6, 3.3 Hz, 1H), 1.58 - 1.49 (m, 3H), 1.26 (dq, J = 12.8, 7.8, 7.1 Hz, 2H).
**¹³C NMR** (126 MHz, CDCl₃) δ 170.63, 168.62, 144.08, 136.53, 136.51, 132.92, 125.14, 118.15, 64.96, 61.19, 60.09, 59.94, 56.00, 55.65, 55.25, 54.45, 46.58, 36.68, 36.07, 33.15, 27.29, 26.43, 25.33, 15.63.
**HRMS** (ESI) *m*/*z* calculated for sum formula C₂₆H₃₂Cl₂N₄O₄S [M+H]: 567.15941 found 567.15991. 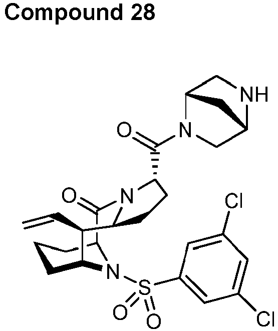
To a solution of (1S,4S)-2-Boc-2,5-diazabicyclo[2.2.1]heptane (21.4 mg, 107.74 µmol,3.0 eq.) in DMF (350.0 µL) tricyclic acid **3** (17.0 mg, 35.91 µmol, 1.0 eq.) was added. Resulting mixture was treated with DIPEA (25.0 µL, 143.65 µmol, 4.0 eq.) and after 5 min HATU (41.0 mg, 107.74 µmol, 3.0 eq.) was added. The reaction mixture was stirred for 6h at room temperature. After the complete conversion of the starting material, the reaction was quenched by addition of sat. aq. NaHCO₃ solution (2.0 mL) and diluted with DCM. The organic phase was washed with brine (2 x 5 mL), then dried over MgSO₄, filtered and concentrated under reduced pressure. The residue after evaporation was dissolved in DCM (2.4 mL) and TFA was added (275.0 µL, 3.60 mmol, 100.0 eq.). The reaction mixture was stirred for 1.5h at room temperature. After completion, the reaction was quenched by slow addition of sat. aq. NaHCO₃ solution and diluted with DCM. The aqueous layer was extracted with mixture of CHCl₃/*i*-PrOH (3:1, 4 x 15 mL). The organic layers were combined, dried over MgSO₄, filtered and concentrated under reduced pressure. The crude product was purified by preparative HPLC on a C-18 reverse-phase column eluting with a gradient of water (0.1% TFA) and acetonitrile (0.1% TFA) giving after lyophilization 18.3 mg (92% over 2 steps) of **28** as a white solid.
**HPLC** (5 - 100 % solvent B, 3 min) Rt = 2.095 min, purity (220 nm): >99%
**¹H NMR** (500 MHz, Chloroform-d) δ 7.72 (dd, J = 9.6, 1.9 Hz, 2H), 7.56 (dt, J = 8.0, 1.9 Hz, 1H), 5.70 (dt, J = 16.8, 9.8 Hz, 1H), 5.16 - 5.09 (m, 2H), 5.06 (dd, J = 16.8, 9.5 Hz, 1H), 4.69 (d, J = 5.5 Hz, 1H), 4.50 (d, J = 16.5 Hz, 1H), 4.38 (dd, J = 9.6, 6.7 Hz, 1H), 4.21 (dtd, J = 33.9, 9.6, 6.0 Hz, 1H), 4.00 (s, 1H), 3.96 - 3.82 (m, 3H), 3.51 (dd, J = 39.9, 10.7 Hz, 1H), 3.37 (s, 1H), 2.41 - 2.24 (m, 1H), 2.18 - 2.04 (m, 4H), 1.86 - 1.73 (m, 1H), 1.72 - 1.60 (m, 1H), 1.51 (d, J = 8.7 Hz, 3H), 1.33 - 1.11 (m, 2H).
**¹³C NMR** (126 MHz, CDCl₃) δ 170.53, 169.65, 144.10, 136.65, 136.47, 132.87, 125.16, 118.10, 61.86, 61.01, 59.98, 58.30, 56.08, 55.06, 54.51, 53.83, 52.25, 49.82, 34.81, 32.85, 26.32, 25.31, 15.60.
**HRMS** (ESI) *m*/*z* calculated for sum formula C₂₅H₃₀Cl₂N₄O₄S [M+H]: 553.14376 found 553.14473. 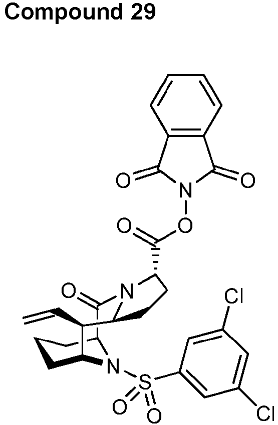
An oven-dried Schlenk flask was charged with tricyclic acid **3** (200.0 mg, 0.42 mmol, 1.0 eq.), N-hydroxyphthalimide (75.8 mg, 0.46 mmol, 1.1 eq.), and DMAP (5.2 mg, 0.04 mmol, 0.10 eq.) were added, the flask was cooled to 0 °C, and anhydrous DCM (2.8 mL) was added. While the reaction mixture was stirring at 0 °C, EDC·HCl (89.1 mg, 0.46 mmol, 1.1 eq.) was added. The ice bath was removed and the reaction mixture was stirred at room temperature for 17h. After completion, the reaction was diluted with DCM and carefully quenched with 1 M HCl (10 mL). The aqueous solution was extracted with DCM (3 x 30 mL). The combined organic layers were washed with aq. 10% K₂CO₃ solution (1x), brine (1x), dried over MgSO₄, filtered and concentrated under reduced pressure. The crude ester was purified by flash column chromatography (Biotage Isolera One, 0-60% ethyl acetate in cyclohexane) to give 254.0 mg (97%) of **29** as white solid.
**HPLC** (5 - 100 % solvent B, 3 min) Rt = 2.973 min, purity (220 nm): 97%
**¹H NMR** (500 MHz, Chloroform-d) δ 7.88 (dd, J = 5.5, 3.1 Hz, 2H), 7.77 (dd, J = 5.5, 3.1 Hz, 2H), 7.69 (d, J = 1.8 Hz, 2H), 7.52 (t, J = 1.9 Hz, 1H), 5.73 (dt, J = 16.9, 9.8 Hz, 1H), 5.15 (dd, J = 10.1, 1.4 Hz, 1H), 5.10 (dd, J = 16.9, 1.4 Hz, 1H), 4.86 - 4.77 (m, 2H), 4.19 (td, J = 9.7, 5.9 Hz, 1H), 4.02 (td, J = 6.0, 5.1, 1.9 Hz, 1H), 2.43 (dtd, J = 12.7, 6.7, 2.1 Hz, 1H), 2.40 - 2.28 (m, 2H), 2.17 (dtd, J = 12.6, 6.3, 2.1 Hz, 1H), 2.08 (tdd, J = 12.4, 9.9, 6.6 Hz, 1H), 1.78 (tdd, J = 12.2, 9.7, 6.6 Hz, 1H), 1.59 - 1.54 (m, 3H), 1.37 - 1.21 (m, 2H).
**¹³C NMR** (126 MHz, CDCl₃) δ 170.65, 167.56, 161.73, 144.03, 136.52, 136.46, 134.78, 132.84, 129.17, 125.15, 124.09, 118.34, 60.41, 59.66, 56.03, 55.13, 54.54, 32.97, 27.25, 26.41, 25.57, 15.66.
**HRMS** (ESI) *m*/*z* calculated for sum formula C₂₈H₂₅Cl₂N₃O₇S [M+H]: 618.08630 found 618.08639. 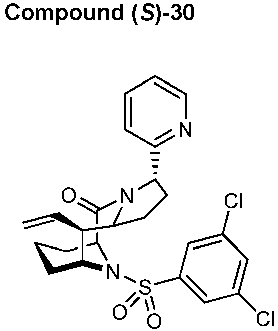
To an oven-dried 1-dram vial NiCl₂(bpy) (3.5 mg, 12.20 µmol, 0.25 eq.), NHP ester **29** (33.2 mg, 53.66 µmol, 1.1 eq.) and granular zinc (25.5 mg, 390.24 µmol, 8.0 eq.) were added. Next, anhydrous DMA (250.0 µL) was added followed by 2-iodopyridine (10.0 mg, 48.78 µmol, 1.0 eq.). The reaction mixture was stirred at room temperature under stream of argon until TMSCI (19.0 µL, 146.34 µmol, 3.0 eq.) was added. The vial was capped with a screw cap fitted with a PTFE-faced silicone septum and equipped with the argon ballon. The reaction mixture was stirred at 900 rpm for 3h at room temperature. Upon reaction completion the reaction mixture was filtered through a short plug of silica gel and eluted with EtOAc. The solvents were removed under reduced pressure. The resulting residue was purified by preparative HPLC on a C-18 reverse-phase column eluting with a gradient of water (0.1% TFA) and acetonitrile (0.1% TFA) giving after lyophilization 5.2 mg of **(*S*)-30** and 2.4 mg of **(*R*)-30** as white solids.
**HPLC** (5 - 100 % solvent B, 3 min) Rt = 2.589 min, purity (220 nm): >99%
**¹H NMR** (500 MHz, Chloroform-d) δ 8.80 (d, J = 5.5 Hz, 1H), 8.16 (t, J = 7.9 Hz, 1H), 7.69 (q, J = 2.1 Hz, 3H), 7.59 (t, J = 2.0 Hz, 1H), 7.56 (d, J = 6.7 Hz, 1H), 5.83 - 5.72 (m, 1H), 5.58 (t, J = 8.6 Hz, 1H), 5.18 (dd, J = 22.2, 13.4 Hz, 2H), 4.48 (dd, J = 9.9, 6.2 Hz, 1H), 4.44 (d, J = 6.0 Hz, 1H), 4.10 (t, J = 6.2 Hz, 1H), 2.64 (dt, J = 13.0, 6.9 Hz, 1H), 2.55 (q, J = 9.1 Hz, 1H), 2.17 (d, J = 11.7 Hz, 1H), 2.13 (d, J = 6.5 Hz, 1H), 1.90 (qd, J = 12.2, 6.3 Hz, 1H), 1.73 (td, J = 11.3, 10.7, 6.2 Hz, 1H), 1.66 (d, J = 14.2 Hz, 1H), 1.58 (q, J = 14.0 Hz, 2H), 1.32 (td, J = 13.5, 12.8, 6.0 Hz, 1H), 1.16 (tt, J = 13.3, 5.5 Hz, 1H).
**¹³C NMR** (126 MHz, CDCl₃) δ 170.99, 159.84, 144.02, 143.75, 142.72, 136.67, 136.00, 133.09, 124.85, 123.91, 121.62, 119.00, 62.93, 61.15, 56.33, 54.78, 54.64, 32.84, 31.92, 25.75, 25.21, 15.45.
**HRMS** (ESI) *m*/*z* calculated for sum formula C₂₄H₂₅Cl₂N₃O₃S [M+H]: 506.10664 found 506.10698.

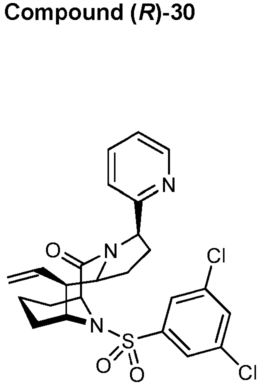

**HPLC** (5 - 100 % solvent B, 3 min) Rt = 2.813 min, purity (220 nm): 95%
**¹H NMR** (500 MHz, Chloroform-d) δ 8.67 (d, J = 5.0 Hz, 1H), 7.80 (t, J = 7.8 Hz, 1H), 7.71 (d, J = 1.8 Hz, 2H), 7.57 (t, J = 1.7 Hz, 1H), 7.30 (dd, J = 14.5, 7.3 Hz, 2H), 5.84 (dt, J = 16.7, 9.8 Hz, 1H), 5.43 (d, J = 8.6 Hz, 1H), 5.16 (d, J = 16.8 Hz, 1H), 5.12 (d, J = 10.0 Hz, 1H), 4.68 (d, J = 5.8 Hz, 1H), 4.34 (td, J = 9.6, 6.1 Hz, 1H), 4.04 (t, J = 5.7 Hz, 1H), 3.04 (q, J = 9.3 Hz, 1H), 2.29 (tq, J = 17.1, 8.3 Hz, 1H), 2.13 (d, J = 13.8 Hz, 1H), 1.92 (t, J = 5.9 Hz, 2H), 1.89 (d, J = 10.9 Hz, 1H), 1.83 - 1.71 (m, 1H), 1.64 (d, J = 13.7 Hz, 1H), 1.56 - 1.49 (m, 1H), 1.29 (dd, J = 9.0, 4.7 Hz, 1H), 1.18 (td, J = 13.6, 6.9 Hz, 1H).
**¹³C NMR** (126 MHz, CDCl₃) δ 169.43, 160.76, 147.27, 144.26, 138.77, 137.34, 136.51, 132.85, 125.06, 122.68, 121.68, 117.51, 64.99, 59.88, 56.54, 55.05, 54.51, 30.56, 30.44, 27.54, 26.48, 15.76.
**HRMS** (ESI) *m*/*z* calculated for sum formula C₂₄H₂₅Cl₂N₃O₃S [M+H]: 506.10664 found 506.10714. 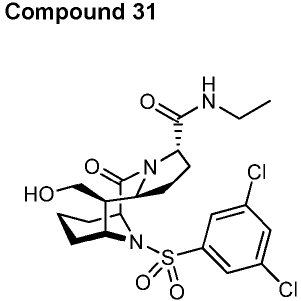
To an ice-cold solution of tricylic compound **24** (7.9 mg, 15.8 µmol,1.0 eq.) in dioxane/H₂O (3:1, 0.8 mL) 2,6-lutidine (8.0 µL, 63.1 µmol, 4.0 eq.), NalO₄ (27.0 mg, 126.1 µmol, 8.0 eq.) and OsO₄ (2.5 wt. % in *t*BuOH, 20.5 µL, 1.6 µmol, 0.1 eq.) were added. The resulting milky solution was stirred for 17h at room temperature. The reaction was quenched by addition of sat. aq. Na₂S₂O₃ solution (1.0 mL) and water (2.0 mL). The aqueous phase was extracted with DCM (3 x 15 mL). The organic layers were combined, dried over MgSO₄, filtered and concentrated under reduced pressure. Obtained intermediate was dissolved in EtOH (2.0 mL) and cooled to 0 °C. Then NaBH₄ (3.0 mg, 63.1 µmol, 4.0 eq.) was added and the mixture was stirred for 17h at room temperature. After the complete conversion of the starting material, the reaction was quenched by addition of sat. aq. NaHCO₃ solution. The aqueous phase was extracted with DCM (4 x 15 mL). The combined organic layers were dried over MgSO₄, filtered and concentrated under reduced pressure. The crude product was purified by preparative HPLC on a C-18 reverse-phase column eluting with a gradient of water (0.1% TFA) and acetonitrile (0.1%TFA) giving after lyophilization 6.2 mg (78% over 2 steps) of **31** as a white solid.
**HPLC** (5 - 100 % solvent B, 3 min) Rt = 2.245 min, purity (220 nm): >99%
**¹H NMR** (500 MHz, Chloroform-d) δ 7.70 (d, J = 1.8 Hz, 2H), 7.59 (d, J = 1.9 Hz, 1H), 6.55 (t, J = 5.5 Hz, 1H), 4.65 (t, J = 8.2 Hz, 1H), 4.46 (t, J = 9.5 Hz, 2H), 4.29 (td, J = 9.3, 5.5 Hz, 1H), 3.80 (dd, J = 11.1, 4.3 Hz, 1H), 3.72 (dd, J = 11.1, 2.9 Hz, 1H), 3.51 (s, 1H), 3.33 - 3.25 (m, 2H), 2.41 (q, J = 7.1 Hz, 1H), 2.31 (d, J = 5.9 Hz, 1H), 2.25 (d, J = 14.0 Hz, 1H), 2.02 (td, J = 8.6, 8.0, 4.1 Hz, 1H), 1.85 (tt, J = 10.1, 5.2 Hz, 2H), 1.72 (d, J = 10.2 Hz, 1H), 1.57 - 1.51 (m, 3H), 1.26 (td, J = 11.9, 11.3, 5.7 Hz, 1H), 1.15 (t, J = 7.3 Hz, 3H).
**¹³C NMR** (126 MHz, CDCl₃) δ 172.42, 171.76, 143.58, 136.62, 133.22, 124.97, 64.07, 62.86, 60.54, 56.17, 52.44, 48.98, 34.73, 31.77, 27.78, 27.53, 25.81, 15.31, 14.49.
**HRMS** (ESI) *m*/*z* calculated for sum formula C₂₁H₂₇Cl₂N₃O₅S [M+H]: 504.11212 found 504.11248. 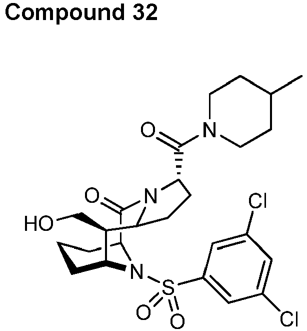
To an ice-cold solution of tricylic compound **25** (7.8 mg, 14.07 µmol,1.0 eq.) in dioxane/H₂O (3:1, 0.8 mL) 2,6-lutidine (7.0 µL, 56.26 µmol, 4.0 eq.), NalO₄ (24.1 mg, 112.53 µmol, 8.0 eq.) and OsO₄ (2.5 wt. % in tBuOH, 18.5 µL, 1.41 µmol, 0.1 eq.) were added. The resulting milky solution was stirred for 17h at room temperature. The reaction was quenched by addition of sat. aq. Na₂S₂O₃ solution (1.0 mL) and water (2.0 mL). The aqueous phase was extracted with DCM (3 x 15 mL). The organic layers were combined, dried over MgSO₄, filtered and concentrated under reduced pressure. Obtained intermediate was dissolved in EtOH (2.0 mL) and cooled to 0 °C. Then NaBH₄ (3.0 mg, 77.36 µmol, 5.5 eq.) was added and the mixture was stirred for 17h at room temperature. After the complete conversion of the starting material, the reaction was quenched by addition of sat. aq. NaHCO₃ solution. The aqueous phase was extracted with DCM (4 x 15 mL). The combined organic layers were dried over MgSO₄, filtered and concentrated under reduced pressure. The crude product was purified by preparative HPLC on a C-18 reverse-phase column eluting with a gradient of water (0.1% TFA) and acetonitrile (0.1%TFA) giving after lyophilization 6.0 mg (76% over 2 steps) of **32** as a white solid.
**HPLC** (5 - 100 % solvent B, 3 min) Rt = 2.461 min, purity (220 nm): 91%
**¹H NMR** (500 MHz, Chloroform-d) δ 7.82 (d, J = 1.9 Hz, 2H), 7.56 (s, 1H), 4.94 (s, 1H), 4.86 (s, 1H), 4.56 (s, 1H), 4.33 (t, J = 5.3 Hz, 1H), 4.18 - 4.06 (m, 4H), 3.93 (s, 1H), 3.77 (dd, J = 11.1, 4.3 Hz, 1H), 3.68 (dd, J = 11.0, 2.9 Hz, 1H), 3.12 (d, J = 56.3 Hz, 1H), 2.65 (d, J = 33.7 Hz, 1H), 2.30 (d, J = 5.9 Hz, 1H), 2.25 (d, J = 13.8 Hz, 1H), 2.16 (s, 1H), 1.90 (q, J = 8.2, 6.3 Hz, 1H), 1.81 (d, J = 9.9 Hz, 2H), 1.67 (d, J = 20.4 Hz, 2H), 1.56 (q, J = 11.8 Hz, 3H), 1.37 - 1.26 (m, 2H), 0.98 (d, J = 6.2 Hz, 3H).
**¹³C NMR** (126 MHz, CDCl₃) δ 171.07, 169.36, 143.98, 136.30, 132.78, 125.50, 63.07, 60.35, 59.09, 56.18, 52.11, 49.87, 43.65, 33.85, 31.70, 31.47, 27.50, 26.59, 26.33, 21.67, 15.64.
**HRMS** (ESI) *m*/*z* calculated for sum formula C₂₅H₃₃Cl₂N₃O₅S [M+H]: 558.15907 found 558.15952.

## Claims

1. A compound of the general formula **1:** 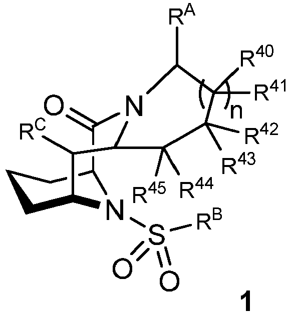
wherein n is 0 or 1,
and wherein **R^{A}** represents:
- CH₂OR¹⁶,
- CH₂NR³⁸R³⁹, 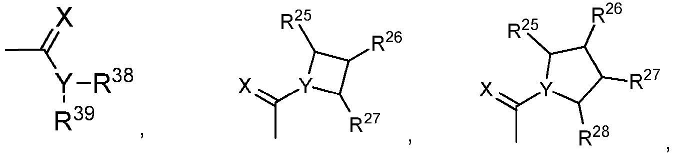 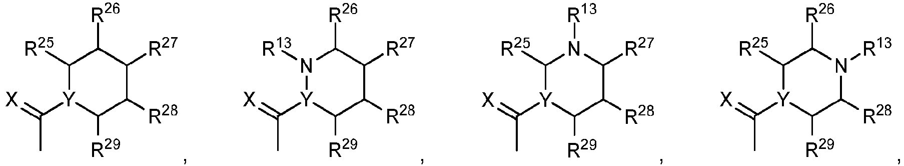 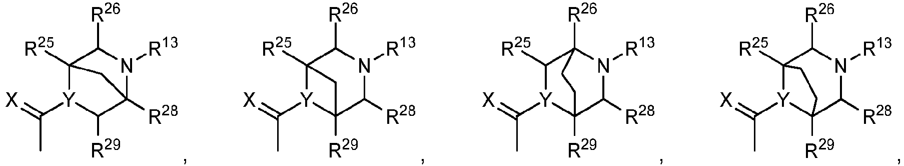 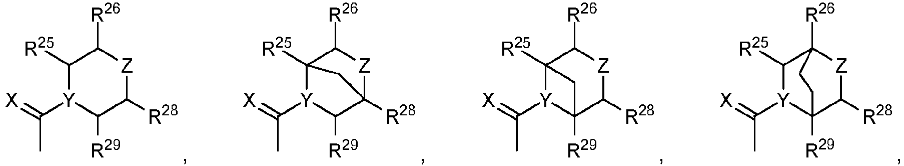 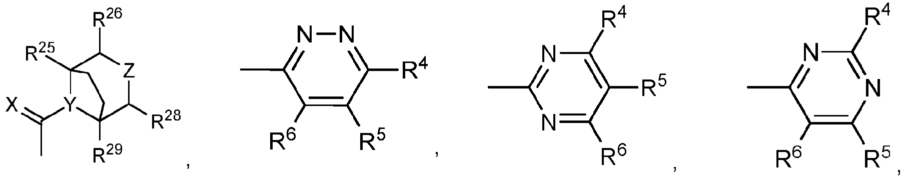 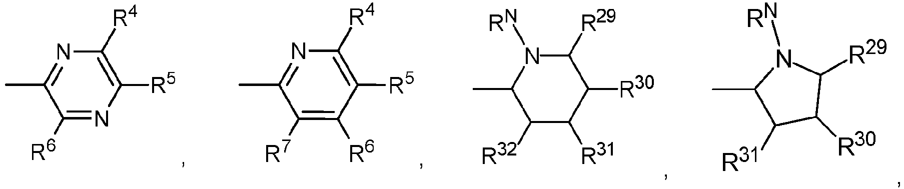 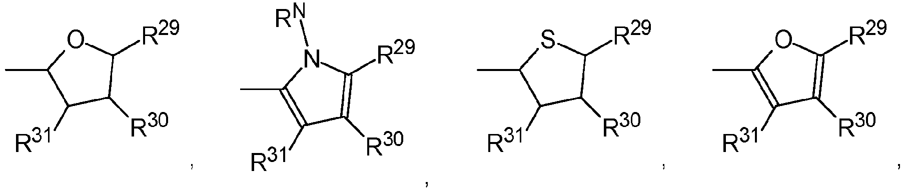 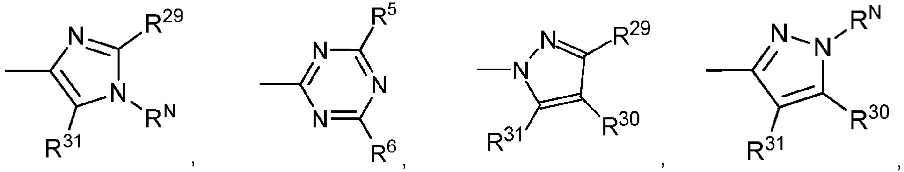 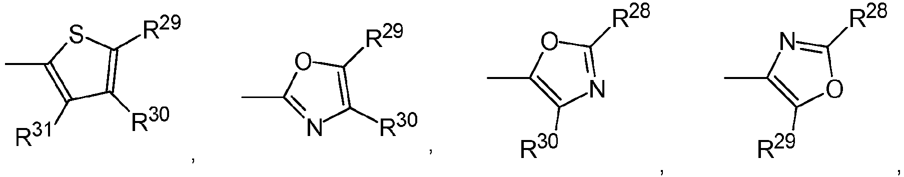 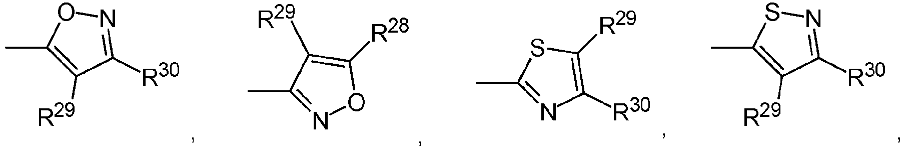 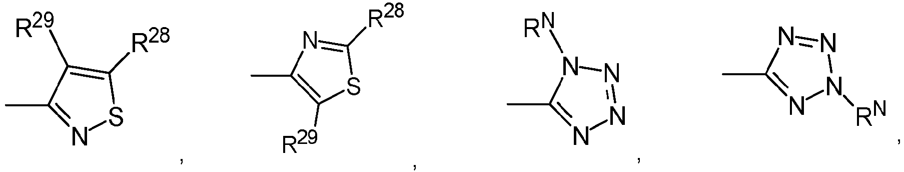 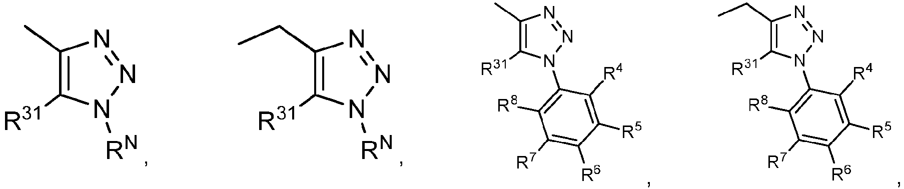 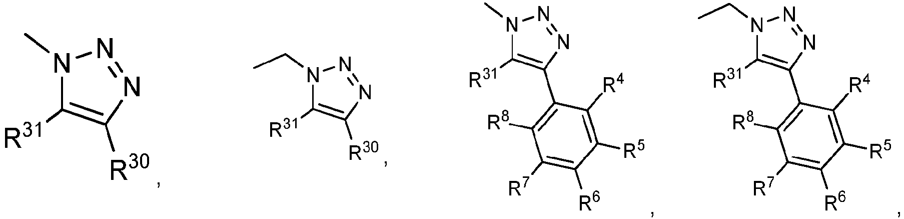 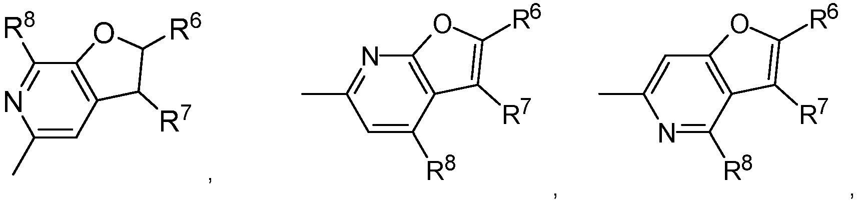 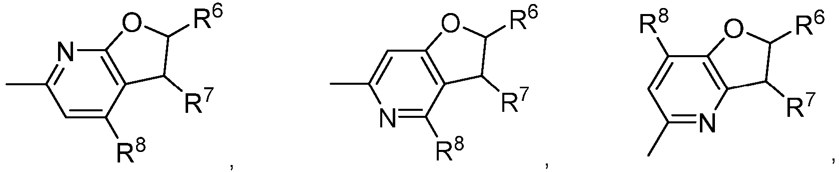 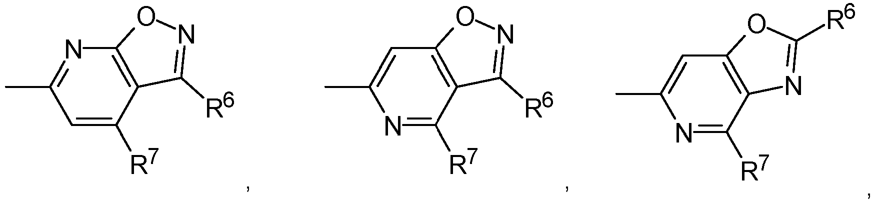 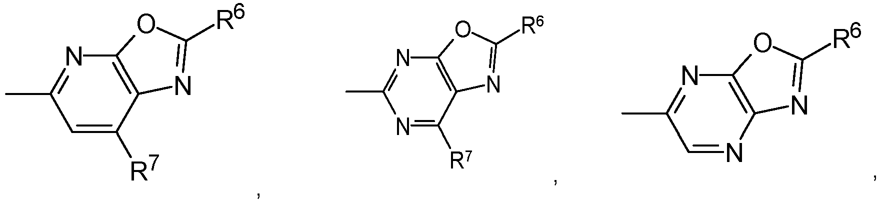 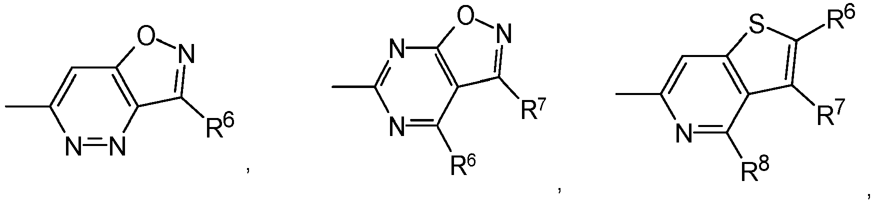 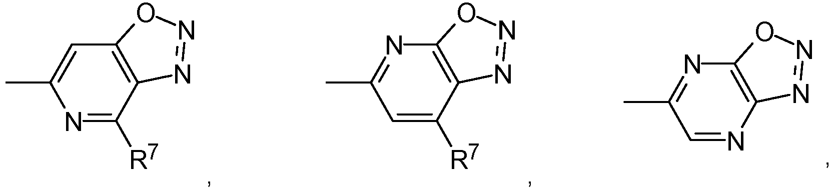 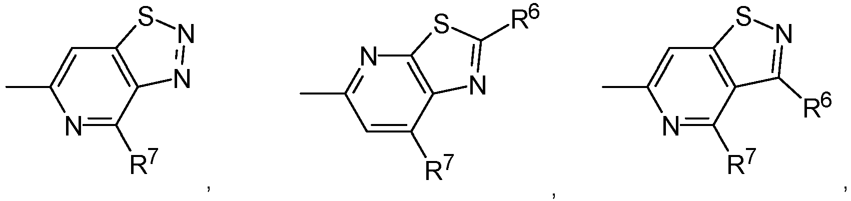 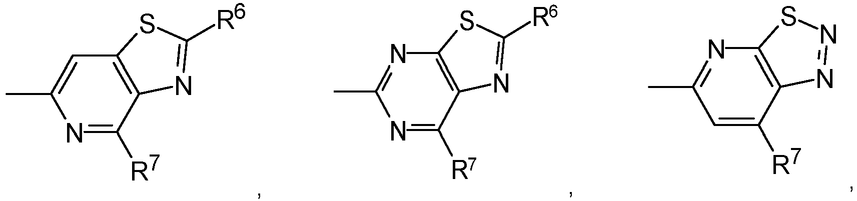 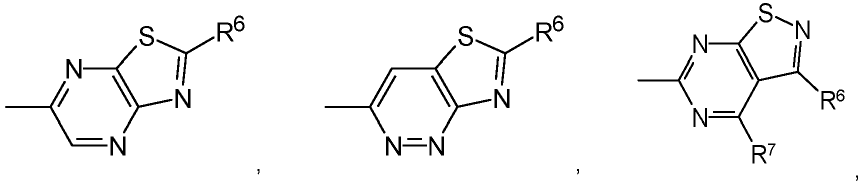 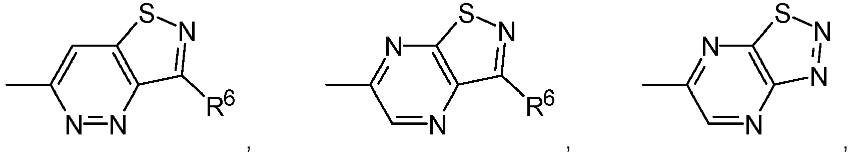 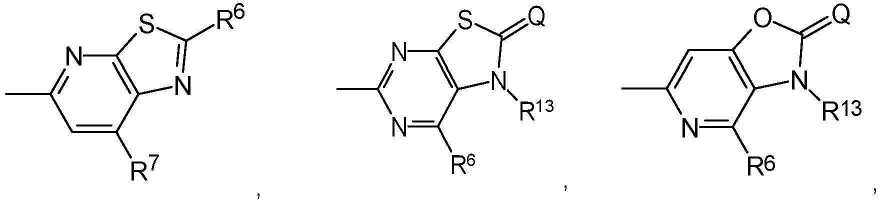 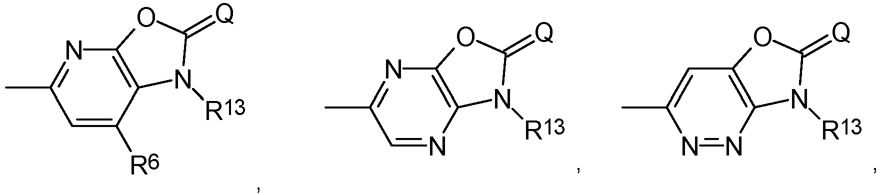 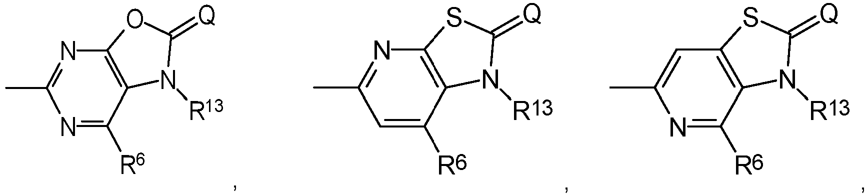 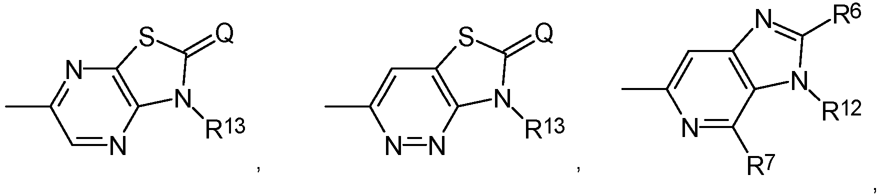 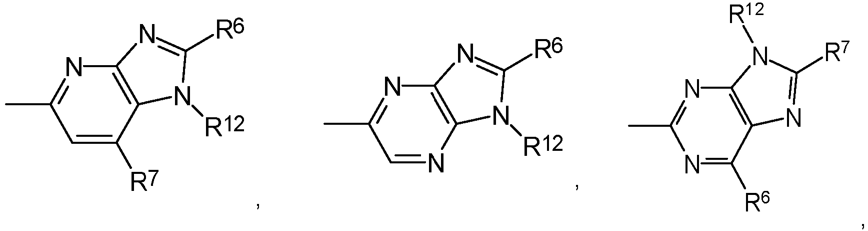 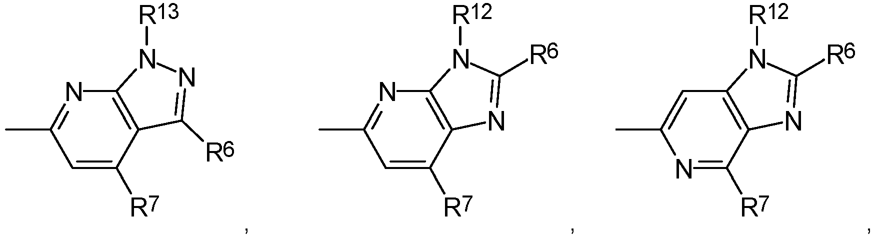 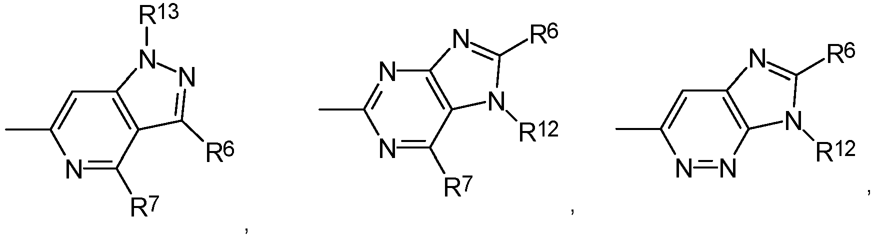 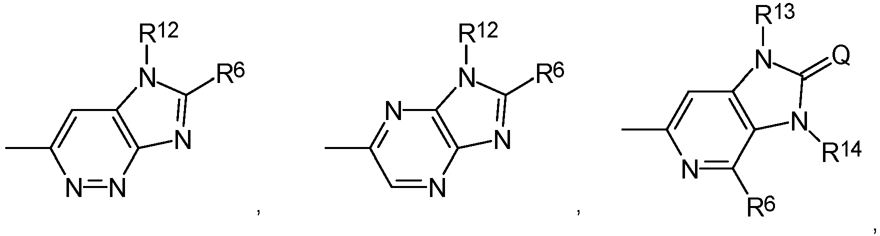 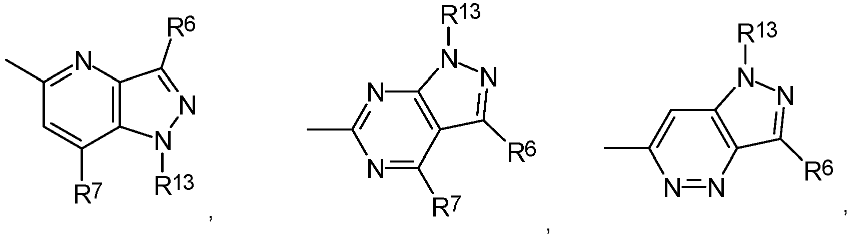 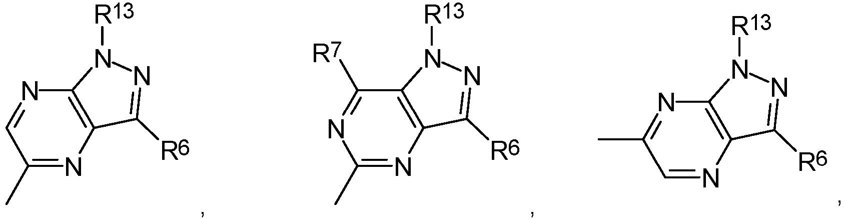 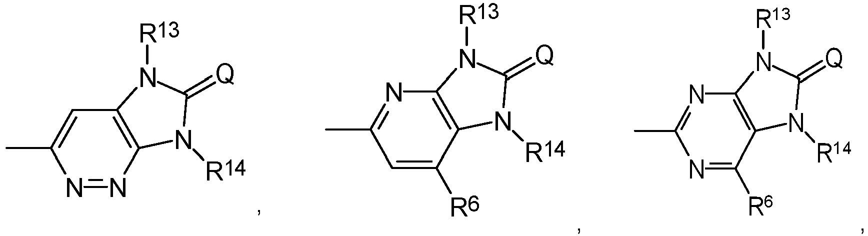 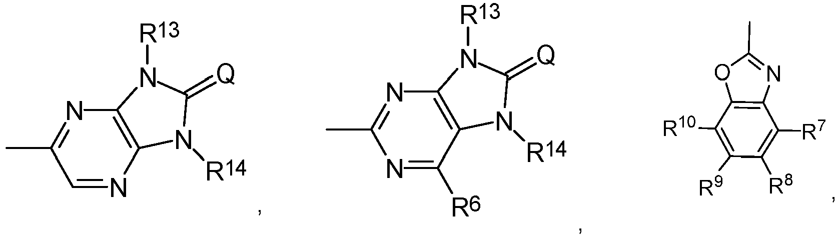 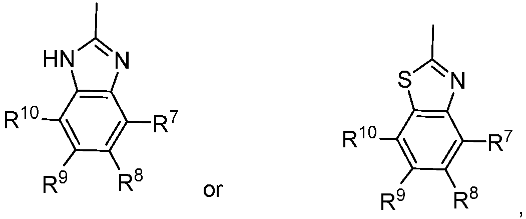
wherein **X** represents O, S, or H, H (e.g., C=X represents CH₂),
wherein **Y** represents N,-CH- or -CH₂-,
wherein **Z** represents O, N-R^{N}, C=O or SO₂
wherein **Q** represents =O, =S, or =N-R¹²;
wherein **R^{N}** represents -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H_{5.}, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, -COC₃H₇, -CO-cyclo-C₃H₅, -COCH(CH₃)₂, -COC(CH₃)₃, -COPh, -COCN, -COOCH₃, -COOC₂H₅, -COOC₃H₇, -COO-cyclo-C₃H₅, -COOCH(CH₃)₂, -COOC(CH₃)₃, -COCH₂Ph, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -CH₂NH₂, -CH₂NHCH₃, -CH₂NHC₂H₅, -CH₂NHC₃H₇, -CH₂NH-cyclo-C₃H₅, -CH₂NH[CH(CH₃)₂], -CH₂NH[C(CH₃)₃], -CH₂N(CH₃)₂, -CH₂N(C₂H₅)₂, -CH₂N(C₃H₇)₂, -CH₂N(cyclo-C₃H₅)₂, -CH₂N[CH(CH₃)₂]₂, -CH₂N[C(CH₃)₃]₂, -CH₂-NHCOCH₃, -CH₂-NHCHO, -CH₂-NHSO₂CH₃, -CONH₂, -CONHCH₃, -CONHC₂H₅, -CONHC₃H₇, -CH₂-NH(C₂H₅), -SO₂CH₃, -SO₂C₂H₅, -SO₂CH₂Ph, -SO₂C₃H₇, -SO₂-cyclo-C₃H₅, -SO₂CH(CH₃)₂, -SO₂C(CH₃)₃, -SO₂Ph, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂Hs, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH₂-C≡C-C₂H₅, -CH=CH-CH=C(CH₃)₂, -CH₂-CH(CH₃)-CH₂-C≡CH, -C₂H₄-C≡C-CH₃, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C≡C-C₃H₇, -CH(CH₃)C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H_{y})-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, - C≡C-CH(CH₃)-C≡CH, -CH(CH₃)-C≡C-C≡CH, -CH(C≡CH)-CH₂-C≡CH, -CH(C≡CH)-C≡C-CH₃,
wherein **R^{B}** represents 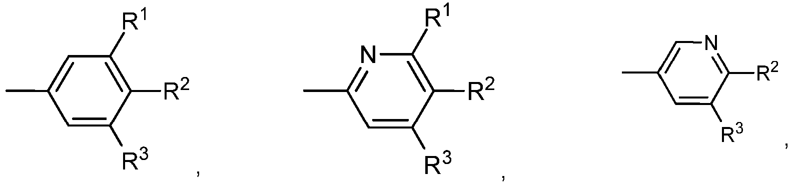 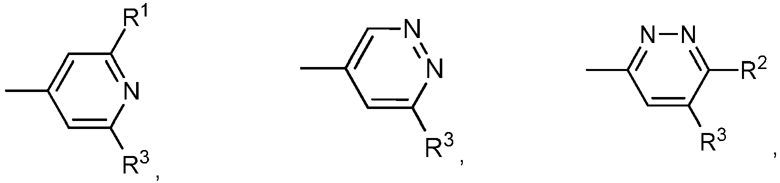 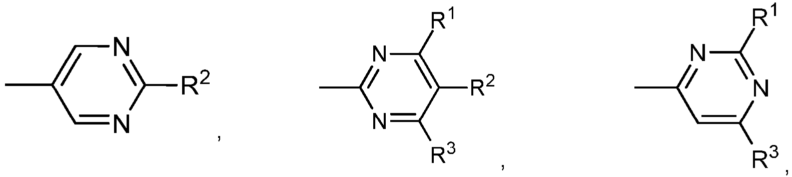 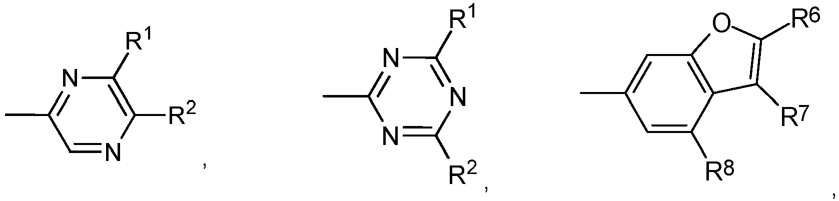 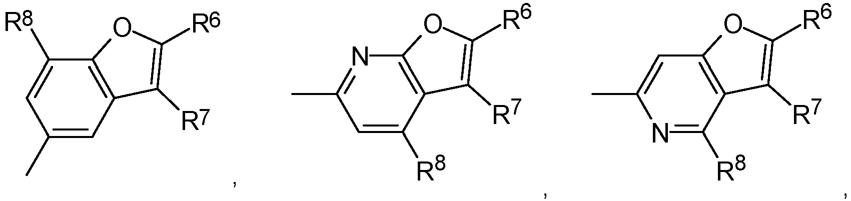 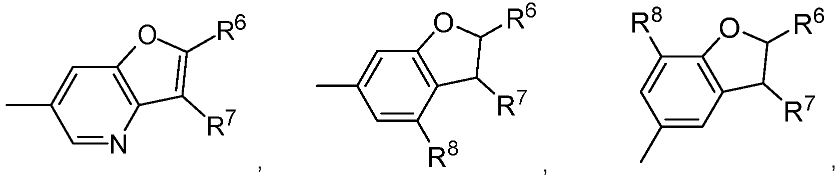 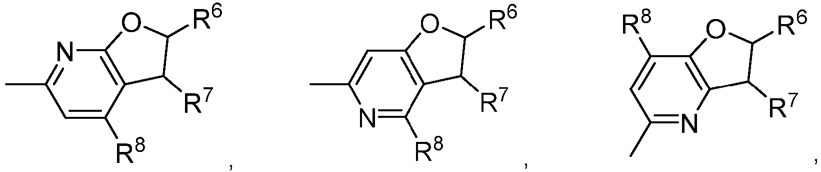 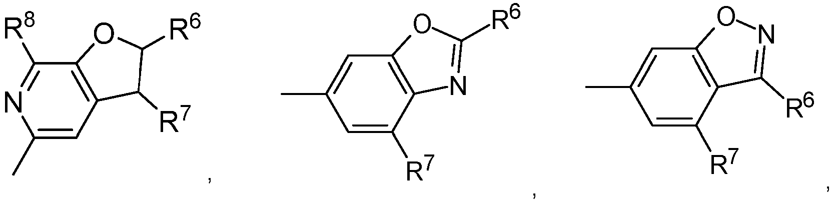 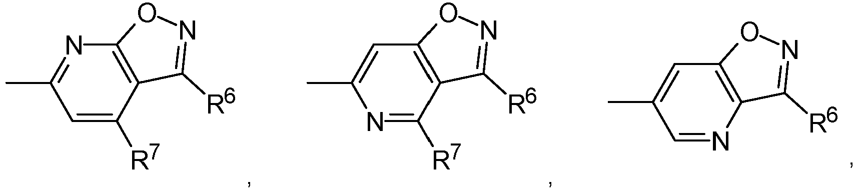 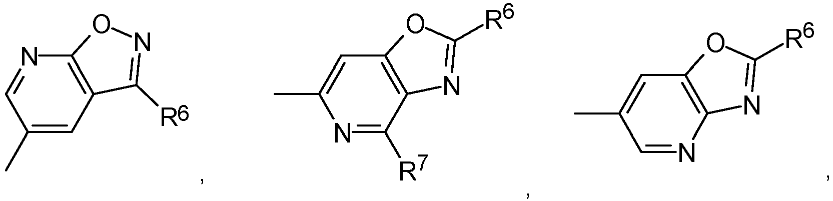 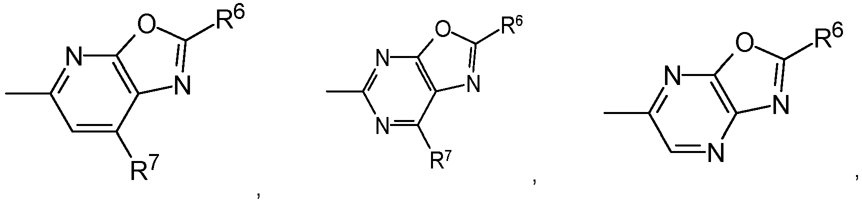 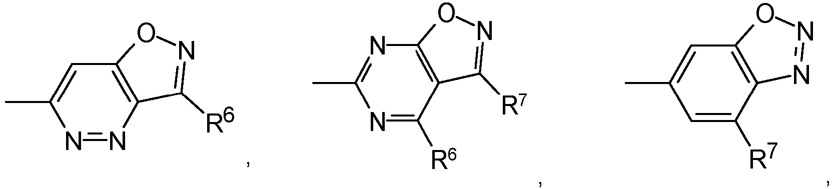 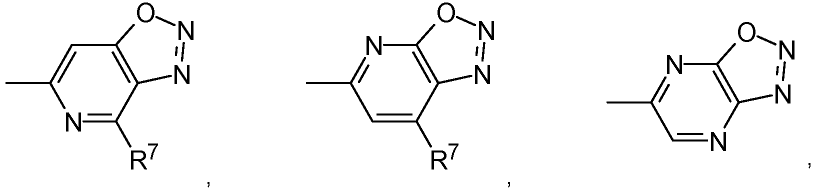 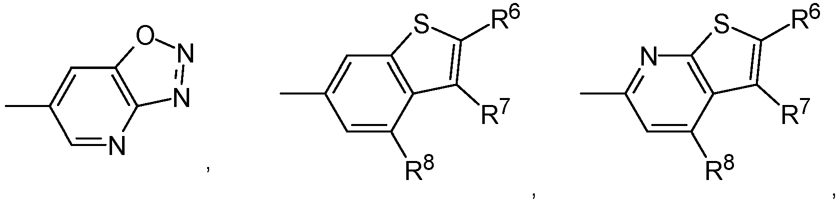 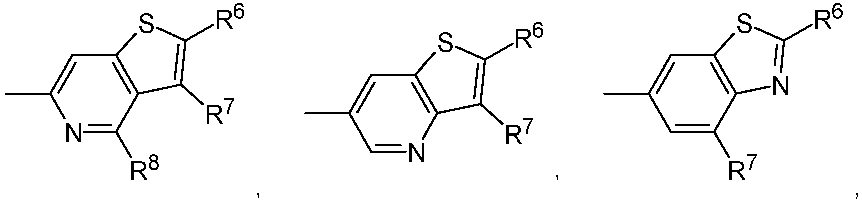 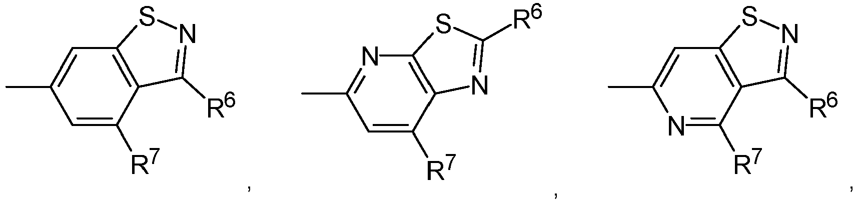 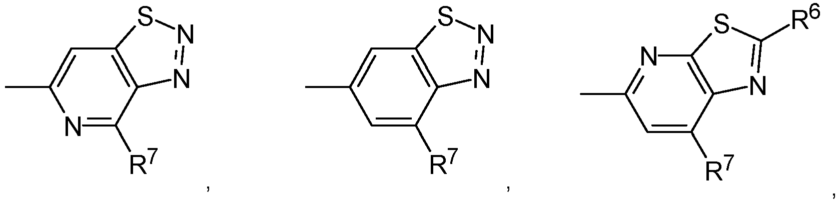 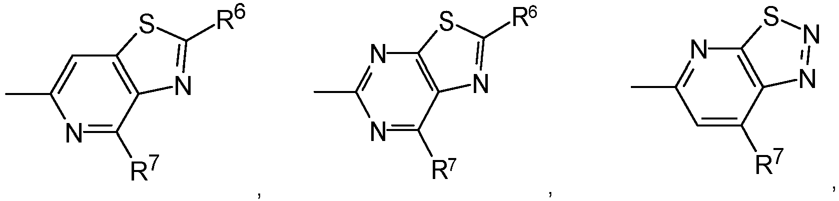 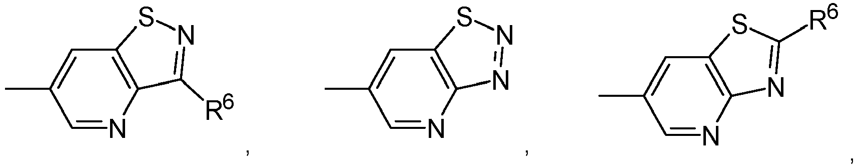 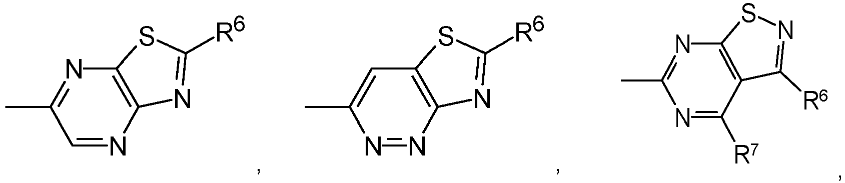 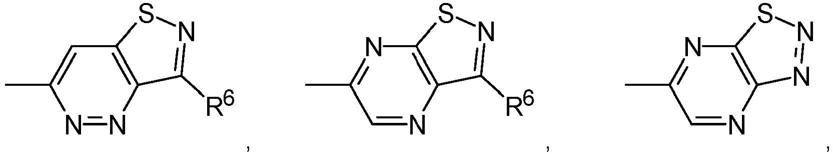 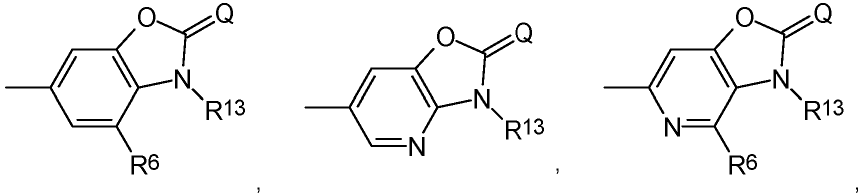 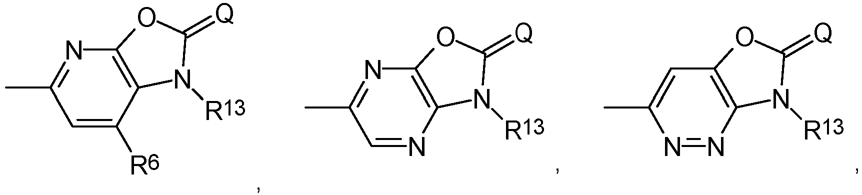 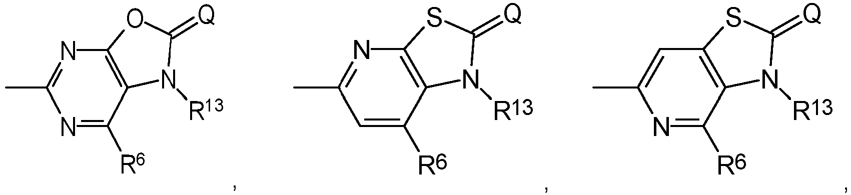 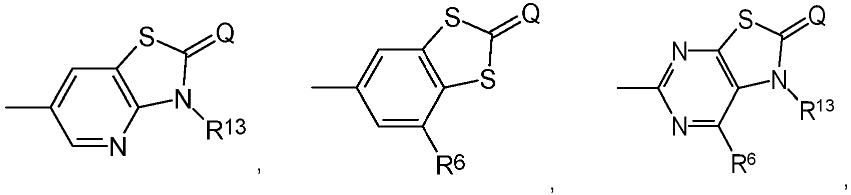 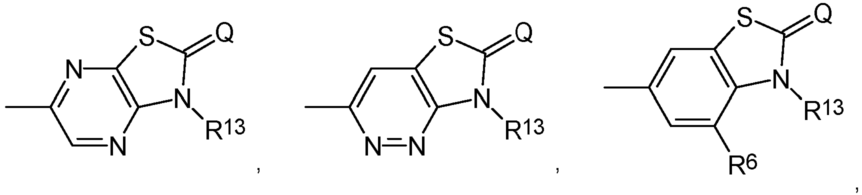 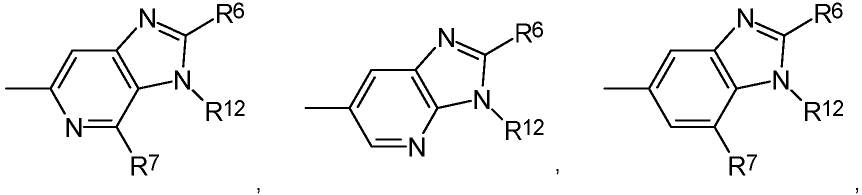 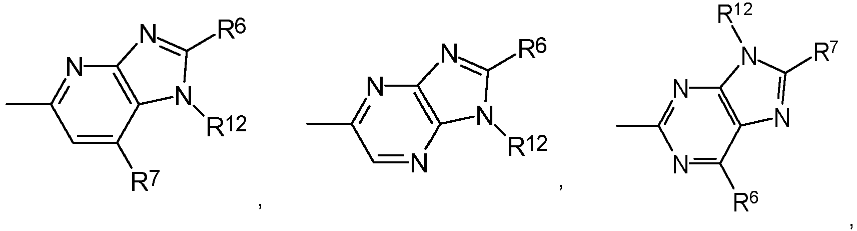 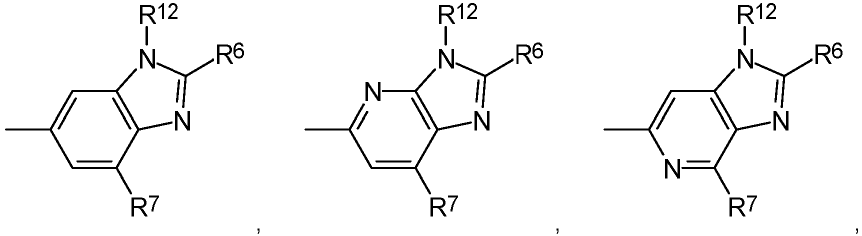 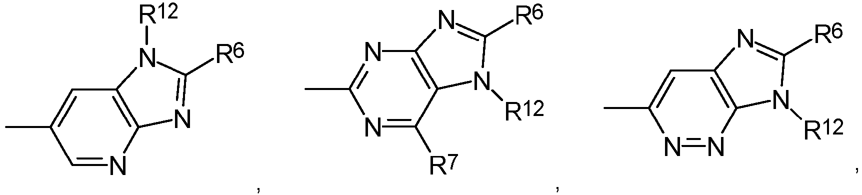 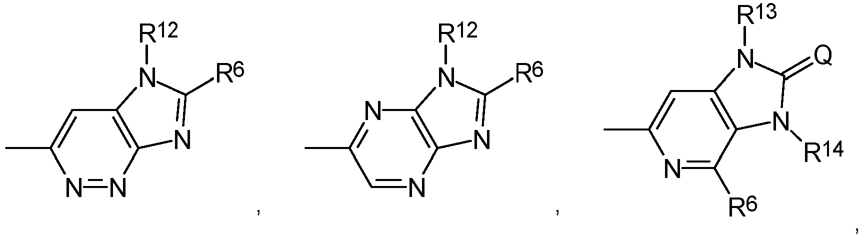 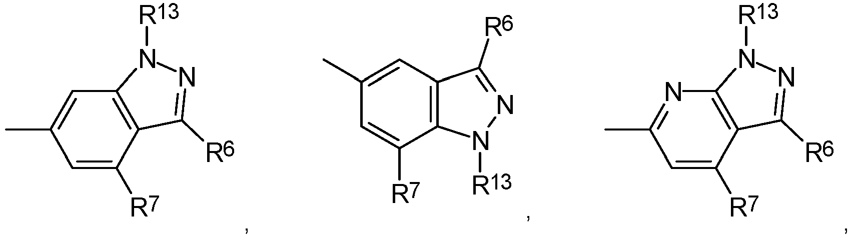 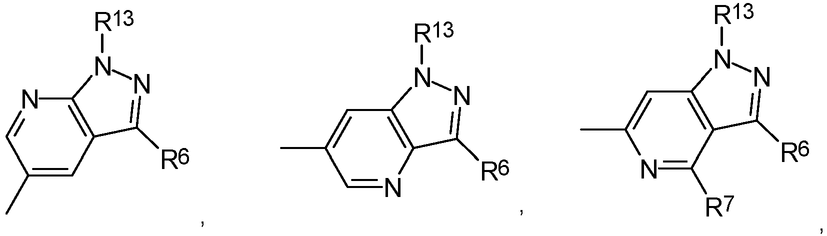 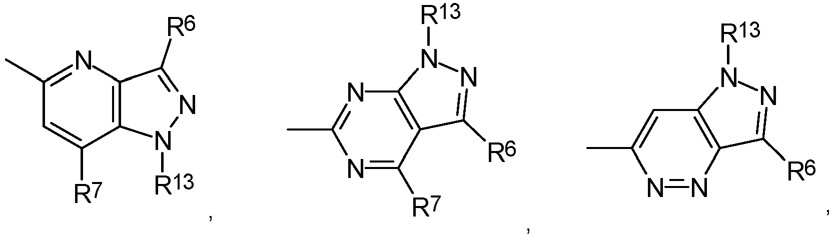 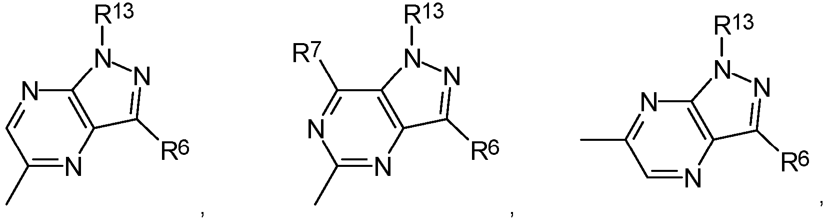 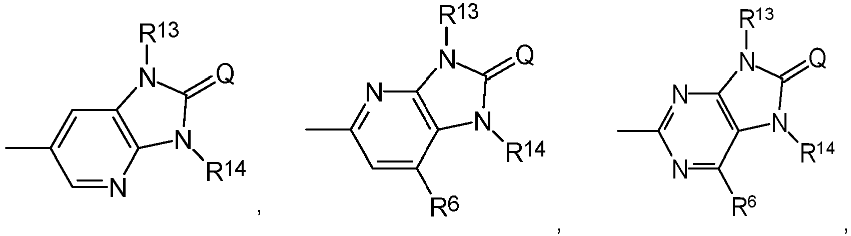 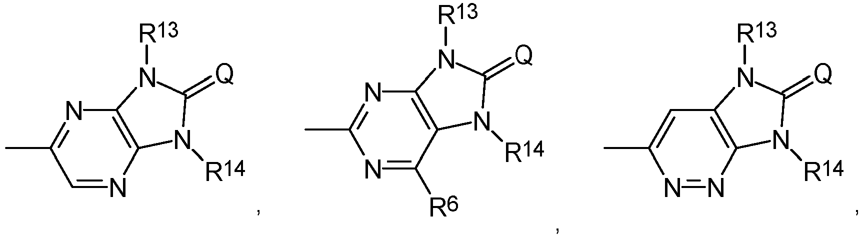 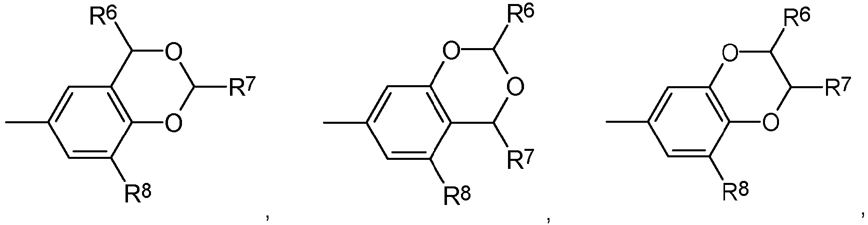 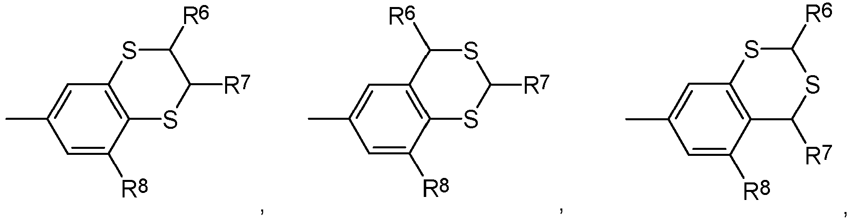 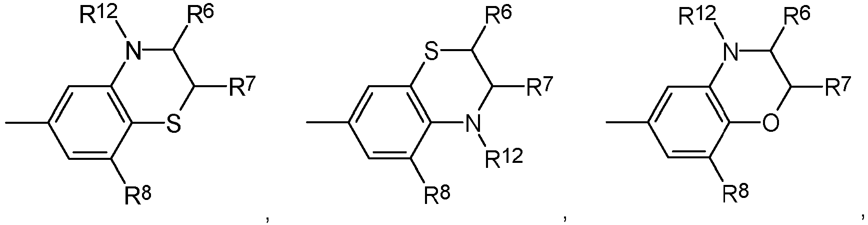 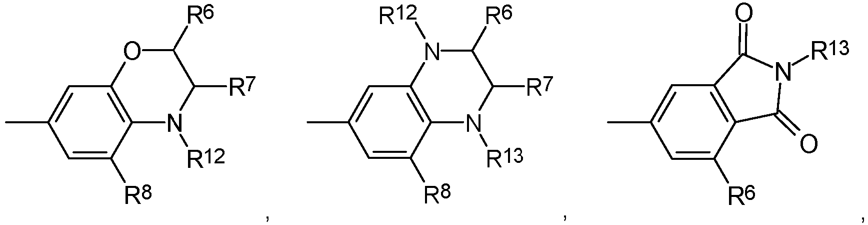 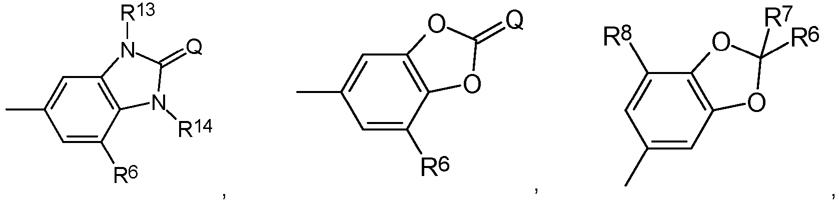 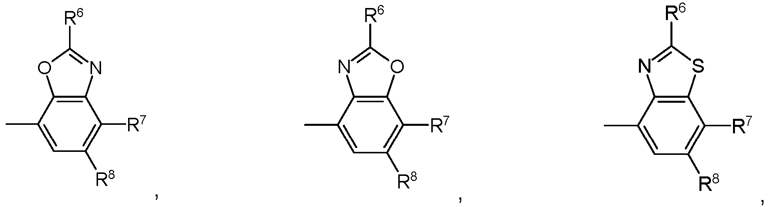 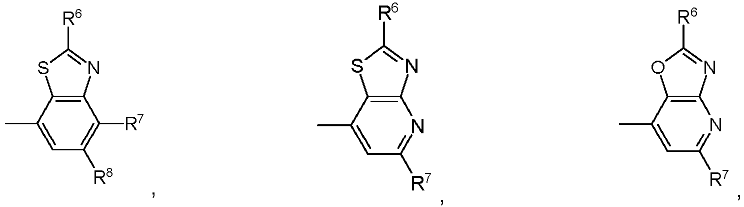 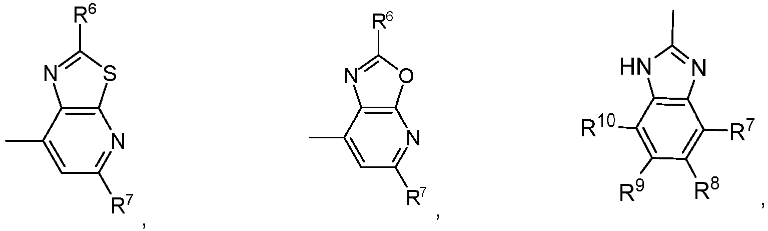 or 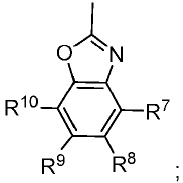
wherein Q represents =O, =S, or =N-R¹²;
wherein R^{c} represents -H, -OH, -CH₂-OH, -CHO, -CH₂CHO, -CH₂CH₂CHO, -C₂H₄-OH, -C₃H₆-OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH₂-OH, -CH₂O-C₂H₅, -CH₂-O-CH(CH₃)₂, -CH₂-O-CH(CH₂)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -CH(OAc)-CH₂OH, -CH(OAc)-CH₂OAc, -CH(OH)-CH₂OAc, -CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, -CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, -CH(N(CH₃)₂)CH₃, -CH(N(C₂H₅)₂)CH₃, -CH(NH₂)CH₂OH, -CH(NHCH₃)CH₂OH, -CH(NHC₂H₅)CH₂OH, -CH(N(CH₃)₂)CH₂OH, -CH(N(C₂Hs)₂)CH₂OH, -CH(NH₂)CH₂OCH₃, -CH(NHCH₃)CH₂OCH₃, -CH(NHC₂H₅)CH₂OCH₃, -CH(N(CH₃)₂)CH₂OCH₃, -CH(N(C₂H₅)₂)CH₂OCH₃, -CH(NH₂)CH₂OC₂H₅, -CH(NHCH₃)CH₂OC₂H₅, -CH(NHC₂H₅)CH₂OC₂H₅, -CH(N(CH₃)₂)CH₂OC₂H₅, -CH(N(C₂H₅)₂)CH₂OC₂H₅, -CH(NH₂)CH₂OAc, -CH(NHCH₃)CH₂OAc, -CH(NHC₂H₅)CH₂OAC, -CH(N(CH₃)₂)CH₂OAc, -CH(N(C₂H₅)₂)CH₂OAC, -CH₂-CH(NHAc)CH₂OH, -CH₂-CH(NHAc)CH₂OCH₃, -CH₂-CH(NHAc)CH₂OC₂H₅, -CH₂-CH(NHCH₃)CH₃, -CH₂-CH(NHC₂H₃)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)CH₂OAc, -CH₂-CH(NHCH₃)CH₂OAc, -CH₂-CH(NHC₂H₅)CH₂OAc, -CH₂-CH(N(CH₃)₂)CH₂OAc, -CH₂-CH(N(C₂H₅)₂)CH₂OAc, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -NHSO₂CH₃, -NHSO₂CF₃, -NHSO₂CH₂CF₃, -CH₂-NHSO₂CH₃, -CH₂-NHSO₂CF₃, -CH₂-NHSO₂CH₂CF₃, -C₂H₄-NHSO₂CH₃, -C₂H₄-NHSO₂CF₃, -C₂H₄-NHSO₂CH₂CF₃, -CONH₂, -CONHCH₃, -CONHC₂H₅, -CONHC₃H₇, -NH(C₂H₅), -N(C₂H₅)₂, -CH₂-NH(C₂H₅), - CH₂-N(C₂H₅)₂, -C₂H₄-NH(C₂H₅), -C₂H₄-N(C₂H₅)₂, -NO₂, -CH₂-NO₂, -C₂H₄-NO₂, -CH(OH)-NO₂, -CH(NO₂)-OH, -CO₂H, -CH₂-CO₂H, -C₂H₄-CO₂H, -CH=CH-CO₂H, -CO₂CH₃, -C0₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, -C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -O-Si(CH₃)₃, -0-Si(C₂H₅)₃, -CO-CHO, -CO-CO-CH₃, -C(OH)-CO-CH₃, -CO-C(OH)-CH₃, -CO-CH₂-CO-CH₃, -C(OH)-CH₂-CO-CH₃, -CO-CH₂-C(OH)-CH₃, -C(OH)-CH₂-C(OH)-CH₃, -F, -CI, -Br, -CH₂-F, -CHF₂, -CF₃, -CH₂Cl, -CH₂Br, -CH₂l, -C₂H₄-F, -CH₂-CHF₂, -CH₂-CF₃, -CF₂-CF₃, -O-CHF₂, -O-CF₃, -O-CH₂-CF₃, -O-C₂F₅, -CH₃, -CH₂CH₃, -CsH₇, -CH(CH₃)₂, -CH=CH₂, -C≡CH, -CH₂-CH=CH₂, or -CH₂-C≡CH, -CH₂-N₃, 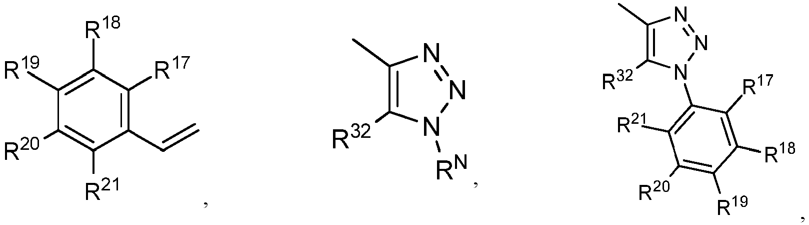 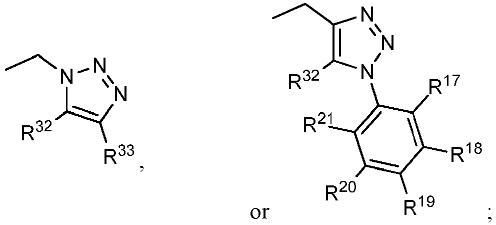
wherein **R⁴⁰, R⁴¹, R⁴², R⁴³, R⁴⁴, R⁴⁵** represent independently of each other -H, -CH₃, -C₂H₅, - OH, -OCH₃, -F, -CI, -CN, -CF₃, or -NH₂, -NHMe, -NMe₂, or wherein **R⁴⁰** and **R⁴¹** or **R⁴²** and **R⁴³** or **R⁴⁴** and **R⁴⁵** together form a double bond =CH₂ or a ketone =O;
wherein **R¹- R¹⁰** and **R¹⁷- R²¹** represent independently of each other -H, -OH, -OCH₃, -OC₂H₅, -OC₃H₇, -O-cyclo-C₃Hs, -OCH(CH₃)₂, -OC(CH₃)₃, -OC₄H₉, -OCH₂-COOH, -OPh, -OCH₂-Ph, -OCPh₃, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃Hₑ-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -CH₂NH₂, -CH₂NHCH₃, -CH₂NHC₂H₅, -CH₂NHC₃H₇, -CH₂NH-cyclo-C₃H₅, -CH₂NH[CH(CH₃)₂], -CH₂NH[C(CH₃)₃], -CH₂N(CH₃)₂, -CH₂N(C₂H₅)₂, -CH₂N(C₃H₇)₂, -CH₂N(cyclo-C₃H₅)₂, -CH₂N[CH(CH₃)₂]₂, -CH₂N[C(CH₃)₃]₂, -NHCOCH₃, -NHCOC₂H_{5,} -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]z, -O-CO-OCH₃, -0-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-CₑH₁₁, -cyclo-C_{y}H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂Hs, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂Hs)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡-C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, 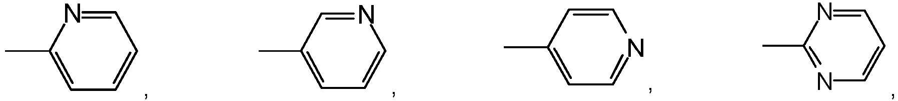 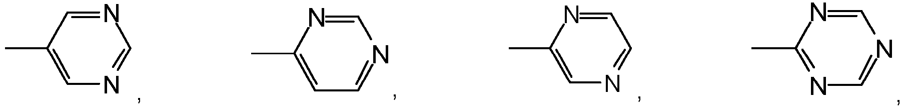 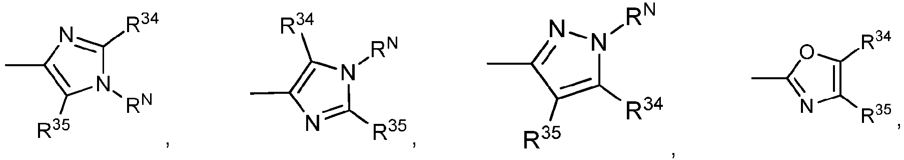 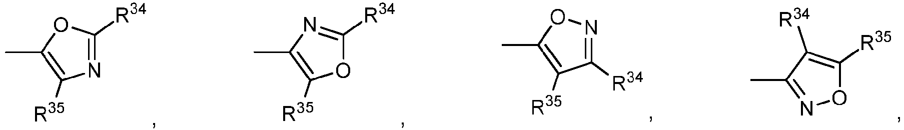 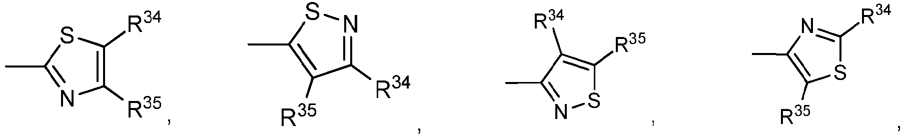 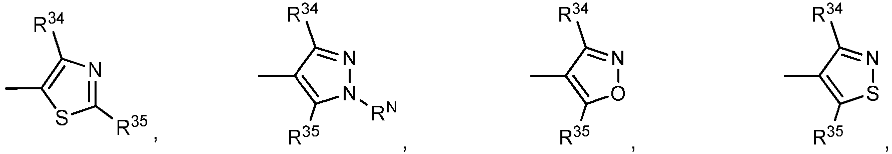 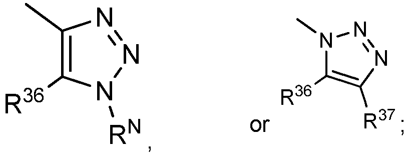
wherein **R¹⁶, R³⁸, R³⁹** represent independently of each other a lone pair, -H, -CH₃, -C₂H₅, - C₃H₇, -CH(CH₃)₂, -CH(CH₂)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH₂OH, -CH₂-SH, -CH₂CH(OH)CH₃, -OH, -OCH₃, -OCH₂CH₃, -OCH(CH₃)₂, -C₂H₄OH, -C₃H₆OH, -C₄H₈OH, -CH(CH₃)-C₂H₄OH, -C₅H₁₀OH, -CH₂-S-CH₃, -CH₂-CH₂-S-CH₃, -C₃H₆-S-CH₃, -CH₂OCH₃, -C₂H₄OCH₃, -C₃H₆OCH₃, -C₄H₈OCH₃, -CH(CH₃), -C₂H₄OCH₃, -C₅H₁₀OCH₃, -NH₂, -NHCH₃, -NHCH₂CH₃, -NHCH(CH₃)₂, -N(CH₃)₂, -N(CH₃)CH₂CH₃, -N(CH₂CH₃)₂, -N(CH₃)CH(CH₃)₂ -CH₂NH₂, - C₂H₄NH₂, -C₃H₆NH₂, -C₄H₈NH₂, -CH(CH₃)-C₂H₄NH₂, -C₅H₁₀NH₂, -CH₂-CH₂-CH₂-NH-C(NH)NH₂, -CH₂-CO₂H, -CH₂-CONH₂, -CH₂-CH₂-CO₂H, -CH₂-CH₂-CONH₂, -CH₂-CO₂CH₃, -CH₂-CONHCH₃, -CH₂-CON(CH₃)₂, -CH₂-CH₂-CO₂CH₃, -CH₂-CH₂-CONHCH₃, -CH₂-CH₂-CONH(CH₃)₂, -CH=CH-CO₂H, -CH=CH-CO₂CH₃, -CH=CH-CONHCH₃, -CH=CH-CONHC₂H₅, -CH=CH-CON(CH₃)₂, -CH=CH-CON(C₂Hs)₂, -CH₂-CH=CH-CO₂H, -CH₂-CH=CH-CO₂CH₃, -CH₂-CH=CH-CONHCH₃, -CH₂-CH=CH-CON(CH₃)₂, -CH₂-CH=CH-CONHC₂H₅, -CH₂-CH=CH-CON(C₂H₅)₂, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂Hs, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, - CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, - C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, - CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, - CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡-C-C₂H₅, -CH₂-C≡C-CH(CH₃)₂, -C≡-C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡-CH, -CH₂-Ph, 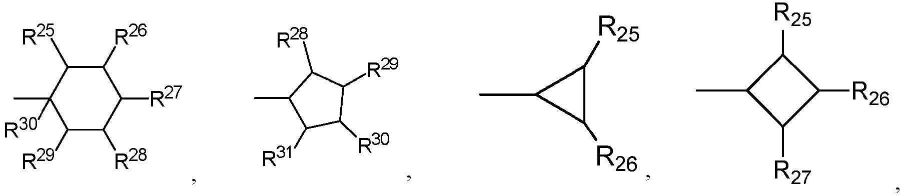 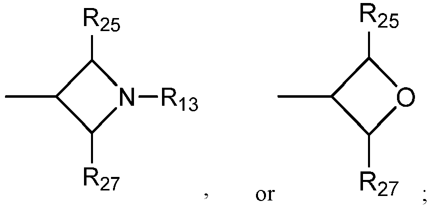
wherein **R¹²- R¹⁴** represents independently of each other -H, -CH₂F, -CHF₂, -CH₂-OCH₃, -CH₂-OH, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₃H₅, -cyclo-C₄H₇, -cyclo-C₅H₉, -cyclo-C₆H₁₁, -cyclo-C₇H₁₃, -cyclo-C₈H₁₅, -SO₂CH₃, -Ph, -CH₂-Ph, -CH₂-CH₂-Ph, -CH=CH-Ph, -CPh₃, -CHO, -CO₂CH₃, -COCH₃ -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₃H₆-C≡C-CH₃, -CH(CH₃)-CH₂-C≡CH, -CH(CH₃)-C≡C-CH₃, -C₂H₄-CH(CH₃)-C≡CH, -CH₂-CH(CH₃)-CH₂-C≡CH, -CH₂-CH(CH₃)-C≡CH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡-C-C₂H₅, -CH₂-C≡C-CH(CH₃)₂, -C≡-C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡-CH, or -CH₂-CH(C≡CH)₂;
wherein **R²⁵-R³⁷** represents independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -SO₂CH₃, -NO₂, -F, -Cl, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, - CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡-C-C₂H₅, -CH₂-C≡C-CH(CH₃)₂, -C≡-C-CH(CH₃)-C₂H₅, -C≡C-CH₂-CH(CH₃)₂, -CH(C₂H₅)-C≡C-CH₃,-C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃,
and enantiomers, stereoisomeric forms, mixtures of enantiomers, anomers, deoxy-forms, diastereomers, mixtures of diastereomers, tautomers, hydrates, solvates and racemates of the above mentioned compounds and pharmaceutically acceptable salts.

2. The compound according to claim 1, wherein **R^{A}** is selected from:
• **R^{A}** represents: 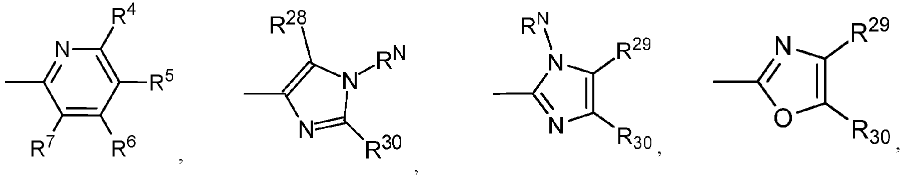 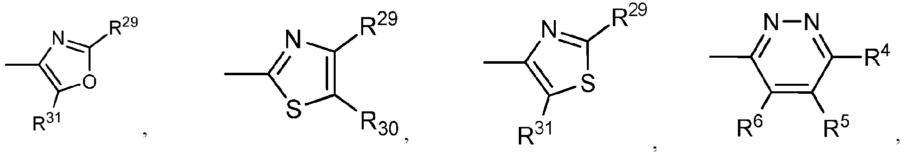 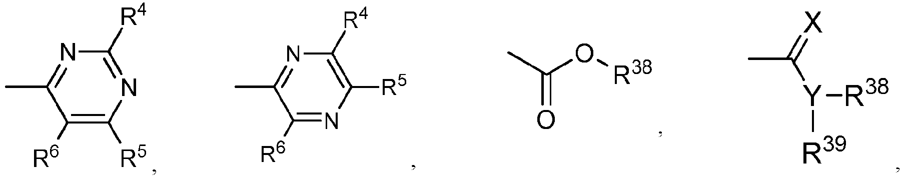 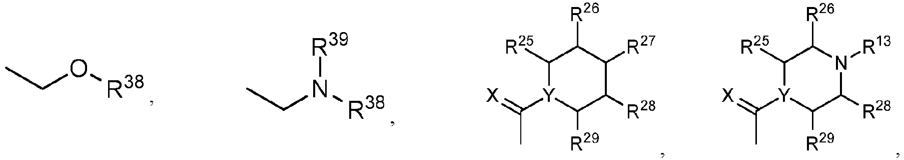 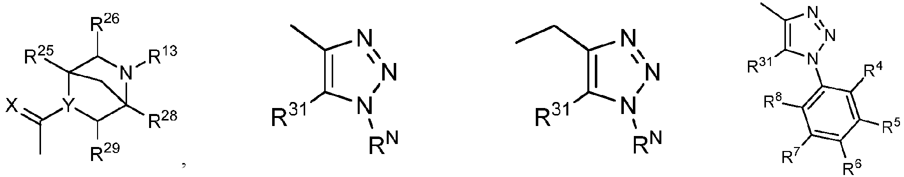 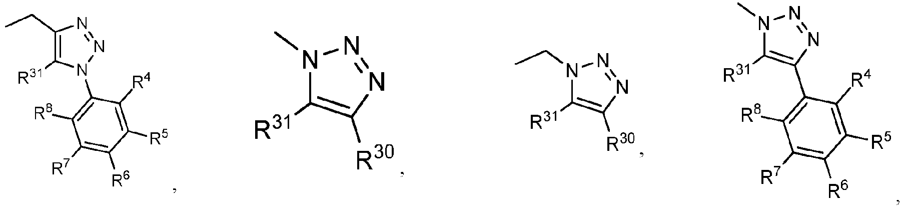 or 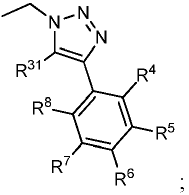 wherein **R⁴ - R³⁹, R^{N}** and **X, Y** have the meanings as defined in the general formula **1,** or **R³⁸** and **R³⁹** represents independently of each other -H, -CH₃, -C₂H₅, -C₃H₇, -CH(CH₃)₂, -CH(CH₂)₂, -C₄H₉, -CH₂-CH(CH₃)₂, -CH(CH₃)-C₂H₅, -C(CH₃)₃, -CH₂-Ph, -CF₃.
• **R^{A}** represents: -COOH, -COOCH₃, -COOC₂H₅, -COOC₃H₇, -CH₂NH₂, -CH₂NHCH₃, -CH₂NHC₂H₅, -CH₂NHC₃H₇, -CH₂N(CH₃)₂, -CH₂N(C₂H₅)₂, -CH₂N(C₃H₇)₂, -CONH₂, -CONHCH₃, -CONHC₂H₅, -CONHCH(CH₃)₂, -CONHC₃H₇, -CON(CH₃)₂, -CON(C₂H₅)₂, or -CON(C₃H₇)₂;
• **R^{A}** represents: -COOH, -COOCH₃, -COOC₂H₅, -CH₂NHCH₃, 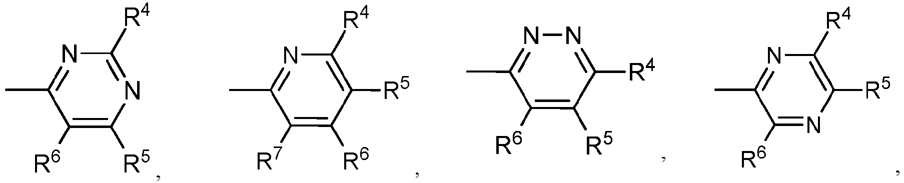 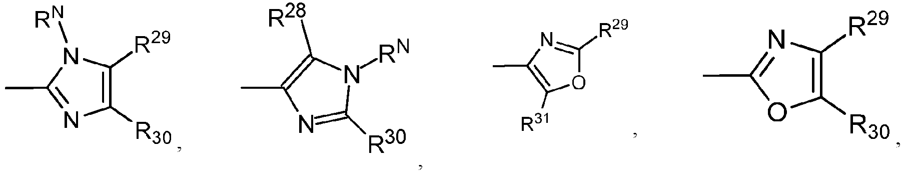 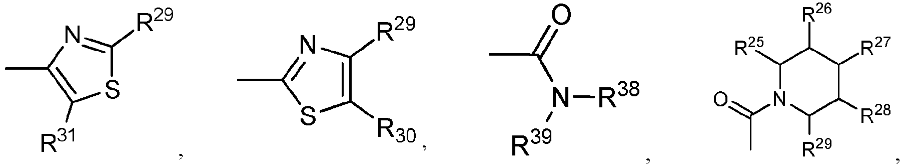 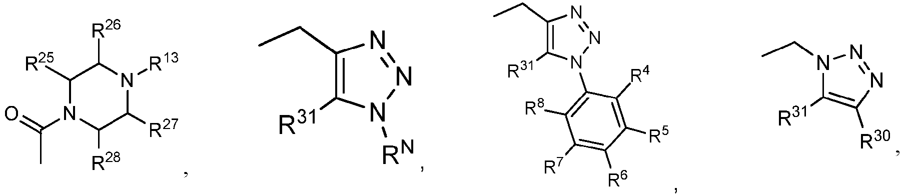 or 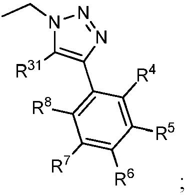
wherein **R⁴** - **R⁸** have the meanings as defined in the general formula 1, or **R⁴** - **R⁸** and **R²⁵** - **R³¹** represents independently of each other -H, -CH₃, -OMe, or -F, and more preferably -H or -CH₃
wherein **R³⁸** - **R³⁹** have the meanings as defined in the general formula **1,** or represents independently of each other -H, -CH₃, -C₂H₅, -C₃H₇, -CH(CH₃)₂, -CH(CH₂)₂, -C₄H₉, -CH₂-CH(CH₃)₂, -CH(CH₃)-C₂H₅, or -C(CH₃)₃,
wherein **R**^{N} has the meaning as defined in the general formula **1,** or **R^{N}** represents -H, -COCH₃, -COC₂H₅, -COPh, -COCH₂Ph, -SO₂Ph, -SO₂CH₂Ph, -CH₃, -C₂H₅, -C₃H₇, -CH(CH₃)₂, -Ph, or -CH₂-Ph.

3. The compound according to any of the previous claims, wherein the substituents for **R^{B}** represents: 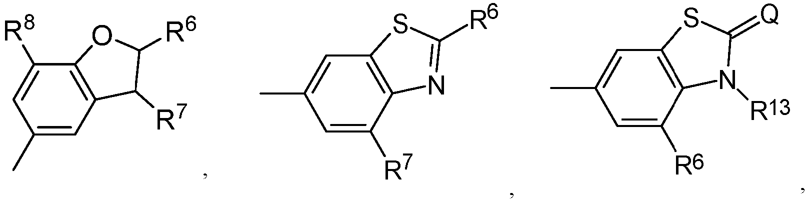 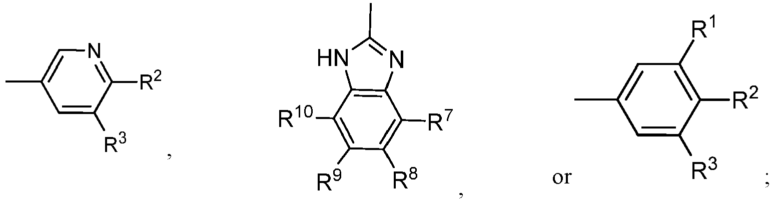 wherein **R¹ - R³, R⁶ - R¹⁰, R¹³** and **Q** have the meanings as defined in the general formula 1; or **R¹ - R³, R⁶ - R¹⁰, R¹³** and **Q** represent:
**Q** represents =O;
**R¹ - R³** and **R⁷ - R⁹** represents independently of each other -H, -F, -Cl, -Br, -I, -OH, -OCH₃, -OC₂H₅, -OC₃H₇, -CN, -CONH₂, -NHCOCH₃, -NHCOC₂H₅, -NHCOC₃H₇, -COOH, -COOCH₃, -COOC₂H₅, -COOC₃H₇, -Ph, 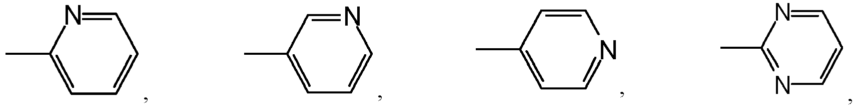 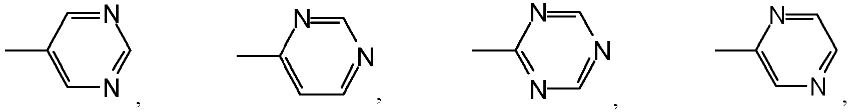 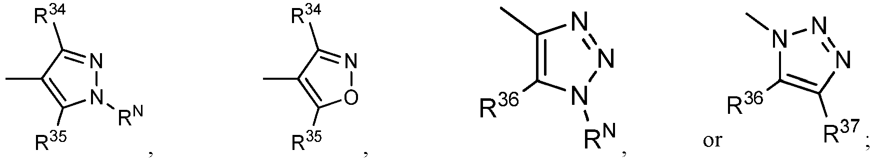
wherein R¹⁰ represents -H;
wherein R¹³ represents -H, -CH₃, or -C₂H₅, or -H;
wherein **R³⁴ - R³⁵** represents -H or -CH₃,
wherein **R³⁶ - R³⁷** and **R^{N}** have the meanings as defined in the general formula **1,**
or wherein one of **R¹ - R³ is** different from hydrogen.

4. The compound according to any of the previous claims, wherein **R^{B}** represents: 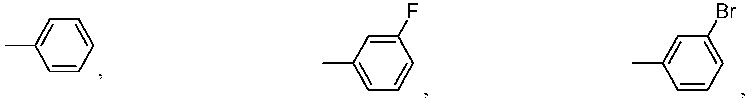 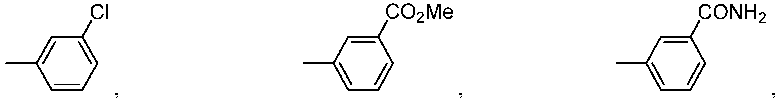 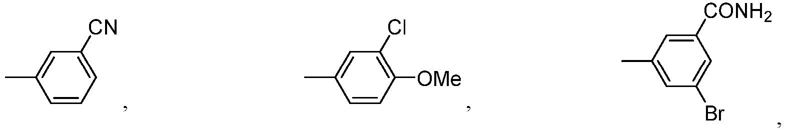 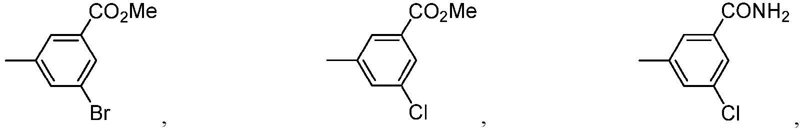 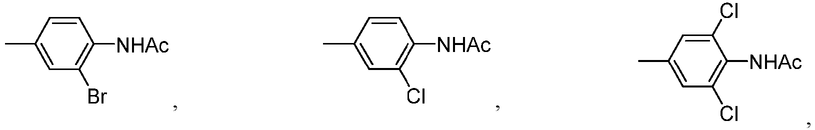 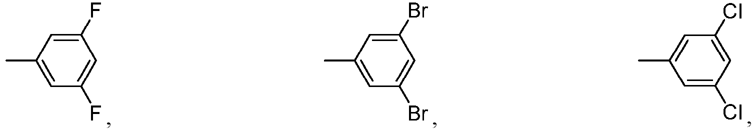 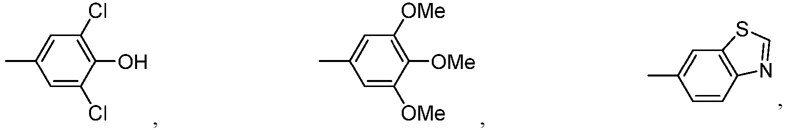 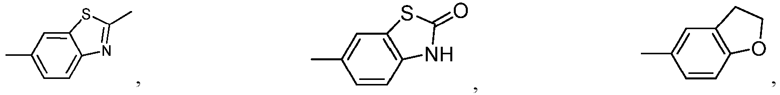 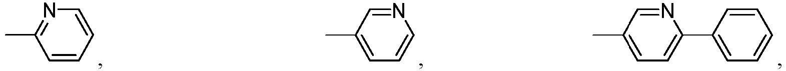 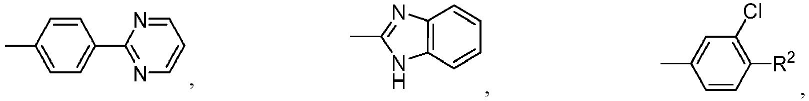 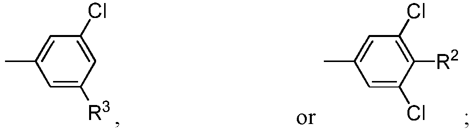
wherein **R²** represents: 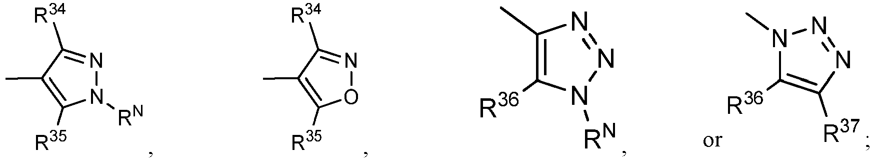
wherein **R³** represents: 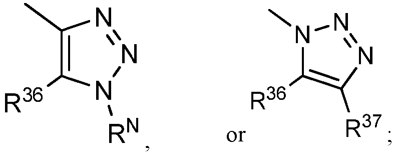
wherein **R³⁴** - **R³⁵** represents -H or -CH₃,
wherein **R³⁶** - **R³⁷** and **R^{N}** have the meanings as defined in the general formula 1.

5. The compound according to any of the previous claims, wherein **R^{c}** is selected from:
• **R^{c}** represents: -CH₂-OH, -CHO, -CH₂CHO, -CH₂CH₂CHO, -C₂H₄-OH, -C₃H₆-OH, -OH, -O-CH₃, -O-C₂H₅, -O-CH₂-OH, -O-CH(CH₃)₂, -O-CH₂-O-CH₃, -O-C₂H₄-O-CH₃, -CH₂-O-CH₃, -CH₂-O-CH(CH₂)₂, -CH₂-O-CH₂-OH, -CH₂O-C₂H₅, -CH₂-O-CH(CH₃)₂, -CH₂-O-C₃H₇, -CO-CH₃, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -CH(OAc)-CH₂OH, -CH(OAc)-CH₂OAc, -CH(OH)-CH₂OAc, -CH(OH)-CH₂-NH₂, -CH₂-CH(OH)-CH₂-NH₂, -CH(OCH₃)-CH₂-NH₂, -CH(OC₂H₅)-CH₂-NH₂, -CH₂-CH(OCH₃)-CH₂-NH₂, -CH₂-CH(OC₂H₅)-CH₂-NH₂, -CH(OH)-CH₂-NHCH₃, -CH(OH)-CH₂-NHC₂H₅, -CH₂-CH(OH)-CH₂-NHCH₃, -CO-C₃H₇, -CH₂-CH(OH)-CH₂-NHC₂H₅, -CH(OCH₃)-CH₂NHCH₃, -CO-C₂H₅, -CO-CH(CH₃)₂, -CH(OC₂H₅)-CH₂NHCH₃, -CH₂-CH(OCH₃)-CH₂-NHCH₃, -O-C₃H₇, -CH₂-CH(OC₂H₅)-CH₂-NHCH₃, -CH(OCH₃)-CH₂NHC₂H₅, -CH(OC₂H₅)-CH₂NHC₂H₅, -CH(OCH₃)-CH₂N(CH₃)₂, -CH(OC₂H₅)-CH₂N(CH₃)₂, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -CH(NHCH₃)CH₃, -CH(NHC₂H₅)CH₃, -CH(N(CH₃)₂)CH₃, -CH(N(C₂H₅)₂)CH₃, -CH(NH₂)CH₂OH, -CH(NHCH₃)CH₂OH, -CH(NHC₂H₅)CH₂OH, -CH(N(CH₃)₂)CH₂OH, -CH(N(C₂H₅)₂)CH₂OH, -CH(NH₂)CH₂OCH₃, -CH(NHCH₃)CH₂OCH₃, -CH(NHC₂H₅)CH₂OCH₃, -CH(N(CH₃)₂)CH₂OCH₃, -CH(N(C₂H₅)₂)CH₂OCH₃, -CH(NH₂)CH₂OC₂H₅, -CH(NHCH₃)CH₂OC₂H₅, -CH(NHC₂H₅)CH₂OC₂H₅, -CH(N(CH₃)₂)CH₂OC₂H₅, -CH(N(C₂H₅)₂)CH₂OC₂H₅, -CH(NH₂)CH₂OAc, -CH(NHCH₃)CH₂OAc, -CH(NHC₂H₅)CH₂OAC, -CH(N(CH₃)₂)CH₂OAc, -CH(N(C₂H₅)₂)CH₂OAc, -CH₂-CH(NHAc)CH₂OH, -CH₂-CH(NHAc)CH₂OCH₃, -CH₂-CH(NHAc)CH₂OC₂H₅, -CH₂-CH(NHCH₃)CH₃, -CH₂-CH(NHC₂H₅)CH₃, -CH₂-CH(N(CH₃)₂)CH₃, -CH₂-CH(N(C₂H₅)₂)CH₃, -CH₂-CH(NH₂)CH₂OH, -CH₂-CH(NHCH₃)CH₂OH, -CH₂-CH(NHC₂H₅)CH₂OH, -CH₂-CH(N(CH₃)₂)CH₂OH, -CH₂-CH(N(C₂H₅)₂)CH₂OH, -CH₂-CH(NH₂)CH₂OCH₃, -CH₂-CH(NHCH₃)CH₂OCH₃, -CH₂-CH(NHC₂H₅)CH₂OCH₃, -CH₂-CH(N(CH₃)₂)CH₂OCH₃, -CH₂-CH(N(C₂H₅)₂)CH₂OCH₃, -CH₂-CH(NH₂)CH₂OC₂H₅, -CH₂-CH(NHCH₃)CH₂OC₂H₅, -CH₂-CH(NHC₂H₅)CH₂OC₂H₅, -CH₂-CH(N(CH₃)₂)CH₂OC₂H₅, -CH₂-CH(N(C₂H₅)₂)CH₂OC₂H₅, -CH₂-CH(NH₂)CH₂OAc, -CH₂-CH(NHCH₃)CH₂OAc, -CH₂-CH(NHC₂H₅)CH₂OAc, -CH₂-CH(N(CH₃)₂)CH₂OAc, -CH₂-CH(N(C₂H₅)₂)CH₂OAc, -CH₂-CH(NHAc)CH₂OH, -CH₂-CH(NHAc)CH₂OCH₃, -CH₂-CH(NHAc)CH₂OC₂H₅, -NHCOCH₃, -CH₂-NHCOCH₃, -C₂H₄-NHCOCH₃, -NHCHO, -CH₂-NHCHO, -C₂H₄-NHCHO, -CONH₂, -CONHCH₃, -CONHC₂H₅, -CONHC₃H₇, -NH(C₂H₅), -N(C₂H₅)₂, -CH₂-NH(C₂H₅), -CH₂-N(C₂H₅)₂, -C₂H₄-NH(C₂H₅), -C₂H₄-N(C₂H₅)₂, -CO₂H, -CH₂-CO₂H, -C₂H₄-CO₂H, -CH=CH-CO₂H, -CO₂CH₃, -CO₂C₂H₅, -CO₂CH(CH₃)₂, -CH₂-CO₂CH₃, -CH₂-CO₂C₂H₅, -CH₂-CO₂CH(CH₃)₂, -C₂H₄-CO₂CH₃, -C₂H₄-CO₂C₂H₅, -C₂H₄-CO₂CH(CH₃)₂, -CO₂NH₂, -CO₂NHCH₃, -CO₂N(CH₃)₂, -CH₂-CO₂NH₂, -CH₂-CO₂NHCH₃, -CH₂-CO₂N(CH₃)₂, -C₂H₄-CO₂NH₂, -C₂H₄-CO₂NHCH₃, -C₂H₄-CO₂N(CH₃)₂, -CH₂-F, -CH₂Cl, -CH₂Br, -CH₂l, -CHF₂, -CF₃, -C₂H₄-F, - CH₂-CF₃, -CF₂-CF₃, -O-CHF₂, -O-CF₃, -CH₃, -CH₂CH₃, -C₃H₇, -CH(CH₃)₂, - CH=CH₂, -C≡CH, -CH₂-CH=CH₂, -CH₂-C≡CH, -CH₂-N₃, 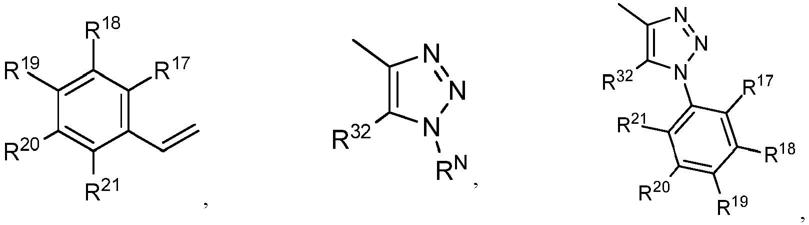 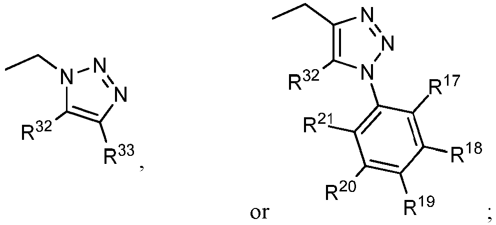 wherein R¹⁷ - R²¹, R³² - R³³ and R^{N} have the meanings as defined in the general formula 1;
• R^{C} represents: -OH, -CH₂-OH, -C₂H₄-OH, -C₃H₆-OH, -CHO, -CH₂CHO, -CH₂CH₂CHO, -O-CH₃, -O-C₂H₅, -O-C₃H₇, -O-CH(CH₃)₂, -CONH₂, -CONHCH₃, -CONHC₂H₅, -CONHC₃H₇, -CO-CH₃, -CO-C₂H₅, -CO-C₃H₇, -CO-CH(CH₃)₂, -CO₂H, -CH₂-CO₂H, -C₂H₄-CO₂H, -O-CH₂-OH, -O-CH₂-O-CH₃, -O-C₂H₄-O-CH₃, -CH₂-O-CH₃, -CH₂-O-CH(CH₂)₂, -CH₂-O-CH₂-OH, -CH₂O-C₂H₅, -CH₂-O-CH(CH₃)₂, -CH₂-O-C₃H₇, -CH₂-CO-CH₃, -CO-CH₂-OH, -CH(OH)-CH₃, -C(OH)(CH₃)₂, -CH(CH₃)CH₂OH, -CH(OH)-CH₂-OH, -CH₂-CH(OH)-CH₃, -CH₂-CH(OH)-CH₂-OH, -CH(OCH₃)-CH₂OH, -CH(OC₂H₅)-CH₂OH, -CH(OCH₃)-CH₂OCH₃, -CH(OC₂H₅)-CH₂OCH₃, -CH(OC₂H₅)-CH₂OC₂H₅, -NH₂, -NHCH₃, -N(CH₃)₂, -CH₂-NH₂, -CH₂-NHCH₃, -CH₂-N(CH₃)₂, -C₂H₄-NH₂, -C₂H₄-NHCH₃, -C₂H₄-N(CH₃)₂, -NH(C₂H₅), -N(C₂H₅)₂, -CH₂-NH(C₂H₅), -CH₂-N(C₂H₅)₂, -C₂H₄-NH(C₂H₅), -C₂H₄-N(C₂H₅)₂, -CH₂-F, -CH₂Cl, -CH₂Br, -CH₂I, -CHF₂, -CF₃, -C₂H₄-F, -CH₂-CF₃, -CF₂-CF₃, -CH₃, -CH₂CH₃, -C₃H₇, -CH(CH₃)₂, -CH=CH₂, -C≡CH, -CH₂-CH=CH₂, -CH₂-C≡CH, or -CH₂-N₃,
• R^{c} represents: -OH, -NH₂, -CH₂F, -CHF₂, -CH₂Br, -CH₂I, -CH₂CH₃, -CH=CH₂, -CH₂OH, -CHO, -CO₂H, -CONH₂, -COCH₃, -OCH₃, -OCH₂CH₃, -OCH(CH₃)₂, -OCH₂OCH₃, -OC₂H₄OCH₃, -CH₂OCH₃, -CH₂OCH₂CH₃, -CH₂-O-CH(CH₂)₂, -CH₂CH₂OH, -CH₂CHO, -CH₂CH₂CHO, - CH₂NH₂, -CH₂NHCH₃, -CH₂N(CH₃)₂, -CH(OH)CH₃, -C(OH)(CH₃)₂, -CH₂CH(OH)CH₃, -CH(OH)CH₂OH, -C≡CH, or -CH₂-N₃, and
• R^{C} represents: a substituent comprising a hydroxyl group or an alkoxy group such as - CH₂OH, -CH₂CH₂OH, -CH(OH)CH₃, -C(OH)(CH₃)₂, -CH₂CH(OH)CH₃, - CH(OH)CH₂OH -CH₂OCH₃, or -CH₂-O-CH(CH₂)₂.

6. The compound according to any of the previous claims, wherein R⁴⁰, R⁴¹, R⁴², R⁴³, R⁴⁴, R⁴⁵ are independently selected from -H, -CH₃, -C₂H₅, -OH, -OCH₃, -F, -Cl, or -NH₂.

7. The compound according to any of the previous claims, wherein the general formula 1 is replaced by general formula 2 or 3: 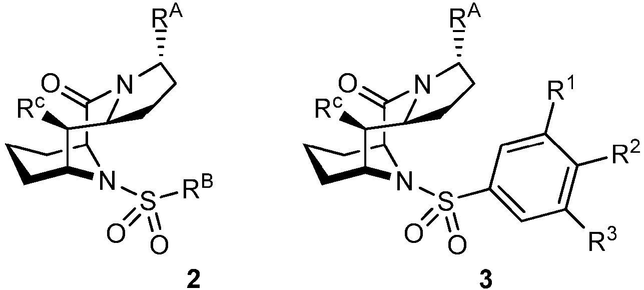
wherein the substituents **R^{A}**, **R^{B}** , **R^{C}** and **R¹** - **R³** have the meanings as defined for formula 1 herein;
as well as enantiomers, stereoisomeric forms, mixtures of enantiomers, anomers, deoxy-forms, diastereomers, mixtures of diastereomers, tautomers, hydrates, solvates and racemates of the above mentioned compounds and pharmaceutically acceptable salts.

8. Compound according to any one of the previous claims for use as ligand of FK506-binding proteins selected from the group consisting of human FKBP12, FKBP12.6, FKBP51, FKBP52, LpMip, CtMip, CpMip, NgMip, KpMip, BpMip, TcMip, EcFKLB, EcFKPA, PaFKLB, PaFKPA, AbFKLB, and AbFKPA, as well as combinations thereof.

9. Compound according to any one of the previous claims for use as pharmaceutically active agent in medicine.

10. Compound according to any one of the previous claims for use in the treatment or prophylaxis of psychiatric disorders, neurological disorders, metabolic diseases, cancers, glucocorticoid hyposensitivity syndromes, peripheral glucocorticoid resistance, infectious diseases, alopecia, abnormally elevated intraocular pressure, macular degeneration, oxidative damage to eye tissues, vision disorder, sleeping disorders, asthma, diabetes, traumatic brain injury, nerve injury, Alzheimer's disease, Huntington's disease, Parkinson's disease, ischemia, memory impairment and for neuroprotection, neuroregeneration, promoting hair growth, stimulating neurite growth, wound healing, antiglaucomatous medications, improving vision, enhancing memory performance, for the use in treatment or prevention of multi-drug resistance, for the use in limiting or preventing haemorrhage or neovascularization and for the use in treatment of diseases relating to neurodegeneration.

11. Use of a compound according to any one of claims 1 to 8, as agents for inducing protein-protein interactions (PPIs) in vitro acting as molecular glues.

12. Compound according to any one of claims 1 to 8, for use in the treatment of a disease and/or condition by inducing protein-protein interactions (PPIs) in vivo acting as molecular glues.

13. Pharmaceutical composition comprising at least one compound according to any one of claims 1 to 8, together with at least one pharmaceutically acceptable carrier, solvent or excipient or together with at least one pharmaceutically acceptable carrier, solvent or excipient and at least one active agent selected from the group consisting of an anti-depressant and other psychotropic drugs, as well as diabetes drugs, pain drugs and/or antibiotics.
